# EUROPEAN PATENT APPLICATION

(11) **EP 4 782 541 A1**
(43) Date of publication of application: **29.07.2026**
(21) Application number: 24867440.0
(22) Date of filing: 18.09.2024
(51) Int. Cl.: C12N 15/63, C07K 16/46, A61P 35/00, C07K 16/28, A61K 39/395, G01N 33/574, C12N 15/13, A61K 47/68

(54) **BISPECIFIC ANTIBODY TARGETING HUMAN ROR1 AND CD3**

(30) Priority: 18.09.2023 CN 202311206180
(71) Applicant: Nanjing Probio Biotech Co., Ltd., Nanjing, Jiangsu 211100 (CN)
(72) Inventor: JIA, Wenshuang, Nanjing, Jiangsu 211100 (CN); LIANG, Yu, Nanjing, Jiangsu 211100 (CN); CHEN, Li, Nanjing, Jiangsu 211100 (CN); LI, Lianqu, Nanjing, Jiangsu 211100 (CN); ZHOU, Yi, Nanjing, Jiangsu 211100 (CN); LI, Yingyu, Nanjing, Jiangsu 211100 (CN); XIN, Yang, Nanjing, Jiangsu 211100 (CN); NIE, Jingjing, Nanjing, Jiangsu 211100 (CN)
(74) Representative: Ström & Gulliksson AB
(86) International application number: PCT/CN2024/119415
(87) International publication number: WO 2025/061032

(57) **Abstract**

Provided are a bispecific antibody targeting ROR1 and CD3 and the use thereof. The bispecific antibody comprises a first antigen-binding portion and a second antigen-binding portion, wherein the first antigen-binding portion is an antibody that specifically binds to ROR1 or an antigen-binding fragment thereof, and the second antigen-binding portion is an antibody that specifically binds to CD3 or an antigen-binding fragment thereof.

## Description

### CROSS-REFERENCE TO RELATED APPLICATION

The present application claims the right of priority for Chinese patent application no. CN 202311206180.4 filed on September 18, 2023, which is incorporated herein by reference in its entirety.

### TECHNICAL FIELD

The present invention belongs to the fields of tumour immunotherapy and molecular immunology, specifically related to a bispecific antibody targeting human ROR1×CD3 with high affinity, high specificity and higher safety, and the use thereof.

### BACKGROUND

Receptor tyrosine kinase-like orphan receptor 1 (ROR1) is a oncofetal protein. As an orphan receptor tyrosine kinase-like surface antigen, it is expressed by many tissues during embryogenesis and functions in embryonic skeletal, cardiopulmonary and neural development. ROR1 is mainly expressed in embryonic tissues; its expression levels in adult tissues (including the parathyroid gland, the pancreatic islet, and the esophagus, the stomach, and duodenal regions) are restricted (Michael Y. et al., 2019). ROR1 is expressed in many B-cell malignant tumours and various cancer cell lines, including solid tumours such as chronic lymphocytic leukaemia (CLL), mantle cell lymphoma (MCL) and ovarian cancer. ROR1 is expressed on the surface of CLL cells and is an accurate and reliable biomarker of minimal residual disease in CLL. High ROR1 expression level is associated with increased circulating leukaemia cells and greater disease aggressiveness, and ROR1 expression is retained on CLL cells during and after treatment. Therefore, ROR1 is an ideal drug target for cancer treatment (Balakrishnan et al., Clin Cancer Res. 2017 Jun 15; 23(12):3061-3071, DOI:10.1158/1078-0432.CCR-16-2083; Aghebati-Maleki et al., Biomedicine & Pharmacotherapy. 88(2017):814-822, doi.org/10.1016/j.biopha.2017.01.070; De Propris et al., British Journal of Haematology, 2020,190,e329-e363, DOI:10.1111/bjh.16910).

Currently, there are a variety of anticancer therapies targeting ROR1. These therapies include monoclonal antibodies, CAR-T, antibody-drug conjugates (ADCs) and bispecific antibodies and other treatment modes. Zilovertamab inhibits Wnt-5a-induced NF-κB activation by blocking the binding between ROR1 and Wnt-5a, thereby suppressing autocrine IL-6-dependent STAT3 activation in CLL (Chen et al, Blood (2019) 134(13):1084-1094, doi.org/10.1182/blood.2019001366). Zilovertamab is in a phase III clinical trial for the treatment of relapsing or refractory MCL. Clinical trial results of Zilovertamab Vedotin (MK-2140) have shown significant therapeutic effects in both MCL and diffuse large B-cell lymphoma (DLBCL) (Michael L. Wang et al, 2021, NEJM Evid 2022; 1(1), DOI:10.1056/EVIDoa2100001).

T-cell redirection bispecific antibody (T cell engage, TCE) has been widely studied and used in cancer treatment, and remarkable progress has been achieved especially in the treatment of hematologic malignancies. For example, Blinatumomab (CD19×CD3), the first one approved for marketing by the FDA in 2014, has shown significant clinical effects in the treatment of refractory or relapsed B-cell acute lymphoblastic leukaemia (Miguel J Franquiz et al, Biologics: Targets and Therapy 2020:14 23-34, DOI:10.2147/BTT.S202746). The ROR1×CD3 antibody is constructed by combining the ROR1 antigen and the CD3ε subunit. The antibody may recognize and bind the ROR1 antigen on the surface of tumour cells and the CD3ε subunit on the surface of T cells, thereby contacting T cells closely with tumour cells and releasing a large number of cytotoxic molecules, such as perforin, targetin and interferon-y, to kill cancer cells. Compared with traditional monoclonal antibody therapy, the ROR1×CD3 bispecific antibody has higher specificity and stronger antitumour activity. Currently, only NVG-111 (NovalGen Ltd) and EMB-07 (EpimAb) are ROR1×CD3 bispecific antibodies in clinical research stages, while ROR1 is a highly promising target in cancer treatment. Thus, it is still necessary in the field to develop various forms of ROR1×CD3 antibodies to enhance tumour killing effect and reduce the risk of Off-tumor.

Targeting ROR1 for tumour immunotherapy is a very useful treatment method. The present patent develops a new bispecific antibody specifically targeting human ROR1×CD3, which exhibits enhanced therapeutic window and safety. (1) TCE distinguishes between binding to tumour cells and normal cells based on ROR1 specific binding valence; (2) cytotoxicity is decoupled from cytokine release, manifested as lower cytokine secretion during target cell killing activity. The antibody can be used to inhibit the growth of ROR1-positive tumour cells and treat cancers expressing ROR1.

### Summary of the Invention

The present invention relates to a TCE molecule that is functionally against the human ROR1 target.

One aspect of the present invention provides a bispecific antibody targeting ROR1 and CD3, comprising a first antigen-binding portion and a second antigen-binding portion, wherein the first antigen-binding portion is an antibody that specifically binds to ROR1 or an antigen-binding fragment thereof, and the second antigen-binding portion is an antibody that specifically binds to CD3 or an antigen-binding fragment thereof.

In some embodiments, the first antigen-binding portion comprises a heavy chain variable region VH. The VH comprises HCDR1, HCDR2 and HCDR3. The HCDR1, HCDR2 and HCDR3 are selected from the following groups: (1) HCDR1 as set forth in SEQ ID NO: 19, HCDR2 as set forth in SEQ ID NO: 20, and HCDR3 as set forth in SEQ ID NO: 21; (2) HCDR1 as set forth in SEQ ID NO: 23, HCDR2 as set forth in SEQ ID NO: 24, and HCDR3 as set forth in SEQ ID NO: 25; (3) HCDR1 as set forth in SEQ ID NO: 27, HCDR2 as set forth in SEQ ID NO: 28, and HCDR3 as set forth in SEQ ID NO: 29; (4) HCDR1 as set forth in SEQ ID NO: 31, HCDR2 as set forth in SEQ ID NO: 32, and HCDR3 as set forth in SEQ ID NO: 33; and (5) HCDR1 as set forth in SEQ ID NO: 35, HCDR2 as set forth in SEQ ID NO: 36 and HCDR3 as set forth in SEQ ID NO: 37.

In some embodiments, the VH of the first antigen-binding portion comprises an amino acid sequence as set forth in SEQ ID NO: 18, 22, 26, 30 or 34, or a sequence having at least 80% sequence identity to the amino acid sequence as set forth in SEQ ID NO: 18, 22, 26, 30 or 34. In some embodiments, the first antigen-binding portion is a single domain antibody.

In some embodiments, the second antigen-binding portion comprises a heavy chain variable region VH and a light chain variable region VL. The VH comprises HCDR1 as set forth in SEQ ID NO: 39, HCDR2 as set forth in SEQ ID NO: 40, and HCDR3 as set forth in SEQ ID NO: 41. The VL comprises LCDR1 as set forth in SEQ ID NO: 43, LCDR2 as set forth in SEQ ID NO: 44, and LCDR3 as set forth in SEQ ID NO: 45.

In some embodiments, the second antigen-binding portion comprises an amino acid sequence as set forth in SEQ ID NO: 38 or a sequence having at least 80% sequence identity to the amino acid sequence as set forth in SEQ ID NO: 38, and the VL of the second antigen-binding portion comprises an amino acid sequence as set forth in SEQ ID NO: 42 or a sequence having at least 80% sequence identity to the amino acid sequence as set forth in SEQ ID NO: 38. In some embodiments, the second antigen-binding portion is an Fab fragment.

In some embodiments, the bispecific antibody further comprises an Fc fragment. In some embodiments, the Fc fragment has a Knob-Hole structure. In some embodiments, the Fc fragment has a mutation that reduces or eliminates the binding of the antibody to Fcy receptor, preferably a L234S/L235T/G236R triple mutation.

In some embodiments, the first antigen-binding portion is fused to the N-terminus of the first Fc fragment, and the second antigen-binding portion is fused to the N-terminus of the second Fc fragment. In some embodiments, the second antigen-binding portion is an Fab fragment and the heavy chain of the Fab fragment is fused to the N-terminus of the second Fc fragment. In some embodiments, the first Fc fragment and the second Fc fragment may form a mutation of the Knob-Hole structure. In some embodiments, both the first Fc fragment and the second Fc fragment have a mutation that reduces or eliminates the binding of the antibody to the Fcγ receptor, preferably a L234S/L235T/G236R triple mutation.

In some embodiments, the bispecific antibody further comprises a third antigen-binding portion, wherein the third antigen-binding portion is an antibody that specifically binds to ROR1 or an antigen-binding fragment thereof.

In some embodiments, the third antigen-binding portion comprises a heavy chain variable region VH. The VH comprises HCDR1, HCDR2 and HCDR3. The HCDR1, HCDR2 and HCDR3 are selected from the following groups: (1) HCDR1 as set forth in SEQ ID NO: 19, HCDR2 as set forth in SEQ ID NO: 20, and HCDR3 as set forth in SEQ ID NO: 21; (2) HCDR1 as set forth in SEQ ID NO: 23, HCDR2 as set forth in SEQ ID NO: 24, and HCDR3 as set forth in SEQ ID NO: 25; (3) HCDR1 as set forth in SEQ ID NO: 27, HCDR2 as set forth in SEQ ID NO: 28, and HCDR3 as set forth in SEQ ID NO: 29; (4) HCDR1 as set forth in SEQ ID NO: 31, HCDR2 as set forth in SEQ ID NO: 32, and HCDR3 as set forth in SEQ ID NO: 33; and (5) HCDR1 as set forth in SEQ ID NO: 35, HCDR2 as set forth in SEQ ID NO: 36 and HCDR3 as set forth in SEQ ID NO: 37.

In some embodiments, the VH of the third antigen-binding portion comprises an amino acid sequence as set forth in SEQ ID NO: 18, 22, 26, 30 or 34, or a sequence having at least 80% sequence identity to the amino acid sequence as set forth in SEQ ID NO: 18, 22, 26, 30 or 34. In some embodiments, the third antigen-binding portion is a single domain antibody.

In some embodiments, the third antigen-binding portion has the same HCDR1, HCDR2, and HCDR3 as the first antigen-binding portion. In some embodiments, the third antigen-binding portion has the same VH as the first antigen-binding portion.

In some embodiments, the third antigen-binding portion is fused to the N-terminus of the first antigen-binding portion or to the N-terminus of the Fab heavy chain of the second antigen-binding portion. In some embodiments, the third antigen-binding portion is fused to the N-terminus of the first antigen-binding portion or the N-terminus of the Fab heavy chain of the second antigen-binding portion via a peptide linker, preferably, the sequence of the peptide linker is (GGGGS)n, wherein n is an integer of not less than 1.

In some embodiments, the bispecific antibody consists of a first chain, a second chain and a third chain. The first chain comprises a fusion protein of the first antigen-binding portion and the first Fc fragment, the second chain comprises a fusion protein of the Fab heavy chain of the second antigen-binding portion and the second Fc fragment, the third chain comprises an Fab light chain of the second antigen-binding portion. In some embodiments, the first chain comprises an amino acid sequence as set forth in SEQ ID NO: 1, 4, 5, 6 or 7, or a sequence having at least 80% sequence identity to the amino acid sequence as set forth in SEQ ID NO: 1, 4, 5, 6 or 7, the second chain comprises an amino acid sequence as set forth in SEQ ID NO: 2, or a sequence having at least 80% sequence identity to the amino acid sequence as set forth in SEQ ID NO: 2, the third chain comprises an amino acid sequence as set forth in SEQ ID NO: 3, or a sequence having at least 80% sequence identity to the amino acid sequence as set forth in SEQ ID NO: 3.

In some embodiments, the bispecific antibody consists of a first chain, a second chain and a third chain. The first chain comprises a fusion protein of the third antigen-binding portion, the first antigen-binding portion and the first Fc fragment, the second chain comprises a fusion protein of the Fab heavy chain of the second antigen-binding portion and the second Fc fragment, the third chain comprises an Fab light chain of the second antigen-binding portion. In some embodiments, the first chain comprises an amino acid sequence as set forth in SEQ ID NO: 8, 9, 10, 11 or 12, or a sequence having at least 80% sequence identity to the amino acid sequence as set forth in SEQ ID NO: 8, 9, 10, 11 or 12, the second chain comprises an amino acid sequence as set forth in SEQ ID NO: 2, or a sequence having at least 80% sequence identity to the amino acid sequence as set forth in SEQ ID NO: 2, the third chain comprises an amino acid sequence as set forth in SEQ ID NO: 3, or a sequence having at least 80% sequence identity to the amino acid sequence as set forth in SEQ ID NO: 3.

In some embodiments, the bispecific antibody consists of a first chain, a second chain and a third chain. The first chain comprises a fusion protein of the first antigen-binding portion and the first Fc fragment, the second chain comprises a fusion protein of the third antigen-binding portion, the Fab heavy chain of the second antigen-binding portion and the second Fc fragment, the third chain comprises the Fab light chain of the second antigen-binding portion. In some embodiments, the first chain comprises an amino acid sequence as set forth in SEQ ID NO: 1, 4, 5, 6 or 7, or a sequence having at least 80% sequence identity to the amino acid sequence as set forth in SEQ ID NO: 1, 4, 5, 6 or 7, the second chain comprises an amino acid sequence as set forth in SEQ ID NO: 13, 14, 15, 16 or 17, or a sequence having at least 80% sequence identity to the amino acid sequence as set forth in SEQ ID NO: 13, 14, 15, 16 or 17, the third chain comprises an amino acid sequence as set forth in SEQ ID NO: 3, or a sequence having at least 80% sequence identity to the amino acid sequence as set forth in SEQ ID NO: 3. In some embodiments, the first chain, the second chain and the third chain are selected from the following groups: (1) the first chain comprises an amino acid sequence as set forth in SEQ ID NO: 1, or a sequence having at least 80%, at least 85%, at least 90%, at least 95%, at least 96%, at least 97%, at least 98% or at least 99% sequence identity to the amino acid sequence as set forth in SEQ ID NO: 1, the second chain comprises the amino acid sequence as set forth in SEQ ID NO: 13, or a sequence having at least 80%, at least 85%, at least 90%, at least 95%, at least 96%, at least 97%, at least 98% or at least 99% sequence identity to the amino acid sequence as set forth in SEQ ID NO: 13, and the third chain comprises the amino acid sequence as set forth in SEQ ID NO: 3, or a sequence having at least 80%, at least 85%, at least 90%, at least 95%, at least 96%, at least 97%, at least 98% or at least 99% sequence identity to the amino acid sequence as set forth in SEQ ID NO: 3; (2) the first chain comprises an amino acid sequence as set forth in SEQ ID NO: 4, or a sequence having at least 80% sequence identity to the amino acid sequence as set forth in SEQ ID NO: 4, the second chain comprises an amino acid sequence as set forth in SEQ ID NO: 14, or a sequence having at least 80% sequence identity to the amino acid sequence as set forth in SEQ ID NO: 14, and the third chain comprises an amino acid sequence as set forth in SEQ ID NO: 3, or a sequence having at least 80% sequence identity to the amino acid sequence as set forth in SEQ ID NO: 3; (3) the first chain comprises an amino acid sequence as set forth in SEQ ID NO: 5, or a sequence having at least 80% sequence identity to the amino acid sequence as set forth in SEQ ID NO: 5, the second chain comprises an amino acid sequence as set forth in SEQ ID NO: 15, or a sequence having at least 80% sequence identity to the amino acid sequence as set forth in SEQ ID NO: 15, and the third chain comprises an amino acid sequence as set forth in SEQ ID NO: 3, or a sequence having at least 80% sequence identity to the amino acid sequence as set forth in SEQ ID NO: 3; (4) the first chain comprises an amino acid sequence as set forth in SEQ ID NO: 6, or a sequence having at least 80% sequence identity to the amino acid sequence as set forth in SEQ ID NO: 6, the second chain comprises an amino acid sequence as set forth in SEQ ID NO: 16, or a sequence having at least 80% sequence identity to the amino acid sequence as set forth in SEQ ID NO: 16, the third chain comprises an amino acid sequence as set forth in SEQ ID NO: 3, or a sequence having at least 80% sequence identity to the amino acid sequence as set forth in SEQ ID NO: 3; and (5) the first chain comprises an amino acid sequence as set forth in SEQ ID NO: 7, or a sequence having at least 80% sequence identity to the amino acid sequence as set forth in SEQ ID NO: 7, the second chain comprises an amino acid sequence as set forth in SEQ ID NO: 17, or a sequence having at least 80% sequence identity to the amino acid sequence as set forth in SEQ ID NO: 17, and the third chain comprises an amino acid sequence as set forth in SEQ ID NO: 3, or a sequence having at least 80% sequence identity to the amino acid sequence as set forth in SEQ ID NO: 3.

Another aspect of the present invention provides a polynucleotide encoding any one of the aforementioned bispecific antibodies, a recombinant expression vector comprising the polynucleotide, and a host cell comprising the polynucleotide or the recombinant expression vector.

Another aspect of the present invention provides a pharmaceutical composition, comprising any one of the aforementioned bispecific antibodies, the aforementioned polynucleotide, the aforementioned recombinant expression vector or the aforementioned host cell, and a pharmaceutically acceptable carrier.

Another aspect of the present invention provides the use of any one of the aforementioned bispecific antibodies, the aforementioned polynucleotide, the aforementioned recombinant expression vector or the aforementioned host cell in the preparation of a drug for treating a disease associated with ROR1. In some embodiments, the disease associated with ROR1 is a tumour or cancer.

Another aspect of the present invention provides a method for treating a disease associated with ROR1, including administering to a subject in need thereof any one of the aforementioned bispecific antibodies, the aforementioned polynucleotide, the aforementioned recombinant expression vector, the aforementioned host cell or the aforementioned pharmaceutical composition. In some embodiments, the disease associated with ROR1 is a tumour or cancer.

### Brief Description of the Drawings

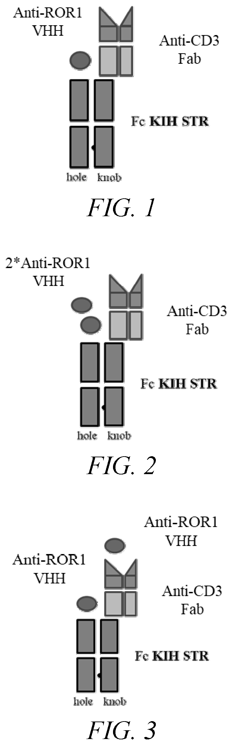

FIG. 1: Configuration diagram of 1+1 bispecific antibody.
FIG. 2: Configuration diagram of 2+1 bispecific antibody.
FIG. 3: Configuration diagram of 1+1+1 bispecific antibody.
FIG. 4: Detection of binding of 1+1 configuration bispecific antibody to overexpressing cell line CHO-K1/ROR1.
FIG. 5: Detection of binding of 2+1 configuration bispecific antibody to overexpressing cell line CHO-K1/ROR1.
FIG. 6: Detection of binding of 1+1+1 configuration bispecific antibody to overexpressing cell line CHO-K1/ROR1.
FIG. 7: Detection of binding of 1+1 configuration bispecific antibody to CD3 expressing cell line Jurkat.
FIG. 8: Detection of binding of 2+1 configuration bispecific antibody to CD3 expressing cell line Jurkat.
FIG. 9: Detection of binding of 1+1+1 configuration bispecific antibody to CD3 expressing cell line Jurkat.
FIG. 10A: SPR analysis of the CD3×ROR1 bispecific antibody BsAb-12.
FIG. 10B: SPR analysis of the CD3×ROR1 bispecific antibody BsAb-9.
FIG. 10C: SPR analysis of control antibody NVG-11.
FIG. 11: Detection of cell binding capacity of CD3×ROR1 bispecific antibody.
FIG. 12: Detection of T cell activation mediated by CD3×ROR1 bispecific antibody based on reporter gene.
FIG. 13: Detection of tumour cell killing by PBMC (Donor: NF0058) mediated by CD3×ROR1 bispecific antibody.
FIG. 14: Detection of tumour cell killing by PBMC (Donor: Y1701) mediated by CD3×ROR1 bispecific antibody.
FIG. 15: Experimental results of release of IFN-γ, TNF-α, IL-2 and IL-6 from PBMC (Donor: NF0058) mediated by CD3×ROR1 bispecific antibody.
FIG. 16: Experimental results of release of IFN-γ and TNF-α from PBMC (Donor: Y1701) mediated by CD3×ROR1 bispecific antibody.
FIG. 17: Experimental results of the anti-tumour in vivo efficacy of CD3×ROR1 bispecific antibody in C-NKG mice transplanted with MDA-MB-231 breast cancer cells and human PBMC.

### Detailed Description of Embodiments

Unless otherwise indicated, the terms used in the present invention have the meanings commonly understood in the art and can be understood by reference to standard textbooks, reference books, and literature known to those skilled in the art. All publications mentioned herein are incorporated herein by reference in their entirety.

It should be understood that the specific methods and materials described in the examples of the present invention are merely for the purpose of describing the specific examples and are not intended to be limiting. Any methods and materials similar to or equivalent to those described herein may be used in the practice or testing of the present invention. The explanations of relevant theories or mechanisms in the present invention is merely for the purpose of facilitating the understanding of the invention and should not be construed as limiting the solutions claimed by the present invention.

The term "comprise" used herein, and other synonymous terms such as "include", "contain" or "have", should be understood to include the listed elements, but not to exclude the presence of other elements. These terms also include the case of consisting of the listed elements only. The term "consist of" means consisting of the listed elements only. The term "essentially consist of" means that elements that do not significantly affect the novel features of the involved solutions are not excluded.

Unless otherwise specifically stated, "a", "an", or "the" herein includes both the singular and plural forms. The meaning of the terms "at least one" or "at least one kind" or other similar expressions, is equivalent to the meaning of "one or more" or "one or more kinds".

The numerical ranges described herein, such as temperature ranges, time ranges, composition or concentration ranges, or other numerical ranges, include end values within the ranges, all intermediate ranges, subranges (e.g., ranges between a certain intermediate value and a certain end value), and all individual numerical values, particularly intermediate ranges and subranges with integers as end values, and individual integer values. Moreover, any intermediate ranges, subranges, and all individual values described in the numerical ranges, may be excluded from the numerical ranges.

As used herein, the terms "about" and "approximately" mean a range of +10% of the stated numerical value.

As used herein, "and/or" should be understood as referring to any one of the multiple elements modified by this term, or any combination of several of these elements.

As used herein, "first" and "second" are merely for the purpose of distinction and do not imply a specific order.

In the present invention, unless otherwise stated, the description of nucleic acid sequences is typically from the 5' end to the 3' end, and the description of amino acid sequences is typically from the N-terminus to the C-terminus.

As used herein, when referring to a polypeptide being fused to the N-terminus of another polypeptide, it means that the C-terminus of the former is linked to the N-terminus of the latter via a peptide bond or a peptide linker.

### Definitions

Unless otherwise explicitly stated, the practice of the present disclosure will employ conventional methods of virology, immunology, microbiology, molecular biology, and recombinant DNA technology within the skill of the art, many of which will be described below for illustrative purposes. Such techniques are fully described in the literature, see, e.g., Current Protocols in Molecular Biology or Current Protocols in Immunology, John Wiley&Sons, New York, N.Y., 2009; Frederick M. Ausubel et al., Short Protocols in Molecule Biology, 4th Edition, Wiley, 1999; Michael R. Green and Joseph Sambrook, Molecule Cloning: A Laboratory Manual, 4th Edition, 2012 and other similar references.

The terms "nucleic acid molecule", "nucleic acid" and "polynucleotide" are used interchangeably and refer to a sequence composed of bases, sugars and a phosphate backbone linked together. The nucleic acid sequence of the present invention may be a deoxyribonucleic acid (DNA) sequence or a ribonucleic acid (RNA) sequence, and may include natural bases or non-natural bases, which may be single-stranded or double-stranded, and may be a coding sequence or a noncoding sequence.

The terms "polypeptide" and "protein" are used interchangeably and refer to a polymer of amino acid residues. Such polymers may contain natural or unnatural amino acid residues and include, but are not limited to, peptides, oligopeptides, dimers, trimers, and multimers composed of amino acid residues. Both full-length proteins and fragments thereof are encompassed by this definition. The term also includes polypeptides having post-expression modifications, such as glycosylation, sialylation, acetylation, phosphorylation and similar modifications.

The term "isolated protein" or "isolated polypeptide" refers to such a protein or polypeptide that: (1) it is not associated with the naturally associated components that accompanies it in its native state; (2) it does not contain other proteins that are not derived from the same species; (3) it is expressed by cells from different species; or (4) it does not occur naturally. A polypeptide chemically synthesized or synthesized in a cellular system that is different from the cell in which the peptide originates, will be "isolated" from the component with which it is naturally associated. Isolation may also be performed by using the protein purification techniques well known in the art, such that the protein is substantially free of naturally associated components. Proteins or polypeptides referred to in the present invention, such as antibodies or antigen-binding fragments thereof, are preferably isolated unless otherwise stated.

The term "isolated nucleic acid" refers to a nucleic acid molecule of a genome, cDNA, or synthetic source, or a combination thereof, which is separated from other nucleic acid molecules present in the natural source of the nucleic acid. For genomic DNA, the term "isolated" includes nucleic acid molecules that are isolated from chromosomes naturally associated with the genomic DNA. Preferably, an "isolated" nucleic acid does not include sequences naturally flanking the nucleic acid (i.e., sequences located at the 5' and 3' ends of the nucleic acid of interest). The polynucleotides or nucleic acid sequences referred to in the present invention are preferably isolated.

The term "CD3" refers to the cluster of differentiation 3 protein, which is an important differentiation antigen on the T cell membrane. In mammals, CD3 is composed of four different chains, comprising one CD3γ chain, one CD3δ chain and two CD3ε chains. These chains associate with T cell receptor (TCR) and CD3-zeta (ζ chain) to form a TCR complex, which produces activation signals in T lymphocytes that plays an extremely important role in the process of T cell antigen recognition and the immune response. The term "CD3" also includes any variants, isoforms, derivatives, and homologs of different species of CD3, which may be expressed naturally by cells, or expressed in cells containing exogenous or heterologous nucleic acid sequences encoding CD3 via gene recombination technology. CD3 herein may refer to CD3 proteins from any vertebrate source, including mammals such as primates (e.g., humans), non-human primates (e.g., macaques) and rodents (e.g., mice and rats), particularly human CD3.

The term "ROR1" refers to receptor tyrosine kinase-like orphan receptor 1. ROR1 is an evolutionarily conserved membrane protein that is widely expressed in embryonic development and in a variety of human cancers. In terms of molecular structure, ROR1 includes an extracellular domain composed of an Ig-like domain, a Frizzled domain, and a juxtamembrane Kringle domain; a transmembrane domain; and a cytoplasmic domain composed of a tyrosine kinase-like domain, two serine/threonine-rich domains, and a proline-rich domain (PRD). ROR1 has become a promising target for tumour therapy due to its expression pattern and function in tumour progression. The term "ROR1" also includes any variants, isoforms, derivatives, and homologs of different species of ROR1, which may be expressed naturally by cells, or expressed in cells containing exogenous or heterologous nucleic acid sequences encoding ROR1 via gene recombination technology. ROR1 herein may refer to ROR1 proteins from any vertebrate source, including mammals such as primates (e.g., humans), non-human primates (e.g., macaques) and rodents (e.g., mice and rats), particularly human ROR1.

The term "antigen" has the meaning commonly understood by those of ordinary skill in the art and includes polypeptides, polysaccharides, glycoproteins, polynucleotides and the like. The term "antigenic epitope", also referred to as "antigenic determinant", refers to a chemical functional group on the surface of an antigen that determines the antigenic specificity. The antigenic epitope can be recognized and bound by an antigen-binding site in an antibody.

The term "antibody" is used in its broadest sense and includes immunoglobulins or other types of molecules that comprise one or more antigen-binding domains that specifically bind to an antigen, and refers to a protein or polypeptide that exhibits binding specificity for a particular antigen. Specific examples of antibodies may include intact antibodies (e.g., classical four-chain antibody molecules), single chain antibodies, single domain antibodies, multispecific antibodies, etc. An intact antibody, which in the present invention may also be referred to as a full-length antibody or a classical antibody molecule, is an immunoglobulin molecule composed of four polypeptide chains (e.g., IgG) or a polymer thereof (e.g., IgA or IgM). The four polypeptide chains include two identical heavy chains (H) and two identical light chains (L), which are interconnected by disulfide bonds to form a tetramer. Each heavy chain is composed of a heavy chain variable region ("HCVR" or "VH") and a heavy chain constant region (CH, including domains CH1, CH2 and CH3). Each light chain is composed of a light chain variable region ("LCVR or "VL") and a light chain constant region (CL). The constant regions of the heavy chain and the light chain (CH and CL) are not directly involved in the binding of an antibody to an antigen, but exhibit a variety of effector functions, such as mediating the binding of the antibody to host tissues or factors, including various cells of the immune system (e.g. effector cells) and the first component (C1q) of the classical complement system. The variable regions of the heavy chain and the light chain (VH and VL) form an antigen-binding site. The VH and the VL each have a high-variable region known as the complementarity determining region (CDR), whose amino acid composition and arrangement order have a high degree of variability, serving as the key positions for the binding of antibodies to antigens, and a more conserved sequence called framework region (FR) is interspersed between CDRs. Each VH and VL is composed of three CDRs and four FRs arranged in the following order from the amino terminus to the hydroxyl terminus: FR1, CDR1, FR2, CDR2, FR3, CDR3, FR4. Herein, the three heavy chain complementarity determining regions are referred to as HCDR1 (CDR-H1), HCDR2 (CDR-H2) and HCDR3 (CDR-H3), respectively, and the four heavy chain framework regions are referred to as HFR1 (FR-H1), HFR2 (FR-H2), HFR3 (FR-H3) and HFR4 (FR-H4), respectively; the three light chain complementarity determining regions are referred to as LCDR1 (CDR-L1), LCDR2 (CDR-L2), and LCDR3 (CDR-L3), respectively, and the four light chain framework regions are referred to as LFR1 (FR-L1), LFR2 (FR-L2), LFR3 (FR-L3), and LFR4 (FR-L4), respectively. The variable regions of the heavy chain and the light chain (VH and VL) form an antigen-binding site.

The term "complementarity determining region" or "CDR" refers to the amino acid residues in the variable region of an antibody responsible for antigen binding. The precise boundary of the CDR may be defined in accordance with various numbering systems known in the art, for example, in accordance with the Kabat numbering system (Kabat et al., Sequences of Proteins of Immunological Interest, 5th Ed. Public Health Service, National Institutes of Health, Bethesda, Md., 1991), Chothia numbering system (Chothia & Lesk (1987)J. Mol. Biol. 196:901-917; Chothia et al.,(1989) Nature 342:878-883), IMGT numbering system (Lefranc et al., Dev. Comparat. Immunol. 27:55-77, 2003), AbM numbering system or Contact numbering system. For a given antibody, those skilled in the art will readily identify the CDRs as defined in accordance with each numbering system. Moreover, the correspondence between different numbering systems is well known to those skilled in the art (for example, see Lefranc et al., Dev. Comparat. Immunol. 27:55-77, 2003). Although the CDRs are defined using the Kabat numbering system in the examples of the present invention and in the description of specific sequences, it should be understood that the antibodies of the present invention may use any of these numbering systems to define the CDRs.

The term "framework region" or "FR" refers to the amino acid sequences within the antibody variable region other than the CDR sequences as defined above.

Based on the amino acid sequence of the antibody heavy chain constant region, antibodies can be classified into five main different types: IgA, IgD, IgE, IgG and IgM. These antibody types can be further divided into subclasses based on differences in the size of the hinge region, the position of the interchain disulfide bonds and the molecular weight, for example, IgG1, IgG2a, IgG2b, IgG3 and IgG4. Based on differences in the amino acid composition and arrangement of the light chain constant region of the antibody, light chains can be classified into two types: κ and λ. Antibodies of the present invention include antibodies of any of the aforementioned classes or subclasses.

The term "monoclonal antibody" refers to an antibody that is homogeneous and directed against only to a specific antigenic epitope. Each monoclonal antibody is directed against a single antigenic epitope on an antigen, as compared to typical conventional polyclonal antibody formulations comprising different antibodies directed against different antigenic epitopes. Although monoclonal antibodies can be obtained by hybridoma culture, in the present invention, the modifier "monoclonal" indicates a homogeneous characteristic of the antibody and should not be construed as requiring the antibody to be produced by any specific method. The monoclonal antibodies of the present invention may be produced by recombinant DNA methods, or obtained by other screening methods.

The term "antigen-binding fragment" refers to one or more portions of an intact antibody that retain the ability to specifically bind to the antigenic epitope against which the antibody is directed, for example, see Fundamental Immunology, Ch.7 (Paul,W., ed., 2th Edition, Raven Press, N.Y.(1989). Examples of antigen-binding fragments include: (1) Fab fragment: An antigen-binding fragment produced by the digestion of a complete antibody with papain, composed of the intact light chain (including the VL domain and the CL domain) of a full-length antibody and the heavy chain variable region VH and heavy chain constant region domain CH1 of the full-length antibody, without the hinge region; (2) F(ab')₂ fragment: An antigen-binding fragment produced by the digestion of a complete antibody with pepsase, comprising the intact light chains (including the VL domain and the CL domain) of two full-length antibodies and the heavy chain variable region VH and heavy chain constant region domain CH1 and the hinge region of the two full-length antibodies, which can be regarded as a fragment formed by pairing two Fab' fragments with disulfide bonds therebetween; (3) Fab' fragment, which can be formed by treating F(ab')₂ with a reducing agent to cleave the interchain disulfide bonds between the hinge regions; (4) Fd fragment, composed of a VH domain and a CH1 domain; (5) Fv fragment, composed of the VL domain and the VH domain of the single arm of an antibody. The term "antigen-binding fragment" typically comprises one of the heavy chain variable region and the light chain variable region of an intact antibody or both, and may further comprise a portion or all of the light chain constant region and/or the heavy chain constant region of an intact antibody, for example, one or two or more of the CL domain, the CH1 domain, the CH2 domain, the CH3 domain.

The term "Fc region" or "Fc fragment" refers to the C-terminal region of the immunoglobulin heavy chain, i.e. the fragment crystallizable (Fc) that constitutes the stem region of the classical Y-shaped antibody molecule, including the second constant domain and the third constant domain (CH2 domain and CH3 domain) of the heavy chain. The Fc region can be obtained by hydrolysing an intact antibody molecule with papain. In some examples, the Fc region may comprise a hinge, CH2 and CH3. Dimerization between two Fc-containing polypeptides can be mediated when the Fc region comprises a hinge. The Fc fragment may be derived from IgG, IgM, IgD, IgE, or IgA. In some examples, the Fc region is derived from IgG1, IgG2, IgG3, or IgG4. The "Fc fragment" also includes a variant Fc fragment derived from a native Fc fragment, which has been modified but still retains its effector function. The "variant Fc fragment" comprises an amino acid sequence having at least one amino acid substitution in the amino acid sequence of the native Fc fragment. In some examples, the variant Fc fragment has at least one amino acid substitution as compared to the parental Fc fragment (native Fc fragment), for example, about 1 to about 10 amino acids are substituted in the parental Fc fragment, with preferably about 1 to about 5 amino acid substitutions. In some examples, the variant Fc fragment Fc region has at least about 80%, at least about 90%, at least about 95%, at least about 96%, at least about 97%, at least about 98% or at least about 99% sequence identity to the parental Fc fragment. Effector functions of the "Fc fragment" may include binding to an Fc receptor, Clq binding and complement-dependent cytotoxicity (CDC), antibody-dependent cell-mediated cytotoxicity (ADCC), mediation of phagocytosis and the like.

The term "single domain antibody (sdAb)" refers to an antigen-binding polypeptide having a single variable domain, which comprises three complementarity determining regions (CDRs). A single domain antibody is capable of binding to an antigen independently without requiring pairing with another polypeptide containing CDRs. Common single domain antibodies comprise a heavy chain but lack the light chain commonly found in antibodies, such as camelidae sdAb (see *e.g.,* Hamers-Casterman et al., Nature 363:446-8 (1993); Greenberg et al., Nature 374:168-73(1995); Hassanzadeh-Ghassabeh et al., Nanomedicine (Lond), 8:1013-26(2013)). The single domain antibody can be artificially engineered from heavy chain antibody of the camel, and referred to as V_{H}H antibody, which has the following structure from the N-terminus to the C-terminus: FR1-CDR1-FR2-CDR2-FR3-CDR3-FR4. The single domain antibody may also be considered as a special type of "antigen-binding fragment" of a full-length antibody.

The term "single-chain antibody (scFv)" refers to a single polypeptide chain comprising a VL domain and a VH domain, wherein the VL and VH are linked by a linker (see, e.g., Bird et al., Science 242:423-426 (1988); Huston et al., Proc. Natl. Acad. Sci. USA 85:5879-5883(1988); and Pluckthun, The Pharmacology of Monoclonal Antibodies, 113, Roseburg & Moore, Springer-Verlag, New York, 269-315 (1994)). Such scFv molecules may have a general structure: NH₂-VL-linker-VH-COOH or NH₂-VH-linker-VL-COOH. A suitable linker may be composed of a repeated GGGGS amino acid sequence, i.e., (GGGGS)n, or a variant thereof, wherein n is an integer greater than or equal to 1. For example, a linker with an amino acid sequence (GGGGS)₃, or a variant thereof (Holliger et al., (1993), Proc. Natl. Acad. Sci. USA 90:6444-6448) may be used. In some cases, a disulfide bond may also be present between the VH and the VL of the scFv.

The term "chimeric antibody" refers to an antibody, in which a portion of the light chain or/and heavy chain is derived from one antibody (the antibody may be derived from a particular species or belong to a specific antibody class or subclass), and another portion of the light chain or/and heavy chain is derived from another antibody (the antibody may be derived from the same or different species or belong to the same or different antibody classes or subclasses), while still retaining binding activity to a target antigen (Morrison et al., Proc. Natl. Acad. Sci. USA, 81:6851-6855(1984)). The term "chimeric antibody" may refer to an antibody comprising a heavy chain variable region sequence and a light chain variable region sequence from one species and a constant region sequence from another species, for example, an antibody with a human constant region chain and a murine heavy chain variable region and light chain variable region.

The term "human antibody" refers to an antibody in which the entire sequence (for example, variable regions and constant regions) of the antibody is derived from a human immunoglobulin. A "non-human antibody" refers to an antibody in which the entire sequence (e.g., variable regions and constant regions) of the antibody is derived from an immunoglobulin of a non-human species, for example, an antibody derived from a murine immunoglobulin may be referred to as a murine antibody.

The term "humanized antibody" refers to a chimeric antibody obtained by modifying a non-human antibody, such as an antibody whose variable regions and constant regions, if present, are not derived from a human immunoglobulin, to increase its homology to a human antibody sequence. Generally, all or part of the CDR regions of a humanized antibody are derived from a non-human antibody (donor antibody), and all or part of the non-CDR regions (e.g., variable region FR and/or constant region) are derived from a human immunoglobulin (recipient antibody). A humanized antibody typically retains the expected properties of a donor antibody, including but not limited to, antigen specificity, affinity, reactivity, ability to improve immune cell activity, ability to enhance immune responses and the like. The donor antibody may be a mouse, rat, rabbit or non-human primate (e.g., cynomolgus monkey) antibody having the expected properties (e.g., antigen specificity, affinity, reactivity, ability to improve immune cell activity and/or ability to enhance immune responses). Humanized antibodies are particularly advantageous because they can both retain the expected properties of non-human donor antibodies (e.g., murine antibodies) and effectively reduce the immunogenicity of non-human donor antibodies (e.g., murine antibodies) in human subjects. However, due to matching issues between the CDRs of the donor antibody and the FRs of the receptor antibody, the expected properties of humanized antibodies (e.g., antigen specificity, affinity, reactivity, ability to improve immune cell activity and/or ability to enhance immune responses) are typically lower than those of non-human donor antibodies (e.g., murine antibodies).

The term "bispecific antibody" refers to an antibody capable of specifically binding to two antigenic epitopes. The two antigenic epitopes may be different epitopes on the same antigen or different epitopes on different antigens. The bispecific antibody comprises two or more antigen-binding portions, wherein one or more antigen-binding portions target one antigen epitope and the other antigen-binding portions target another antigen epitope. Each antigen-binding portion may be independently selected from a monoclonal antibody (e.g., an IgG immunoglobulin), an Fab fragment, an Fab' fragment, an F(ab')₂ fragment, an Fv fragment, an Fd fragment, scFv, a single domain antibody, a chimeric antibody, a humanized antibody and the like. An antigen-binding portion may be functionally linked (e.g., by chemical conjugation, gene fusion, non-covalent binding or otherwise) to one or more additional antigen-binding portions to form a bispecific antibody. As used herein, "ROR1×CD3 bispecific antibody" refers to a bispecific antibody that targets ROR1 and CD3.

As is known to those skilled in the art, antibodies can be prepared by a variety of techniques, such as hybridoma technology (see, e.g., Kohler et al., Nature, 256:495, 1975), recombinant DNA technology (see, e.g., U.S. patent application 4,816,567), or phage antibody library technology (see, e.g., Clackson et al., Nature, 352:624-628,1991, or Marks et al., J. Mol. Biol. 222:581-597, 1991).

As is known to those skilled in the art, antibodies can be purified by well-known techniques, such as affinity chromatography with Protein A or Protein G. Subsequently or alternatively, the specific antigen (the target molecule recognized by the antibody) or its antigenic epitope can be immobilized on a column, and the immunospecific antibody can be purified by immunoaffinity chromatography. Purification of antibodies can be carried out by reference to, for example, D.Wilkinson, The Scientist, published by The Scientist, Inc., Philadelphia PA, Vol.14, No.8 (Apr.17,2000), pp.25-28).

With respect to an antibody or an antigen-binding fragment thereof, the term "target", "directed against", or "specifically bind" means that the antibody or antigen-binding fragment thereof forms a relatively stable complex with an antigen under physiological conditions and preferably does not exhibit significant binding to other undesired antigens. A molecule may target, be directed against, or specifically bind to one or more molecules. For example, a bispecific antibody may have a higher binding affinity for two different antigens relative to other molecules. The ability of an antibody or an antigen-binding fragment thereof to bind to an antigen or an antigenic epitope can be determined by technologies, for example, enzyme-linked immunosorbent assay (ELISA), surface plasmon resonance technology (SPR), or other protein interaction assay methods such as biolayer interferometry (BLI). The binding specificity may be characterized by the equilibrium dissociation constant KD of antigen-antibody binding (a smaller KD indicates a tighter binding), and may also be characterized by the EC50 value of antibody-antigen binding.

The term "EC50 (concentration for 50% of maximal effect)" refers to the concentration that elicits 50% of the maximal effect. When used in an enzyme-linked immunosorbent assay (ELISA) to indicate the binding ability of an antibody molecule to a corresponding antigen, it may refer to the concentration of the antibody molecule at which half of the maximum detectable signal (e.g., colorimetric or fluorescent intensity) is produced. The lower the EC50 value, the greater the binding affinity of the antibody to the antigen.

The term "ka" refers to the association rate constant of a specific antibody-antigen interaction, indicating the association rate of an antibody to its target antigen, or the complex formation rate of an antibody and an antigen, in the unit of M⁻¹s⁻¹.

The term "kd" refers to the dissociation rate constant of a specific antibody-antigen interaction, indicating the dissociation rate of an antibody from its target antigen, or the dissociation rate at which an antigen-antibody complex separates into free antibody and antigen over time, in the unit of s ⁻¹.

The term "KD" refers to the equilibrium dissociation constant of a specific antibody-antigen interaction, which is derived from the ratio of kd to ka (i.e. kd/ka), expressed as molar concentration (M). KD can be used to measure the binding affinity of an antibody for its binding ligand (e.g., an antigen). A smaller KD indicates a tighter binding of the antibody to the antigen, or a higher affinity of the antibody for the antigen. For example, an antibody with a nanomolar (nM)-level equilibrium dissociation constant binds more tightly to a specific antigen than an antibody with a micromolar (µM) dissociation constant. The KD value of an antibody can be determined using methods well known in the art. One method for determining the ka, kd and KD values of an antibody is to use surface plasmon resonance, typically measured using biosensor systems such as Biacore^{™} system. Another method for determining the KD value of an antibody is biolayer interferometry (BLI) technology.

The term "fusion protein" means that two or more polypeptide portions are covalently linked together to form one polypeptide. Two or more polypeptide portions may be covalently linked by a peptide bond, or linked together by a peptide linker.

The term "variant" or "mutant" refers to a polypeptide comprising an amino acid sequence that differs by at least one amino acid residue from that of a parental or reference polypeptide (including, but not limited to, wild-type polypeptides). The variant may have one or more insertions, deletions or substitutions of amino acid sequences as compared to the parental polypeptide.

The term "sequence identity" refers to the percentage of nucleotides or amino acid residues that are identical at corresponding positions when two or more sequences are aligned in a manner that maximizes matching, i.e., taking into account the gaps and insertions. Sequence alignment and calculation of sequence identity percentages can be performed using suitable computer programs known in the art. Such programs include local alignment programs and global alignment programs, including but not limited to BLAST, ALIGN, ClustalW, EMBOSS Needle and the like. An example of local alignment programs is BLAST (Basic Local Alignment Search Tool) (see, e.g., Altschul et al., (1990) J. Mol. 215; 403-410), which is available from the National Center for Biotechnology Information website at http://www.ncbi.nlm.nih.gov/. Examples of global alignment programs (optimizing alignment over full-length sequences) are the EMBOSS Needle and EMBOSS Stretcher programs based on the Needleman-Wunsch algorithm (Needleman, Saul B.; and Wunsch, Christian D. (1970),"A general method applicable to the search for similarities in the amino acid sequence of two proteins", Journal of Molecular Biology 48(3):443-53) , all of which are available from http://www.ebi.ac.uk/Tools/psa/.

In variants of the present invention, the form of XnnnY is used to represent an amino acid mutation in the polypeptide. X is the amino acid before the mutation, Y is the amino acid after the mutation and nnn represents the position of the mutation, as determined according to the EU index numbering of Kabat. Amino acids are represented by conventional single-letter or three-letter designations. As is well known to those skilled in the art, the single-letter designations for amino acids are as follows: A represents alanine; C represents cysteine; D represents aspartic acid; E represents glutamic acid; F represents phenylalanine; G represents glycine; H represents histidine; I represents isoleucine; K represents lysine; L represents leucine; M represents methionine; N represents asparagine; P represents proline; Q represents glutamine; R represents arginine; S represents serine; T represents threonine; V represents valine; W represents tryptophan; and Y represents tyrosine.

The term "recombinant" refers to the expression of antibodies or antigen-binding fragments thereof of the present invention by recombinant DNA technology (which includes, for example, DNA splicing and transgene expression), for example, using a recombinant expression vector expression system transfected into non-human mammals (e.g., mice) or a cell (e.g., CHO cells). The antibodies may be antibodies of a recombinant or combinatorial human antibody library or isolated from a recombinant or combinatorial human antibody library.

The term "vector" refers to any molecule, such as a nucleic acid, a plasmid, or a virus, which is capable of transporting a heterologous nucleic acid contained therein into a host cell (existing in a free form or integrated into the host cell genome). The vector may exist independently and replicate autonomously after being introduced into a host cell (e.g. a bacterial vector having a bacterial origin of replication and an episomal mammalian vector), or be integrated into the host cell genome and replicate together with the host genome (e.g. a non-episomal mammalian vector). A vector for expressing a gene of interest in a host cell is referred to as an "expression vector" or a "recombinant expression vector". The vector may comprise a regulatory element operably linked to the gene of interest, such as an origin of replication, an expression regulatory sequence (e.g. a promoter and/or an enhancer), and/or a selectable marker gene (e.g. an antibiotic resistance gene and a gene useful in colorimetric assays, such as β-galactose).

To express the antibodies or antigen-binding fragments thereof of the present invention, expression vectors encoding the heavy chains and light chains can be transfected into host cells by standard techniques. The term "transfection" is intended to include a variety of techniques commonly used to introduce exogenous DNA into prokaryotic or eukaryotic host cells, such as electroporation, calcium phosphate precipitation, DEAE-dextran transfection and the like. Although it is theoretically possible to express the antibodies of the invention in prokaryotic or eukaryotic host cells, expression of the antibodies in eukaryotic cells, most preferably mammalian host cells, is preferable because such eukaryotic cells, particularly mammalian cells, are more likely than prokaryotic cells to assemble and secrete correctly folded immunologically active antibodies.

The term "host cell" refers to a cell that can be or has been a recipient of a vector or isolated polynucleotide. The host cell may be a prokaryotic cell or a eukaryotic cell. Exemplary eukaryotic cells include mammalian cells, such as primate or non-primate cells, e.g., human cells; fungal cells, such as yeast; plant cells; and insect cells. Non-limiting exemplary mammalian cells include, but are not limited to, CHO cells, HEK-293 cells, BHK cells or PER-C6 cells, and derived cells thereof, such as 293-6E, CHO-DG44, CHO-Kl, CHO-S and CHO-DS cells. The host cell includes the progeny of a single host cell, and the progeny may not be completely identical to the original parental cell due to natural, accidental, or deliberate mutations, for example in the aspect of morphology or genomic DNA complementarity. The host cell may be an isolated cell or a cell line, and also includes a cell transfected in vivo with a nucleic acid molecule or an expression vector provided herein.

### Bispecific Antibody Targeting ROR1 and CD3

The present invention provides a bispecific antibody targeting ROR1 and CD3, particularly bispecific antibody targeting human ROR1 and human CD3. The bispecific antibody comprises at least a first antigen-binding portion and a second antigen-binding portion, wherein the first antigen-binding portion specifically binds to ROR1, in particular human ROR1, and the second antigen-binding portion specifically binds to CD3, in particular human CD3.

The first antigen-binding portion may be an antibody or antigen-binding fragment thereof that specifically binds to the ROR1 protein (or the Frizzled domain or Kringle domain thereof), and may be, for example, an Fab, an Fv, a single-chain antibody (scFv) or a single domain antibody (sdAb) and the like. In some embodiments, the first antigen-binding portion is a single domain antibody.

In some embodiments, the first antigen-binding portion includes a heavy chain variable region VH. The VH comprises HCDR1, HCDR2 and HCDR3. The HCDR1, HCDR2 and HCDR3 may be selected from the following groups:
(1) HCDR1 as set forth in SEQ ID NO: 19, HCDR2 as set forth in SEQ ID NO: 20, and HCDR3 as set forth in SEQ ID NO: 21;
(2) HCDR1 as set forth in SEQ ID NO: 23, HCDR2 as set forth in SEQ ID NO: 24, and HCDR3 as set forth in SEQ ID NO: 25;
(3) HCDR1 as set forth in SEQ ID NO: 27, HCDR2 as set forth in SEQ ID NO: 28, and HCDR3 as set forth in SEQ ID NO: 29;
(4) HCDR1 as set forth in SEQ ID NO: 31, HCDR2 as set forth in SEQ ID NO: 32, and HCDR3 as set forth in SEQ ID NO: 33; and
(5) HCDR1 as set forth in SEQ ID NO: 35, HCDR2 as set forth in SEQ ID NO: 36 and HCDR3 as set forth in SEQ ID NO: 37.

In some embodiments, the VH of the first antigen-binding portion comprises an amino acid sequence as set forth in SEQ ID NO: 18, 22, 26, 30 or 34, or a sequence having at least 80%, at least 85%, at least 90%, at least 95%, at least 96%, at least 97%, at least 98% or at least 99% sequence identity to the amino acid sequence as set forth in SEQ ID NO: 18, 22, 26, 30 or 34.

The second antigen-binding portion may be an antibody or an antigen-binding fragment thereof that specifically binds to CD3, and may be, for example, an Fab, an Fv, a single-chain antibody (scFv) or a single domain antibody (sdAb) and the like. In some embodiments, the second antigen-binding portion is an Fab.

In some embodiments, the second antigen-binding portion comprises a heavy chain variable region VH and a light chain variable region VL. The VH comprises HCDR1 as set forth in SEQ ID NO: 39, HCDR2 as set forth in SEQ ID NO: 40, and HCDR3 as set forth in SEQ ID NO: 41. The VL comprises LCDR1 as set forth in SEQ ID NO: 43, LCDR2 as set forth in SEQ ID NO: 44, and LCDR3 as set forth in SEQ ID NO: 45.

In some embodiments, the VH of the second antigen-binding portion comprises an amino acid sequence as set forth in SEQ ID NO: 38, or a sequence having at least 80%, at least 85%, at least 90%, at least 95%, at least 96%, at least 97%, at least 98% or at least 99% sequence identity to the amino acid sequence as set forth in SEQ ID NO: 38.

In some embodiments, the VL of the second antigen-binding portion comprises an amino acid sequence as set forth in SEQ ID NO: 42, or a sequence having at least 80%, at least 85%, at least 90%, at least 95%, at least 96%, at least 97%, at least 98% or at least 99% sequence identity to the amino acid sequence as set forth in SEQ ID NO: 42.

In some embodiments, the second antigen-binding portion is a CD3 antigen-binding fragment in an Fab form comprising two chains, which may be referred to herein as an Fab heavy chain and an Fab light chain. The Fab heavy chain comprises a heavy chain variable region VH and a heavy chain constant region CH1. The Fab light chain comprises a light chain variable region VL and a light chain constant region CL. In some embodiments, the CH1 and CL in the second antigen-binding portion may be CH1 or CL derived from a constant region of IgG, IgM, or IgA (e.g., human IgG or human IgM or human IgA). In some embodiments, the CH1 and CL in the second antigen-binding portion may be derived from IgG1, for example, the CH1 or CL constant regions of human IgG1.

In some embodiments, the bispecific antibody of the present invention includes an Fc fragment. In some embodiments, the first antigen-binding portion and/or the second antigen-binding portion is fused to an Fc fragment to form an Fc fusion protein. The Fc fusion protein comprising the first antigen-binding portion and/or the second antigen-binding portion may form a homodimer or a heterodimer. The Fc fragment may be an Fc fragment or a variant thereof derived from a constant region of IgG, IgM or IgA (e.g. human IgG or human IgM or human IgA). In some embodiments, the Fc fragment may be derived from IgG1, for example, the Fc fragment of human IgG1 or a variant thereof. The Fc fragment may be engineered to contain mutations, such as insertions, substitutions, or deletions of one or more amino acids such that steric complementation effects, electrostatic steering, hydrogen bonding, hydrophobic interactions, and the like may be formed between the resulting Fc variants, or such that the resulting variant Fc fragments have altered effector functions relative to the Fc fragment before mutation, such as binding to Fc receptors (e.g., Fcy receptors, such as FcyRIIIA), Clq binding and complement-dependent cytotoxicity (CDC), antibody-dependent cell-mediated cytotoxicity (ADCC), mediation of phagocytosis and the like, or a combination of these effects.

In some embodiments, the interactions between variant Fc fragments contribute to the formation of stable heterodimeric proteins. Mutation engineering techniques for variant Fc fragments have been widely used in the art for the preparation of bispecific antibodies or heterodimeric Fc fusion proteins. Representative examples are the "Knob-into-Hole" format proposed by Cater et al. (Protein Engineering, vol.9, no.7, pp.617-621, 1996); Fc-containing heterodimeric formats formed by technologists at Amgen utilizing Electrostatic Steering (US 20100286374 A1); heterodimeric forms (SEEDbodies) formed by IgG/1gA chain exchange as proposed by Jonathan H. Davis et al. (Protein Engineering, Design & Selection, pp.1-8, 2010); bispecific molecules formed by Genmab's DuoBody (Science, 2007, 317 (5844)) platform technology; heterodimeric protein formats formed by technologists at Xencor via integrating structural calculations and Fc amino acid mutations, and combining different modes of action (mAbs 3: 6, 546-557; November/December 2011); heterodimeric protein formats obtained by the charge network-based Fc modification method of Alphamab Oncology (CN 201110459100.7); and other genetic engineering methods based on Fc amino acid changes or functional modification means, for forming heterodimeric functional proteins. In some embodiments, the variant Fc fragment may have a Knob-Hole structure. In some embodiments, the variant Fc fragment fused to the first antigen-binding portion and/or the second antigen-binding portion can form a Knob-Hole structure to facilitate formation of a heterodimer. The Knob-Hole structure on the variant Fc fragment of the present invention means that the two Fc fragments are mutated respectively, and can be combined in a "Knob-into-hole" format after the mutations. For example, the "knob-into-hole" model of Cater et al. may be used to perform site-directed mutations on the Fc region such that the resulting first variant Fc fragment and the second variant Fc fragment may be combined together in the "knob-into-hole" format to form a heterodimer. The selection of a specific immunoglobulin Fc region from specific immunoglobulin classes and subclasses is within the capability of those skilled in the art. The Fc region of the human antibody IgG1, IgG2, IgG3, IgG4 is preferable, and the Fc region of the human antibody IgG1 is more preferable. One of the first variant Fc fragment or the second variant Fc fragment is randomly selected to undergo the knob mutation, and the other to undergo the hole mutation.

In some embodiments, the first antigen-binding portion is fused to a first Fc fragment, and the second antigen-binding portion is fused to a second Fc fragment.

The first antigen-binding portion and the first Fc fragment may be linked directly or via a peptide linker. In some embodiments, the first antigen-binding portion is fused to the N-terminus of the first Fc fragment.

The second antigen-binding portion and the second Fc fragment may be linked directly or via a peptide linker. In some embodiments, the second antigen-binding portion is fused to the N-terminus of the second Fc fragment.

In some embodiments, the peptide linker may be a flexible linker, such as a glycine-serine polymer, such as (GGGGS)n(SEQ ID NO: 50), wherein n is an integer not less than 1. In some embodiments the peptide linker may be (GGGGS)₃(SEQ ID NO: 49).

In some embodiments, the first Fc fragment and/or the second Fc fragment is an Fc fragment of IgG1 or a variant thereof.

In some embodiments, the first Fc fragment and the second Fc fragment form a Knob-Hole structure, wherein one of the first Fc fragment and the second Fc fragment may form a Knob structure and the other may form a Hole structure, and the first Fc fragment and the second Fc fragment may be combined in a "Knob-into-Hole" format.

In some embodiments, the Fc fragment, such as the first Fc fragment and/or the second Fc fragment, may have mutations that alter the Fc effector functions, for example mutations that reduce or eliminate the binding of the antibody to an Fcy receptor (e.g., FcyRIIIA), decrease CDC and/or decrease ADCC. Such mutations are known to those skilled in the art and include, but are not limited to, LALA double mutation (L234A/L235A) or LALAPG triple mutation (L234A/L235A/P329G) in IgG1, FES triple mutation (L234F/L235E/P331S), FQQ triple mutation (L234F/L235Q/K322Q) and the like, which can significantly reduce the affinity of the antibody to the Fcy receptor (FcyR), thereby decreasing the ADCC effect. Such mutations further include the P329G mutation, which is capable of significantly reducing the binding of the antibody to various Fcy receptors (see, Schlothauer T, Herter S, Koller CE et al., Protein Eng Des Sel. 2016 Oct; 29(10):457-466). Wilkinson I et al. (Fc-engineered antibodies with immune effector functions completely abolished. PLoS ONE, 2021, 16(12): e0260954.https://doi.org/10.1371/journal.pone.0260954) described various combinations of mutations that may reduce or eliminate the antibody's immune effector functions, such as reducing or eliminating binding of the Fc fragment to an Fcy receptor (FcγR). These mutation combinations can avoid FcγR-mediated systemic T-cell activation, thereby allowing immune effector cells to be activated restrictively only within target tissues. This reference is incorporated herein in its entirety by reference. The bispecific antibodies of the present invention may comprise the mutations described in the reference that reduce or eliminate the immune effector functions of antibodies in the Fc fragment (including the first Fc fragment and/or the second Fc fragment). In some embodiments, the Fc fragment, such as the first Fc fragment and/or the second Fc fragment, may include L234S/L235T/G236R triple mutation.

In some embodiments, the first Fc fragment and the second Fc fragment form a Knob-Hole structure and both have a L234S/L235T/G236R triple mutation. In some embodiments, the first Fc fragment and the second Fc fragment comprise a mutation consisting of a mutation that can form a Knob-Hole structure and a L234S/L235T/G236R triple mutation.

In some embodiments, the second antigen-binding fragment is a CD3 antigen-binding fragment in an Fab form with an Fab heavy chain fused to the second Fc fragment. In some embodiments, the Fab heavy chain of the second antigen-binding portion is fused to the N-terminus of the second Fc fragment.

In some embodiments, the bispecific antibody targeting ROR1 and CD3 of the present invention further includes a third antigen-binding portion. The third antigen-binding portion may be an antibody or an antigen-binding fragment thereof that specifically binds to ROR1 or an antibody or an antigen-binding fragment thereof that specifically binds to CD3. The third antigen-binding portion may be the same as or different from or partially the same as the first antigen-binding portion or the second antigen-binding portion (e.g., the CDR regions are identical).

In some embodiments, the third antigen-binding portion is an antibody or an antigen-binding fragment thereof that specifically binds to the ROR1 protein (or the Frizzled domain or Kringle domain thereof), and may be, for example, an Fab, an Fv, a single-chain antibody (scFv) or a single domain antibody (sdAb) and the like. In some embodiments, the third antigen-binding portion is a single domain antibody.

In some embodiments, the third antigen-binding portion includes a heavy chain variable region VH. The VH comprises HCDR1, HCDR2 and HCDR3. The HCDR1, HCDR2 and HCDR3 may be selected from the following groups:
(1) HCDR1 as set forth in SEQ ID NO: 19, HCDR2 as set forth in SEQ ID NO: 20, and HCDR3 as set forth in SEQ ID NO: 21;
(2) HCDR1 as set forth in SEQ ID NO: 23, HCDR2 as set forth in SEQ ID NO: 24, and HCDR3 as set forth in SEQ ID NO: 25;
(3) HCDR1 as set forth in SEQ ID NO: 27, HCDR2 as set forth in SEQ ID NO: 28, and HCDR3 as set forth in SEQ ID NO: 29;
(4) HCDR1 as set forth in SEQ ID NO: 31, HCDR2 as set forth in SEQ ID NO: 32, and HCDR3 as set forth in SEQ ID NO: 33; and
(5) HCDR1 as set forth in SEQ ID NO: 35, HCDR2 as set forth in SEQ ID NO: 36 and HCDR3 as set forth in SEQ ID NO: 37.

In some embodiments, the VH of the third antigen-binding portion comprises an amino acid sequence as set forth in SEQ ID NO: 18, 22, 26, 30 or 34, or a sequence having at least 80%, at least 85%, at least 90%, at least 95%, at least 96%, at least 97%, at least 98% or at least 99% sequence identity to the amino acid sequence as set forth in SEQ ID NO: 18, 22, 26, 30 or 34.

In some embodiments, the third antigen-binding portion may have CDR regions that are the same as, different from, or partially the same as those of the first antigen-binding portion, for example, having the same or different, or partially same HCDR1, HCDR2 and HCDR3. In some embodiments, the third antigen-binding portion may have variable regions that are the same as, different from, or partially the same as those of the first antigen-binding portion, for example, having the same or different, or partially the same VH.

In some embodiments, the third antigen-binding portion may be fused to the first antigen-binding portion, for example, may be linked directly or via a peptide linker to the first antigen-binding portion. In some embodiments, the third antigen-binding portion may be fused to the N-terminus of the first antigen-binding portion.

In some embodiments, the third antigen-binding portion may be fused to the second antigen-binding portion, for example, may be linked directly or via a peptide linker to the second antigen-binding portion. In cases where the second antigen-binding portion comprises two chains, the third antigen-binding portion may be fused to either chain of the second antigen-binding portion, for example, may be fused to the Fab heavy chain or the Fab light chain of the second antigen-binding portion, i.e., linked directly or via a peptide linker to the Fab heavy chain or the Fab light chain of the second antigen-binding portion. In some embodiments, the third antigen-binding portion may be fused to the N-terminus of either chain of the second antigen-binding portion, such as the N-terminus of the Fab heavy chain or the Fab light chain.

In some embodiments, the peptide linker may be (GGGGS)n (SEQ ID NO: 50), wherein n is an integer not less than 1. In some embodiments, the peptide linker is (GGGGS)₃ (SEQ ID NO: 49).

In some embodiments, the bispecific antibody targeting ROR1 and CD3 comprises a first antigen-binding portion and a second antigen-binding portion, wherein the first antigen-binding portion is a single domain antibody that specifically binds to ROR1, the second antigen-binding portion is an Fab fragment that specifically binds to CD3. The Fab fragment comprises an Fab heavy chain and an Fab light chain. The first antigen-binding portion is fused to the N-terminus of the first Fc fragment, and the Fab heavy chain of the second antigen-binding portion is fused to the N-terminus of the second Fc fragment.

In some embodiments, the bispecific antibody targeting ROR1 and CD3 comprises a first antigen-binding portion, a second antigen-binding portion and a third antigen-binding portion, wherein the first antigen-binding portion and the third antigen-binding portion are single domain antibodies that specifically bind to ROR1, the second antigen-binding portion is an Fab fragment that specifically binds to CD3. The Fab fragment comprises an Fab heavy chain and an Fab light chain. The first antigen-binding portion is fused to the N-terminus of the first Fc fragment. The Fab heavy chain of the second antigen-binding portion is fused to the N-terminus of the second Fc fragment. The third antigen-binding portion is fused to the N-terminus of the first antigen-binding portion or to the N-terminus of the Fab heavy chain of the second antigen-binding portion.

In some embodiments, the bispecific antibody targeting ROR1 and CD3 consists of a first chain, a second chain and a third chain. The first chain comprises a fusion protein of the first antigen-binding portion and the first Fc fragment, the second chain comprises a fusion protein of the Fab heavy chain of the second antigen-binding portion and the second Fc fragment, the third chain comprises an Fab light chain of the second antigen-binding portion. In some embodiments, the first chain comprises an amino acid sequence as set forth in SEQ ID NO: 1, 4, 5, 6 or 7, or a sequence having at least 80%, at least 85%, at least 90%, at least 95%, at least 96%, at least 97%, at least 98% or at least 99% sequence identity to the amino acid sequence as set forth in SEQ ID NO: 1, 4, 5, 6 or 7; the second chain comprises an amino acid sequence as set forth in SEQ ID NO: 2, or a sequence having at least 80%, at least 85%, at least 90%, at least 95%, at least 96%, at least 97%, at least 98% or at least 99% sequence identity to the amino acid sequence as set forth in SEQ ID NO: 2; the third chain comprises an amino acid sequence as set forth in SEQ ID NO: 3, or a sequence having at least 80%, at least 85%, at least 90%, at least 95%, at least 96%, at least 97%, at least 98% or at least 99% sequence identity to the amino acid sequence as set forth in SEQ ID NO: 3.

In some embodiments, the bispecific antibody targeting ROR1 and CD3 consists of a first chain, a second chain and a third chain. The first chain comprises a fusion protein of the third antigen-binding portion, the first antigen-binding portion and the first Fc fragment, the second chain comprises a fusion protein of the Fab heavy chain of the second antigen-binding portion and the second Fc fragment, the third chain comprises an Fab light chain of the second antigen-binding portion. In some embodiments, the first chain comprises an amino acid sequence as set forth in SEQ ID NO: 8, 9, 10, 11 or 12, or a sequence having at least 80%, at least 85%, at least 90%, at least 95%, at least 96%, at least 97%, at least 98% or at least 99% sequence identity to the amino acid sequence as set forth in SEQ ID NO: 8, 9, 10, 11 or 12; the second chain comprises an amino acid sequence as set forth in SEQ ID NO: 2, or a sequence having at least 80%, at least 85%, at least 90%, at least 95%, at least 96%, at least 97%, at least 98% or at least 99% sequence identity to the amino acid sequence as set forth in SEQ ID NO: 2; the third chain comprises an amino acid sequence as set forth in SEQ ID NO: 3, or a sequence having at least 80%, at least 85%, at least 90%, at least 95%, at least 96%, at least 97%, at least 98% or at least 99% sequence identity to the amino acid sequence as set forth in SEQ ID NO: 3.

In some embodiments, the bispecific antibody targeting ROR1 and CD3 consists of a first chain, a second chain and a third chain. The first chain comprises a fusion protein of the first antigen-binding portion and the first Fc fragment, the second chain comprises a fusion protein of the third antigen-binding portion, the Fab heavy chain of the second antigen-binding portion and the second Fc fragment, the third chain comprises an Fab light chain of the second antigen-binding portion. In some embodiments, the first chain comprises an amino acid sequence as set forth in SEQ ID NO: 1, 4, 5, 6 or 7, or a sequence having at least 80%, at least 85%, at least 90%, at least 95%, at least 96%, at least 97%, at least 98% or at least 99% sequence identity to the amino acid sequence as set forth in SEQ ID NO: 1, 4, 5, 6 or 7; the second chain comprises an amino acid sequence as set forth in SEQ ID NO: 13, 14, 15, 16 or 17, or a sequence having at least 80%, at least 85%, at least 90%, at least 95%, at least 96%, at least 97%, at least 98% or at least 99% sequence identity to the amino acid sequence as set forth in SEQ ID NO: 13, 14, 15, 16 or 17; the third chain comprises an amino acid sequence as set forth in SEQ ID NO: 3, or a sequence having at least 80%, at least 85%, at least 90%, at least 95%, at least 96%, at least 97%, at least 98% or at least 99% sequence identity to the amino acid sequence as set forth in SEQ ID NO: 3.

In some embodiments, the first chain, the second chain and the third chain are selected from the following groups:
(1) the first chain comprising an amino acid sequence as set forth in SEQ ID NO: 1, or a sequence having at least 80%, at least 85%, at least 90%, at least 95%, at least 96%, at least 97%, at least 98% or at least 99% sequence identity to the amino acid sequence as set forth in SEQ ID NO: 1, the second chain comprising the amino acid sequence as set forth in SEQ ID NO: 13, or a sequence having at least 80%, at least 85%, at least 90%, at least 95%, at least 96%, at least 97%, at least 98% or at least 99% sequence identity to the amino acid sequence as set forth in SEQ ID NO: 13, and the third chain comprising the amino acid sequence as set forth in SEQ ID NO: 3, or a sequence having at least 80%, at least 85%, at least 90%, at least 95%, at least 96%, at least 97%, at least 98% or at least 99% sequence identity to the amino acid sequence as set forth in SEQ ID NO: 3;
(2) the first chain comprising an amino acid sequence as set forth in SEQ ID NO: 4, or a sequence having at least 80%, at least 85%, at least 90%, at least 95%, at least 96%, at least 97%, at least 98% or at least 99% sequence identity to the amino acid sequence as set forth in SEQ ID NO: 4, the second chain comprising the amino acid sequence as set forth in SEQ ID NO: 14, or a sequence having at least 80%, at least 85%, at least 90%, at least 95%, at least 96%, at least 97%, at least 98% or at least 99% sequence identity to the amino acid sequence as set forth in SEQ ID NO: 14, and the third chain comprising the amino acid sequence as set forth in SEQ ID NO: 3, or a sequence having at least 80%, at least 85%, at least 90%, at least 95%, at least 96%, at least 97%, at least 98% or at least 99% sequence identity to the amino acid sequence as set forth in SEQ ID NO: 3;
(3) the first chain comprising an amino acid sequence as set forth in SEQ ID NO: 5, or a sequence having at least 80%, at least 85%, at least 90%, at least 95%, at least 96%, at least 97%, at least 98% or at least 99% sequence identity to the amino acid sequence as set forth in SEQ ID NO: 5, the second chain comprising the amino acid sequence as set forth in SEQ ID NO: 15, or a sequence having at least 80%, at least 85%, at least 90%, at least 95%, at least 96%, at least 97%, at least 98% or at least 99% sequence identity to the amino acid sequence as set forth in SEQ ID NO: 15, and the third chain comprising the amino acid sequence as set forth in SEQ ID NO: 3, or a sequence having at least 80%, at least 85%, at least 90%, at least 95%, at least 96%, at least 97%, at least 98% or at least 99% sequence identity to the amino acid sequence as set forth in SEQ ID NO: 3;
(4) the first chain comprising an amino acid sequence as set forth in SEQ ID NO: 6, or a sequence having at least 80%, at least 85%, at least 90%, at least 95%, at least 96%, at least 97%, at least 98% or at least 99% sequence identity to the amino acid sequence as set forth in SEQ ID NO: 6, the second chain comprising the amino acid sequence as set forth in SEQ ID NO: 16, or a sequence having at least 80%, at least 85%, at least 90%, at least 95%, at least 96%, at least 97%, at least 98% or at least 99% sequence identity to the amino acid sequence as set forth in SEQ ID NO: 16, and the third chain comprising the amino acid sequence as set forth in SEQ ID NO: 3, or a sequence having at least 80%, at least 85%, at least 90%, at least 95%, at least 96%, at least 97%, at least 98% or at least 99% sequence identity to the amino acid sequence as set forth in SEQ ID NO: 3; and
(5) the first chain comprising an amino acid sequence as set forth in SEQ ID NO: 7, or a sequence having at least 80%, at least 85%, at least 90%, at least 95%, at least 96%, at least 97%, at least 98% or at least 99% sequence identity to the amino acid sequence as set forth in SEQ ID NO: 7, the second chain comprising the amino acid sequence as set forth in SEQ ID NO: 17, or a sequence having at least 80%, at least 85%, at least 90%, at least 95%, at least 96%, at least 97%, at least 98% or at least 99% sequence identity to the amino acid sequence as set forth in SEQ ID NO: 17, and the third chain comprising the amino acid sequence as set forth in SEQ ID NO: 3, or a sequence having at least 80%, at least 85%, at least 90%, at least 95%, at least 96%, at least 97%, at least 98% or at least 99% sequence identity to the amino acid sequence as set forth in SEQ ID NO: 3.

The bispecific antibody of the present invention has enhanced binding affinity for the ROR1 protein (e.g., human ROR1 protein). In some embodiments, the binding affinity (KD) of the bispecific antibody of the present invention for the ROR1 protein (e.g., the human ROR1 protein) increases with the increase in the antibody conjugation concentration, for example, may be determined by the surface plasmon resonance (SPR). The bispecific antibody of the present invention has increased binding affinity for cell lines expressing human ROR1 (e.g., CHO-K1 cell lines expressing human ROR1 or tumour cell lines expressing human ROR1), and/or cell lines expressing human CD3 (e.g., Jurkat cells expressing human CD3).

In some embodiments, the bispecific antibody of the present invention has an increased ability to activate T cells, for example, as assayed by EC50 of T cell activation mediated by the antibody based on reporter gene.

In some embodiments, the bispecific antibody of the present invention has an increased ability to mediate the killing of target cells by PBMCs.

In some embodiments, the bispecific antibody of the present invention reduces cytokine secretion of PBMCs, such as the reduced secretion of the cytokines IFN-γ, TNF-α, IL-2 and/or IL-6.

The present invention also provides polynucleotides encoding the bispecific antibody of the present invention targeting ROR1 and CD3. The polynucleotide may be DNA or RNA (e.g., mRNA).

In some embodiments, the polynucleotides encode a first chain, a second chain and a third chain of the bispecific antibody, respectively.

The present invention further provides a vector comprising the polynucleotides described above, which can be used to express the bispecific antibodies of the present invention in a host cell. The vector may be an expression vector, such as a plasmid vector or a viral vector. The viral vector may be a retroviral vector, a lentiviral vector, an adenoviral vector, or an adeno-associated viral vector.

The present invention also provides a host cell comprising the polynucleotide described above or the vector described above. The host cell can be used to express the bispecific antibody of the present invention. The host cell may be a mammalian cell, such as a mouse cell or a human cell. For example, the host cell may be a CHO cell, a HEK-293 cell, a BHK cell or a PER-C6 cell, as well as derivative cells thereof, such as 293-6E, CHO-DG44, CHO-K1, CHO-S and CHO-DS cells.

### Pharmaceutical Compositions and Treatments

The present invention further provides a pharmaceutical composition comprising the bispecific antibody described above, the polynucleotide described above, the vector described above or the host cell described above, and optionally a pharmaceutically acceptable carrier.

The bispecific antibody, the polynucleotide, the vector, the host cell and the pharmaceutical composition of the present invention may be used in the treatment of a disease associated with ROR1. The bispecific antibody, the polynucleotide, the vector, the host cell and the pharmaceutical composition of the present invention may be used in the preparation of a drug for the treatment of a disease associated with ROR1. The present invention further provides a method for treating a disease associated with ROR1, including administering to a subject in need thereof the bispecific antibody, the polynucleotide, the vector, the host cell or the pharmaceutical composition of the present invention.

The disease associated with ROR1 may be, for example, cancers or tumours associated with ROR1. The disease associated with ROR1 are characterized by the expression of ROR1, such as the expression of ROR1 on the surface of cancer or tumour cells. In some embodiments, the cancer or tumour may be a haematological malignancy or a solid tumour, such as acute myeloid leukaemia (AML), myelodysplastic syndrome (MDS), acute lymphoblastic leukaemia (ALL), diffuse large B-cell lymphoma (DLBCL), chronic myeloid leukaemia (CML), chronic lymphocytic leukaemia (CLL), mantle cell lymphoma (MCL), lung cancer (e.g., non-small cell lung cancer NSCLC or small cell lung cancer SCLC), breast cancer, prostate cancer, pancreatic cancer, colon cancer, ovarian cancer, kidney cancer, uterine cancer, melanoma and the like.

The terms "subject" and "patient" are used interchangeably herein. The term "subject" as used herein refers to any organism to which the antibody or the antigen-binding fragment thereof of the present invention can be administered, for example, for experimental, diagnostic, prophylactic and/or therapeutic purposes. Typical subjects include animals (e.g., mammals such as mice, rats, rabbits, non-human primates such as chimpanzees and other simians, and humans). The subject may be a mammal, particularly a human, including females (women) or males (men), and includes neonates, infants, juveniles, adolescents, adults, or the elderly, and further includes various races and ethnic groups. In some examples, a subject refers to an individual in need of diagnosis, treatment, or prevention of a disease or disorder. The subject may have, or be at risk of developing the disease or disorder.

The term "treatment" as used herein refers to providing a beneficial or desired clinical outcome for a disease, such as eliminating the disease, alleviating symptoms, reducing the extent of the disease, stabilizing, ameliorating, or relieving the state of the disease, or slowing the progression of the disease. The measurement of treatment outcome may be based, for example, on results of physical examinations, pathological tests and/or diagnostic tests known in the art. Treatment may also refer to an extension of survival as compared to the expected survival when the subject dose not receive the treatment. Treatment may also refer to a reduction in the prevalence or incidence of a disease, or recurrence thereof, compared to the disease that would occur without the treatment. Clinically, such a treatment can also be referred to as prophylaxis.

The terms "pharmaceutical composition" and "pharmaceutical formulation" of the present invention are used interchangeably. The pharmaceutical composition may comprise a pharmaceutically acceptable carrier. The term "pharmaceutically acceptable carrier" as used herein refers to any carrier contained in a pharmaceutical composition as an inactive ingredient that imparts suitable appearance and properties to the pharmaceutical composition for administration. The pharmaceutically acceptable carrier is substantially free of long-term or permanent adverse effects when administered to a subject, and is such as a stabilizer, a diluent, an additive, an adjuvant, an excipient and the like. A "pharmaceutically acceptable carrier" should be a pharmaceutically inert material that has substantially no biological activity and constitutes a major part of the formulation.

The pharmaceutical compositions of the present invention can be formulated for various routes of administration using known techniques. For example, see Remington, The Science and Practice of Pharmacy (9th Ed.1995). In the manufacture of pharmaceutical compositions, an active agent is typically mixed with a pharmaceutically acceptable carrier and the like. Of course, the pharmaceutically acceptable carrier must be acceptable, i.e., compatible with any other ingredients in the formulation, and must be non-harmful to the subject. The pharmaceutically acceptable carrier may include, but are not limited to, a buffer, an excipient, a stabilizer, a preservative, a wetting agent, a surfactant, an emulsifier or combinations thereof. Examples of the buffer includes, but are not limited to, acetic acid, citric acid, histidine, boric acid, formic acid, succinic acid, phosphoric acid, carbonic acid, malic acid, aspartic acid, Tris buffer, HEPPSO, HEPES, neutral buffered saline, phosphate-buffered saline and the like.

The bispecific antibody, the polynucleotide, the vector, the host cell and the pharmaceutical composition of the present invention may be administered in any route suitable for the disease and subject to be treated (or prevented). In certain embodiments, the routes of administration may include, but are not limited to, parenteral or non-parenteral routes, for example oral, sublingual, buccal, percutaneous, rectal, vaginal, intradermal, intranasal routes; or parenteral routes such as intravenous (i.v.), intraperitoneal, intradermal, subcutaneous, intramuscular, intracranial, intrathecal, intratumoral, percutaneous, intramucosal, intraarticular, intrathecal, intrathecal, intrahepatic, intraneural, or intracranial injection or infusion. The pharmaceutical composition can be injected directly into a tumour, lymph node, tissue, organ or site of infection.

Dosage forms suitable for oral administration include, but are not limited to, tablets, capsules, powders, pills, granules, suspensions, solutions or pre-concentrates of solutions, emulsions or pre-concentrates of emulsions. Pharmaceutically acceptable carriers suitable for oral dosage forms include water, glycols, oils, alcohols, flavouring agents, preservatives, colorants, and the like. Carriers such as starches, sugars, microcrystalline cellulose, diluents, fillers, glidant, granulating agents, lubricants, binders, stabilizers, disintegrants and the like can be used to prepare oral solid dosage forms such as powders, capsules or tablets.

Dosage forms suitable for parenteral administration include, but are not limited to, sterile liquid formulations, such as isotonic aqueous solutions, emulsions, suspensions, dispersions, or viscous compositions, which can be buffered to a desired pH. The parenteral dosage forms may be either ready-to-use or dry products ready to be dissolved or suspended in a pharmaceutically acceptable carrier. The parenteral dosage forms can be sterile formulations or can be sterilized prior to administration to a subject. Pharmaceutically acceptable carriers suitable for providing parenteral dosage forms include, but are not limited to, water for injection; aqueous carriers such as, but not limited to, sodium chloride injection, Ringer's injection, glucose injection. water-soluble carriers such as, but not limited to, ethanol, polyethylene glycol, and polypropylene glycol; nonaqueous carriers such as, but not limited to, corn oil, cottonseed oil, peanut oil, sesame oil, ethyl oleate, isopropyl myristate, and benzyl benzoate; and solubilizers such as cyclodextrins.

The bispecific antibody, the polynucleotide, the vector, the host cell and the pharmaceutical composition of the present invention are administered to a subject in therapeutically effective amounts. The terms "therapeutically effective amount" and "effective amount" as used herein, are used interchangeably and refer to an amount that is effective in dosages and periods of time necessary to achieve the desired therapeutic effect. A therapeutically effective amount is generally expressed in terms of the amount of the active ingredient, for example, in terms of the amount of the bispecific antibody. The therapeutically effective amount may vary depending on different factors, such as disease state, age, sex, and individual body weight, and the ability of a treatment method or combination of treatment methods to elicit the desired response in an individual. An effective amount may refer to an amount that elicits a detectable change in biological or chemical activity. The detectable change may be detected and/or further quantified by a person in the relevant art. Furthermore, an "effective amount" may refer to an amount that maintains a desired physiological state, i.e. reduces or prevents a significant decline and/or promotes improvement of a disorder. The amount and frequency of administration will be determined by factors such as the condition of the subject (e.g., age, body weight, sex, and the subject's response to the drug) and the type and severity of the subject's disease, although the appropriate dosage may be determined by clinical trials.

The bispecific antibody, the polynucleotide, the vector, the host cell and the pharmaceutical composition of the present invention can be administered to a subject in a therapeutically effective amount of about 0.5 to about 250 mg/kg, for example, about 1 to about 250 mg/kg, about 2 to about 200 mg/kg, about 3 to about 120 mg/kg, about 5 to about 250 mg/kg, about 10 to about 200 mg/kg, or about 20 to about 120 mg/kg.

The bispecific antibody, the polynucleotide, the vector, the host cell, and the pharmaceutical composition of the present invention may be administered once or twice daily; or once every 2, 3, 4, 5, 6, 7, 8, 9 or 10 days, once every 1, 2, 3, 4, 5 or 6 weeks, or once every 1, 2, 3, 4, 5 or 6 months or at longer intervals. The pharmaceutical composition may also be administered in accordance with a regimen of several times per week (e.g., 1, 2, 3, 4 or 5 times) or several times per month (e.g., 1, 2, 3, 4, 5, 6, 7, 8, 9 or 10 times).

The bispecific antibody, the polynucleotide, the vector, the host cell, and the pharmaceutical composition of the present invention may be used in combination with other drugs and treatment methods, such as other antitumour drugs, chemotherapy or radiotherapy. As used herein, "used in combination" refers to the administration of two (or more) different drugs and/or therapies to a subject during the course of treatment thereof. Two or more drugs and/or therapies in the combination may be administered by different routes and regimens. Two or more drugs and/or treatment methods may be administered to a subject simultaneously or sequentially. In some embodiments, administration of one drug or therapy is still ongoing when administration of the second drug or therapy begins, resulting an overlap in administration. Such a regimen may be referred to herein as "simultaneous". When administered simultaneously, two or more drugs and/or therapies may be formulated together into a single dosage form or separately into two or more distinct dosage forms.

The embodiments of the present invention will be described in detail below with reference to examples. Unless otherwise stated, the methods and materials of the examples described below are conventional products that are commercially available or may be prepared by known methods. The experimental methods for which specific conditions are not specified in the following examples are generally based on conventional conditions as described by Michael R. Green and Joseph Sambrook, Molecule Cloning: A Laboratory Manual, 4th Edition, 2012, or as recommended by the manufacturer. Those skilled in the art to which the present invention pertains will understand that the methods and materials described below are merely exemplary and should not be regarded as limiting the scope of the present invention.

In the following examples, the CDRs of the heavy chain variable regions and light chain variable regions of antibodies are defined in accordance with the Kabat numbering scheme.

### Example 1: Design of bispecific antibody targeting ROR1 and CD3

Bispecific antibody configuration design: Based on the binding valence and position for targeting ROR1 in the double antibody, three double antibody configurations targeting ROR1 and CD3 were designed for subsequent expression and activity verification. In order to reduce the generation of homodimers of the bispecific antibody molecule, different amino acid mutations were introduced respectively into the constant regions of the two chains to form a knob-into-hole structure (hereinafter, the peptide chain with the Hole mutation is abbreviated as the hole chain, and the peptide chain with the knob mutation is abbreviated as the knob chain). At the same time, in order to eliminate the effector function of the Fc region, L234S/L235T/G236R triple mutations were introduced into the constant region of the heavy chain. The specific designs of these three configurations and their corresponding names are shown in Table 1 below.

**Table 1 Configuration of bispecific antibody targeting ROR1 and CD3**

| | Bispecific antibody configuration | Configuration diagram | Configuration design |
|---|---|---|---|
| 1 | 1+1 bispecific antibody | FIG. 1 | IgG-like symmetric bispecific antibody, the antigen-binding portions targeting ROR1 and CD3 are monovalent and located in the two binding arms of the antibody, respectively. |
| 2 | 2+1 bispecific antibody | FIG. 2 | IgG-like asymmetric bispecific antibody; the binding portion targeting ROR1, in a tandem bivalent form, is conjugated at the N-terminus of the hole chain; the antigen-binding portion targeting CD3, in a monovalent form, is conjugated to the N-terminus of the knob chain. |
| 3 | 1+1+1 bispecific antibody | FIG. 3 | IgG-like asymmetric bispecific antibody; the binding portion targeting ROR1, in a monovalent form, is conjugated to the N-terminus of the hole chain and the N-terminus of the knob chain, respectively; the antigen-binding portion targeting CD3, in a monovalent Fab form, is conjugated between the N-terminal ROR1 antigen-binding portion of the knob chain and the Fc region. |

There were 5 antigen-binding units targeting ROR1, all from the discovery process of human single domain antibodies targeting ROR1, namely AHP15662-H4-M21721, AHP15662-H4-MV2, AHP15580, AHP15485-H4-MV2, AHP15485-H4-MV2 and AHP15485-H4-M22013. The antigen-binding protein targeting CD3 was selected from humanized antibody AED-2018. The Fc region of human IgG1 and the light chain constant region of human IgG were used, wherein HuIgG1Fc (L234S/L235T/G236R hole) and HuIgG1Fc (L234S/L235T/G236R knob) represent the human IgG1 Fc fragments engineered with either the hole or knob mutations, respectively, to form a knob-into-hole structure and with additional triple mutations L234S/L235T/G236R for silencing Fc effector functions, respectively. HuIgGICH(L234S/L235T/G236R knob) comprises the heavy chain constant region CH1 of human IgG1 and HuIgG1Fc(L234S/L235T/G236R knob). The composition and sequence identifier number of each peptide chain unit of the bispecific antibody targeting ROR1 and CD3 are shown in Table 2 below.

**Table 2 Composition and sequence identifier number of each peptide chain unit of the bispecific antibody**

| Bispecific antibody configuration | Antibody number | Peptide chain unit of the bispecific antibody and composition thereof | Sequence identifier number |
|---|---|---|---|
| 1+1 bispecific antibody | BsAb-1 | AHP15662-H4-M21721-HuIgG1Fc(L234S/L235T/G236R hole) | SEQ ID NO: 1 |
| | | AED-2018-VH-HuIgG1CH(L234S/L235T/G236R knob) | SEQ ID NO: 2 |
| | | AED-2018-VL-HuIgGCL | SEQ ID NO: 3 |
| | BsAb-2 | AHP15662-H4-MV2-HuIgG1Fc(L234S/L235T/G236R hole) | SEQ ID NO: 4 |
| | | AED-2018-VH-HuIgG1CH(L234S/L235T/G236R knob) | SEQ ID NO: 2 |
| | | AED-2018-VL-HuIgGCL | SEQ ID NO: 3 |
| | BsAb-3 | AHP15580-HuIgG1Fc(L234S/L235T/G236R hole) | SEQ ID NO: 5 |
| | | AED-2018-VH-HuIgGICH(L234S/L235T/G236R knob) | SEQ ID NO: 2 |
| | | AED-2018-VL-HuIgGCL | SEQ ID NO: 3 |
| | BsAb-4 | AHP15485-H4-MV2-HuIgG1Fc(L234S/L235T/G236R hole) | SEQ ID NO: 6 |
| | | AED-2018-VH-HuIgG1CH(L234S/L235T/G236R knob) | SEQ ID NO: 2 |
| | | AED-2018-VL-HuIgGCL | SEQ ID NO: 3 |
| | BsAb-15 | AHP15485-H4-M22013-HuIgG1Fc(L234S/L235T/G236R hole) | SEQ ID NO: 7 |
| | | AED-2018-VH-HuIgG1CH(L234S/L235T/G236R knob) | SEQ ID NO: 2 |
| | | AED-2018-VL-HuIgGCL | SEQ ID NO: 3 |
| 2+1 bispecific antibody | BsAb-5 | AHP15662-H4-M21721-(G4S)3-AHP15662-H4-M21721-HuIgG1Fc(L234S/L235T/G236R hole) | SEQ ID NO: 8 |
| | | AED-2018-VH-HuIgG1CH(L234S/L235T/G236R knob) | SEQ ID NO: 2 |
| | | AED-2018-VL-HuIgGCL | SEQ ID NO: 3 |
| | BsAb-6 | AHP 1 5662-H4-MV2-(G4S)3-AHP 1 5662-H4-MV2-HuIgG1Fc(L234S/L235T/G236R hole) | SEQ ID NO: 9 |
| | | AED-2018-VH-HuIgG1CH(L234S/L235T/G236R knob) | SEQ ID NO: 2 |
| | | AED-2018-VL-HuIgGCL | SEQ ID NO: 3 |
| | BsAb-7 | AHP15580-(G4S)3-AHP 15580-HuIgG1Fc(L234S/L235T/G236R hole) | SEQ ID NO: 10 |
| | | AED-2018-VH-HuIgG1CH(L234S/L235T/G236R knob) | SEQ ID NO: 2 |
| | | AED-2018-VL-HuIgGCL | SEQ ID NO: 3 |
| | BsAb-8 | AHP 1 5485-H4-MV2-(G4S)3-AHP 15485-H4-MV2-HuIgG1Fc(L234S/L235T/G236R hole) | SEQ ID NO: 11 |
| | | AED-2018-VH-HuIgG1CH(L234S/L23 5T/G236R knob) | SEQ ID NO: 2 |
| | | AED-2018-VL-HuIgGCL | SEQ ID NO: 3 |
| | BsAb-16 | AHP15485-H4-M22013-(G4S)3-AHP15485-H4-M22013-HuIgG1Fc(L234S/L235T/G236R hole) | SEQ ID NO: 12 |
| | | AED-2018-VH-HuIgG1CH(L234S/L235T/G236R knob) | SEQ ID NO: 2 |
| | | AED-2018-VL-HuIgGCL | SEQ ID NO: 3 |
| 1+1+1 bispecific antibody | BsAb-9 | AHP15662-H4-M21721-HuIgG1Fc(L234S/L235T/G236R hole) | SEQ ID NO: 1 |
| | | AHP15662-H4-M21721-(G4S)3-AED-2018-VH-HuIgGICH(L234S/L235T/G236R knob) | SEQ ID NO: 13 |
| | | AED-2018-VL-HuIgGCL | SEQ ID NO: 3 |
| | BsAb-10 | AHP15662-H4-MV2-HuIgG1Fc(L234S/L235T/G236R hole) | SEQ ID NO: 4 |
| | | AHP15662-H4-MV2-(G4S)3-AED-2018-VH-HuIgG1CH(L234S/L235T/G236R knob) | SEQ ID NO: 14 |
| | | AED-2018-VL-HuIgGCL | SEQ ID NO: 3 |
| | BsAb-11 | AHP15580-HuIgG1Fc(L234S/L235T/G236R hole) | SEQ ID NO: 5 |
| | | AHP15580-(G4S)3-AED-2018-VH-HuIgG1CH(L234S/L235T/G236R knob) | SEQ ID NO: 15 |
| | | AED-2018-VL-HuIgGCL | SEQ ID NO: 3 |
| | BsAb-12 | AHP15485-H4-MV2-HuIgG1Fc(L234S/L235T/G236R hole) | SEQ ID NO: 6 |
| | | AHP15485-H4-MV2-(G4S)3-AED-2018-VH-HuIgG1CH(L234S/L235T/G236R knob) | SEQ ID NO: 16 |
| | | AED-2018-VL-HuIgGCL | SEQ ID NO: 3 |
| | BsAb-17 | AHP15485-H4-M22013-HuIgG1Fc(L234S/L235T/G236R hole) | SEQ ID NO: 7 |
| | | AHP15485-H4-M22013-(G4S)3-AED-2018-VH-HuIgG1CH(L234S/L235T/G236R knob) | SEQ ID NO: 17 |
| | | AED-2018-VL-HuIgGCL | SEQ ID NO: 3 |

### Example 2. Recombinant production of bispecific antibody

Expression fragments of each peptide chain of the codon-optimized bispecific antibody were synthesized, and then inserted into the pcDNA3.4 expression vector to form expression plasmids. Bispecific molecules binding to ROR1 and CD3 were expressed by transiently transfecting a combination of pCDNA3.4 vectors carrying the bispecific antibody coding genes into ExpiCHO-S cells, that is, for each bispecific antibody, the three corresponding plasmids were mixed at a ratio of 1 : 1 : 1 for transfection, followed by culturing in shake flasks at 37°C for 6 days. The supernatant was then collected for antibody purification. Prior to purification, the tubing and Protein A column were depyrogenated with 0.2 M NaOH. The column was re-equilibrated with a buffer containing 0.05 M Tris and 1.5 M NaCl (pH = 8.0). The harvested cell culture supernatant was then diluted 1 : 1 using 2× the above buffer and sterilized by filtration. The filtered supernatant was incubated with the Protein A column at room temperature for 2 hours. After washing the column with 1× the above buffer, IgG was eluted using sterile 0.1 M sodium citrate (pH 3.5). The eluate was collected and neutralized with one-ninth volume of sterile 1 M Tris-HCl (pH = 9.0). Under sterile conditions, the product was buffer-exchanged into PBS (pH 7.4) to remove any residual elution buffer and concentrate the sample. After concentration, the antibody was quantified by measuring OD280 nm using an extinction coefficient Ec (0.1%) of 1.43. The purified antibody was analysed by SDS-PAGE using a 10% precast gel (GenScript) with the BioRad electrophoresis system. The gel was stained with Estain2.0 (GenScript), and the molecular size and purity were estimated by comparing the stained bands with the Protein Ladder (GenScript). The antibody sample was detected by SEC-HPLC using the Agilent Infinity Lab LC1260 system. Based on with the principle that substances with different molecular weights have different retention times in the chromatographic column (TSkgel), the molecular size was estimated by comparing the elution time of the main peak with that of known molecular weight standards (STD, BIO-RAD), and the purity of samples was obtained from the main peak area percentage. The yield and purity information of bispecific antibody targeting ROR1 and CD3 are shown in Table 3 below. All 15 bispecific antibody molecules were homogeneous and of high purity.

**Table 3 Bispecific antibody yield and purity information**

| Bispecific antibody number | Yield (mg/L) | SDS-PAGE Purify (%) | SEC-HPLC Purify (%) |
|---|---|---|---|
| BsAb-01 | 102 | 97 | 96.52 |
| BsAb-02 | 106.5 | 96 | 94.19 |
| BsAb-03 | 153.5 | 92 | 93.94 |
| BsAb-04 | 99 | 97 | 96.01 |
| BsAb-15 | 76 | 93 | 97.61 |
| BsAb-05 | 87.5 | 99 | 95.4 |
| BsAb-06 | 261 | 99 | 92.46 |
| BsAb-07 | 58.5 | 91 | 96.54 |
| BsAb-08 | 191.5 | 94 | 86.75 |
| BsAb-16 | 69 | 96 | 91.3 |
| BsAb-09 | 244 | 97 | 99.69 |
| BsAb-10 | 56 | 96 | 90.37 |
| BsAb-11 | 180.5 | 90 | 88.18 |
| BsAb-12 | 162.5 | 92 | 86.28 |
| BsAb-17 | 92.5 | 93 | 97.61 |

### Example 3: Detection of binding of bispecific antibody to cell line expressing human ROR1and jurkat cell line expressing human CD3

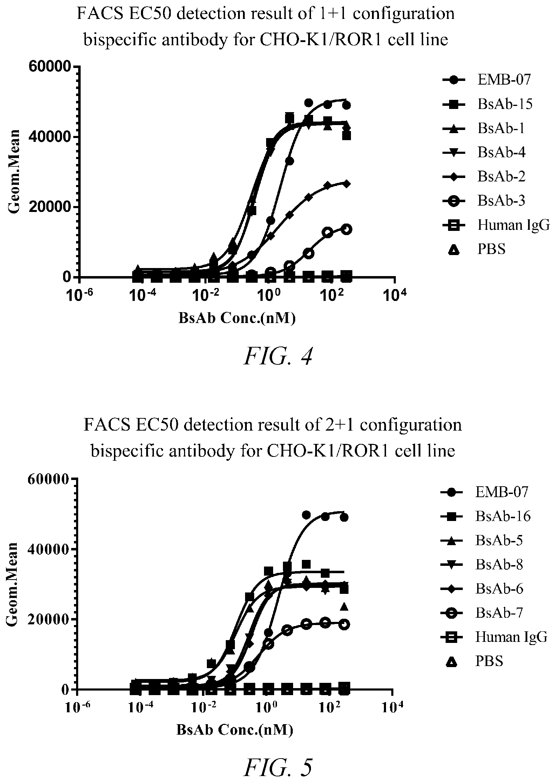

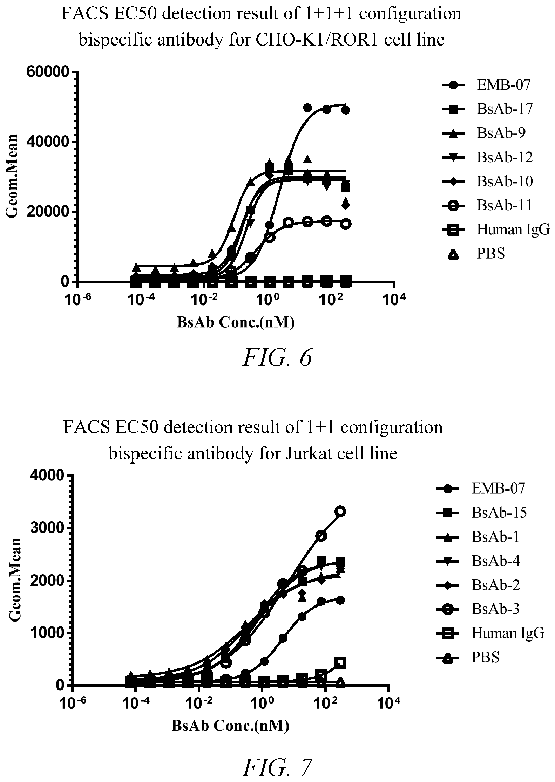

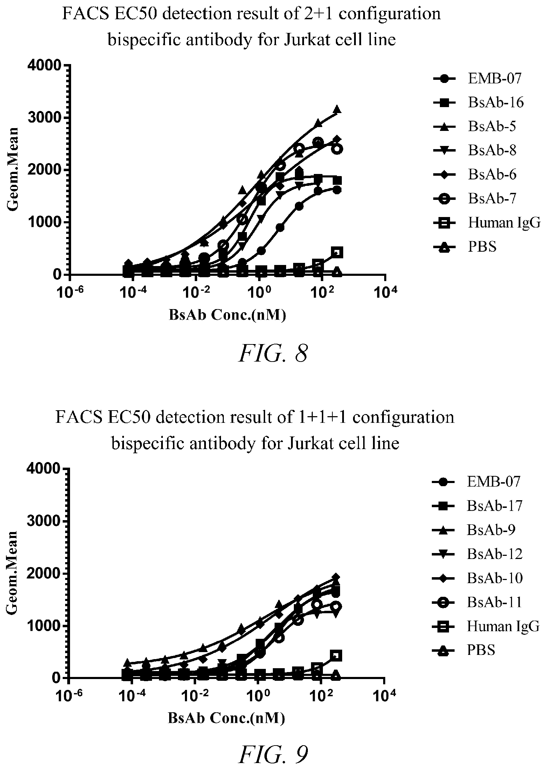

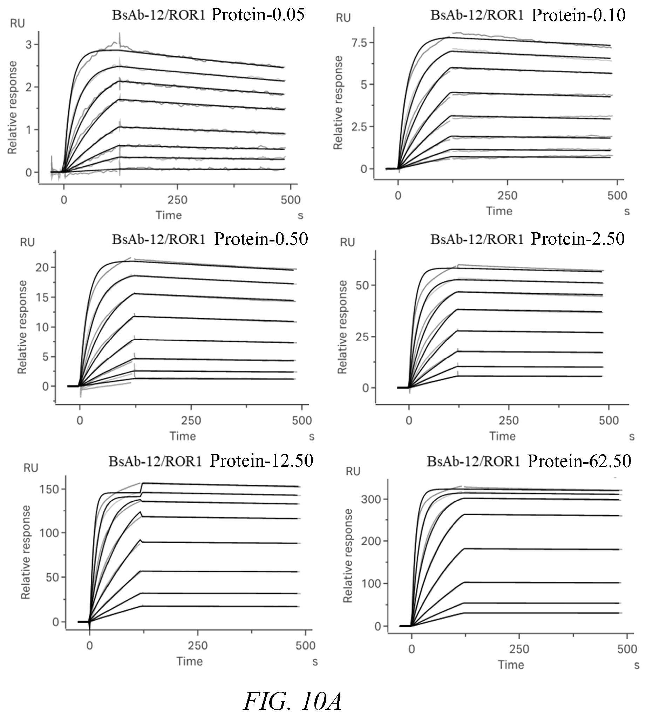

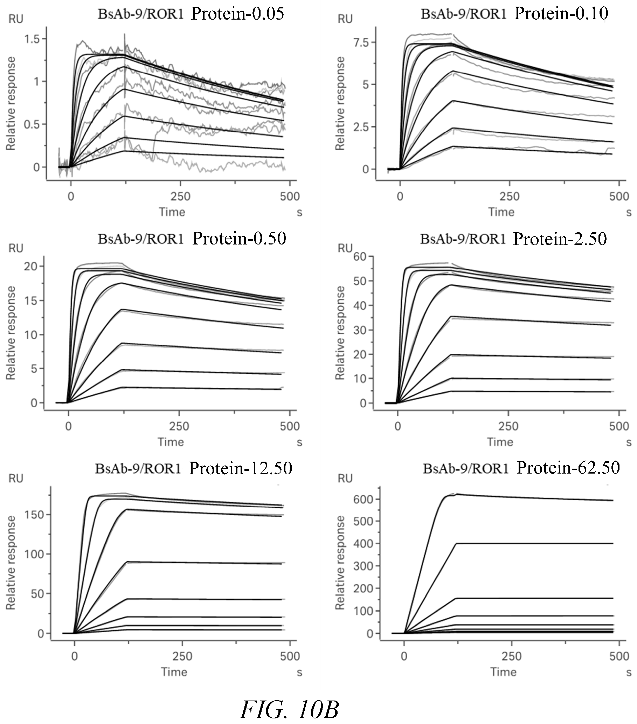

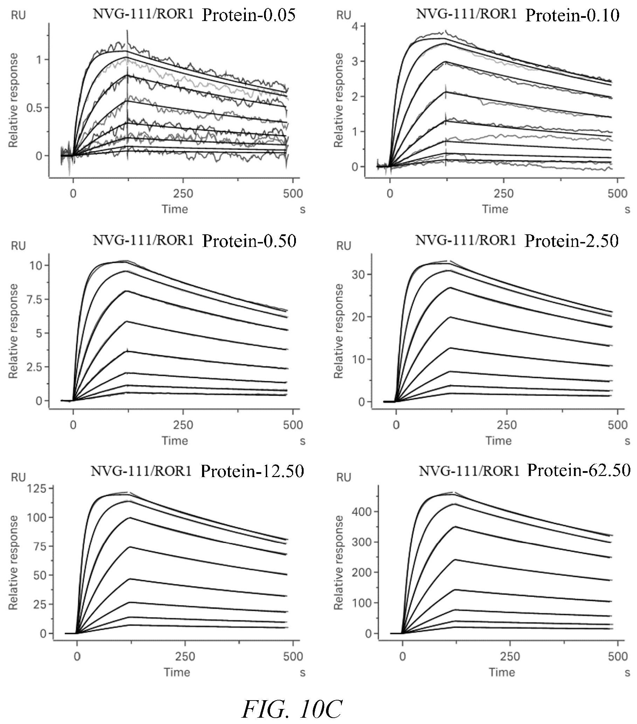

Jurkat cells expressing human CD3 and CHO-K1/ROR1 cell line overexpressing human ROR1 were collected for detection separately, and washed 3 times with PBS. 2.5 × 10⁵ detection cells and 100 µl of the purified antibody (10 µg/ml) were added to a 96-well plate, followed by incubation at 4°C for 1 hour. The cells were then washed 3 times with PBS, and 100 µl of iFluor-labelled goat anti-human IgG, Fcy-specific fragment antibody was added, followed by incubation at 4°C for 45 minutes. Finally, the cells were washed 3 times with PBS, and the signal was read with a FACSBD Calibur. The results of the cell binding detection are shown in Table 4 and FIGs. 4-10 (where FIG. 10 includes panel 10A, panel 10B, and panel 10C). The results showed that bispecific antibodies with different configurations were capable of bind to both cell lines expressing ROR1 and cell lines expressing CD3.

**Table 4 EC₅₀ of the bispecific antibody for binding to cells expressing ROR1 or cells expressing CD3**

| Bispecific antibody number | **EC₅₀(nM)** | |
|---|---|---|
| | CHOK1/ROR1 | Jurkat |
| EMB-07 | 2.386 | 4.453 |
| BsAb-15 | 0.3454 | 0.7431 |
| BsAb-1 | 0.2871 | 0.3837 |
| BsAb-4 | 0.3726 | 0.6288 |
| BsAb-2 | 2.112 | 0.3291 |
| BsAb-3 | 19.3 | 6.655 |
| BsAb-16 | 0.1095 | 0.4691 |
| BsAb-5 | 0.1087 | 0.913 |
| BsAb-8 | 0.2803 | 0.8378 |
| BsAb-6 | 0.3456 | 1.179 |
| BsAb-7 | 0.5793 | 0.526 |
| BsAb-17 | 0.1634 | 4.023 |
| BsAb-9 | 0.08644 | 1.376 |
| BsAb-12 | 0.2233 | 1.386 |
| BsAb-10 | 0.1479 | 2.843 |
| BsAb-11 | 0.4353 | 4.002 |
| Human IgG | NA | NA |

### Example 4: Detection of binding of the bispecific antibody to human ROR1 protein by SPR

The binding affinity or avidity of the bispecific antibody for human ROR1 protein was individually measured using a surface plasmon resonance (SPR) biosensor, Biacore 8K (GE Healthcare). Testing method: antigen proteins at different concentrations were immobilized on the sensor chip by amine coupling, and the bispecific antibody samples were used as the mobile phase analyte to detect the affinity level of the whole antibody molecules for the corresponding antigens. After completion of the detection run, data for dissociation rate constant (kd) and association rate constant (ka) were obtained using Biacore 8K evaluation software. The equilibrium dissociation constant (KD) was calculated as the ratio of kd to ka. The affinity differences under different antigen coupling conditions were compared and the affinity differences of antibodies were evaluated. The SPR affinity assay results are shown in Table 5 below, and the related sensorgrams are shown in FIGs 10A-10C. As shown in the test results, NVG-111 was a monovalent antibody configuration of ROR1, the results showed that the KD value did not change significantly with the increase of ROR1 protein concentration; BsAb-09 and BsAb-12 were bivalent ROR1 antibody configurations, and the results showed that the KD value decreased significantly with the increase of ROR1 protein concentration, suggesting that the higher the protein concentration, the stronger the affinity. The bivalent design of ROR1 facilitates the TCE antibody to distinguish between ROR1 tumour cells and normal cells in vivo, and the bivalent antibody exhibits stronger binding to ROR1 tumour cells.

**Table 5 Detection data of affinity of the bispecific antibody for human ROR1 protein**

| Ligand (µg/mL) | Analyte | Chi²(RU²) | ka (1/Ms) | kd (1/s) | KD (M) | Rmax (RU) |
|---|---|---|---|---|---|---|
| ROR1-0.05 | BsAb-12 | 1.71E-03 | 6.49E+05 | 4.21E-04 | 6.49E-10 | 3.2 |
| ROR1-0.10 | BsAb-12 | 1.34E-02 | 4.71E+05 | 1.69E-04 | 3.59E-10 | 8.1 |
| ROR1-0.50 | BsAb-12 | 9.01E-02 | 5.85E+05 | 2.07E-04 | 3.54E-10 | 21.7 |
| ROR1-2.50 | BsAb-12 | 1.18E+00 | 7.88E+05 | 8.37E-05 | 1.06E-10 | 60.1 |
| ROR1-12.50 | BsAb-12 | 3.59E+00 | 1.67E+06 | 5.83E-05 | 3.49E-11 | 156.3 |
| ROR1-62.50 | BsAb-12 | 7.48E+00 | 1.06E+06 | 2.83E-05 | 2.67E-11 | 324.0 |
| ROR1-0.05 | BsAb-09 | 1.63E-02 | 1.76E+07 | 1.43E-03 | 8.11E-11 | 1.3 |
| ROR1-0.10 | BsAb-09 | 4.73E-02 | 2.28E+07 | 1.13E-03 | 4.97E-11 | 7.5 |
| ROR1-0.50 | BsAb-09 | 7.24E-02 | 3.85E+07 | 8.30E-04 | 2.16E-11 | 19.7 |
| ROR1-2.50 | BsAb-09 | 2.75E-01 | 4.05E+07 | 5.21E-04 | 1.29E-11 | 55.6 |
| ROR1-12.50 | BsAb-09 | 1.35E+00 | 3.65E+07 | 2.42E-04 | 6.64E-12 | 174.1 |
| ROR1-62.50 | BsAb-09 | 9.03E-01 | 2.73E+07 | 1.87E-04 | 6.82E-12 | 648.1 |
| ROR1-0.05 | NVG-111 | 2.16E-03 | 5.32E+05 | 1.38E-03 | 2.59E-09 | 1.1 |
| ROR1-0.10 | NVG-111 | 1.20E-02 | 6.22E+05 | 1.13E-03 | 1.82E-09 | 3.7 |
| ROR1-0.50 | NVG-111 | 3.19E-03 | 6.32E+05 | 1.22E-03 | 1.92E-09 | 10.3 |
| ROR1-2.50 | NVG-111 | 2.35E-02 | 7.23E+05 | 1.18E-03 | 1.64E-09 | 33.1 |
| ROR1-12.50 | NVG-111 | 2.47E-01 | 7.33E+05 | 1.09E-03 | 1.49E-09 | 121.3 |
| ROR1-62.50 | NVG-111 | 3.08E+00 | 5.93E+05 | 1.01E-03 | 1.71E-09 | 463.9 |

### Example 5: Binding of CD3×ROR1 bispecific antibody to human ROR1 cell lines

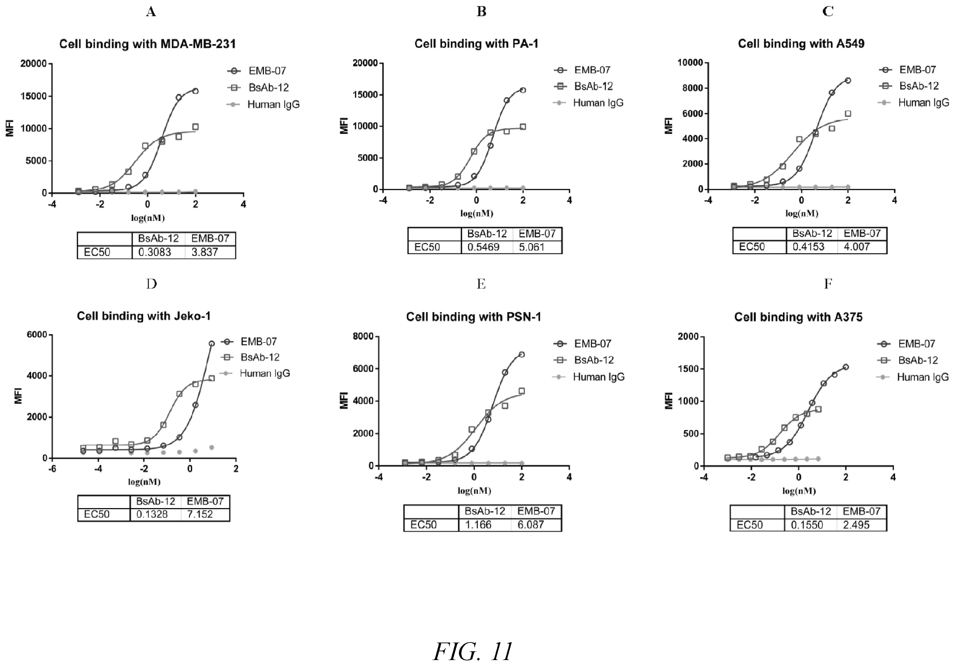

The human ROR1 tumour cell lines for detection were collected, and the cells were washed 3 times with PBS. 3 × 10⁵ detection cells were added to a 96-well plate. The antibodies were prepared at an initial concentration of 100 nM, followed by 5-fold serial dilution to generate 8 concentrations in total. 100 µl of the prepared antibodies was transferred to the 96-well plate, followed by incubation at 4°C for 1 hour. The cells were then washed twice with a FACS buffer (DPBS+1% FBS), and 100 µl of iFluor-labelled goat anti-human IgG, Fcy-specific fragment antibody (Jackson, 109-605-098) was added at a concentration of 1 µg/mL, followed by incubation at 4°C for 45 minutes. Finally, the cells were washed twice with a FACS buffer, and the signal was read with a FACS BD Calibur. As shown in the results of FIG. 11 and Table 6, the EC50 of the bispecific antibody BsAb-12 for binding to 6 ROR1 tumour cell lines are all less than that of the positive control EMB-07, indicating that BsAb-12 has stronger ROR1-binding activity.

**Table 6 EC₅₀ of CD3×ROR1 bispecific antibody for binding to ROR1 cell lines**

| **Sample Name** | **EC₅₀ (nM)** | | | | | |
|---|---|---|---|---|---|---|
| | MDA-MB-231 | PA-1 | A549 | Jeko-1 | PSN-1 | A375 |
| EMB-07 | 3.837 | 5.061 | 4.007 | 7.152 | 6.087 | 2.495 |
| BsAb-12 | 0.3083 | 0.5469 | 0.4153 | 0.1328 | 1.166 | 0.1550 |
| Human IgG1 | N/A | N/A | N/A | N/A | N/A | N/A |

### Example 6: Antigen density detection of ROR1 tumour cell lines

Target cells were collected, and washed 3 times with experimental buffer A (99% DPBS + 1% FBS). 2 × 10⁵ detection cells and 100 µl of PE anti-human ROR1 antibody (Biolegend, Cat#357804) were added to a 96-well plate, followed by incubation at 4°C for 20 minutes. Finally, after washing 3 times with experimental buffer A, 100 µL/well of experimental buffer A was added to an assay plate, and the cells were collected using a flow cytometer and then the fluorescence signal value was read. A tube of BD Quantibrite PE (BD, Cat#340395) was taken out and resuspended with 0.5 mL of experimental buffer B (99.5% DPBS + 0.5% BSA). After resuspension, the BD Quantibrite PE tube was run on the flow cytometer. All instrument settings such as PE channel voltage and compensation needed to be the same as those for the cell assay. The antigen density of the ROR1 tumour cell lines was calculated in accordance with the instructions of PE Fluorescence Quantitation Kit (BD, Cat#340395). By detecting ROR1 cell lines with different antigen densities, the difference in ROR1 expression between tumour cells and normal cells in the in vivo environment was simulated, and the results are shown in Table 7.

**Table 7. Antigen density of ROR1 tumour cell lines**

| **Cell Name** | **Antibody binding amount/1 cell** |
|---|---|
| MDA-MB-231 | 8855 |
| Jeko-1 | 4077 |
| A375 | 1083 |

### Example 7: Detection of T cell activation mediated by CD3×ROR1 bispecific antibody based on reporter gene.

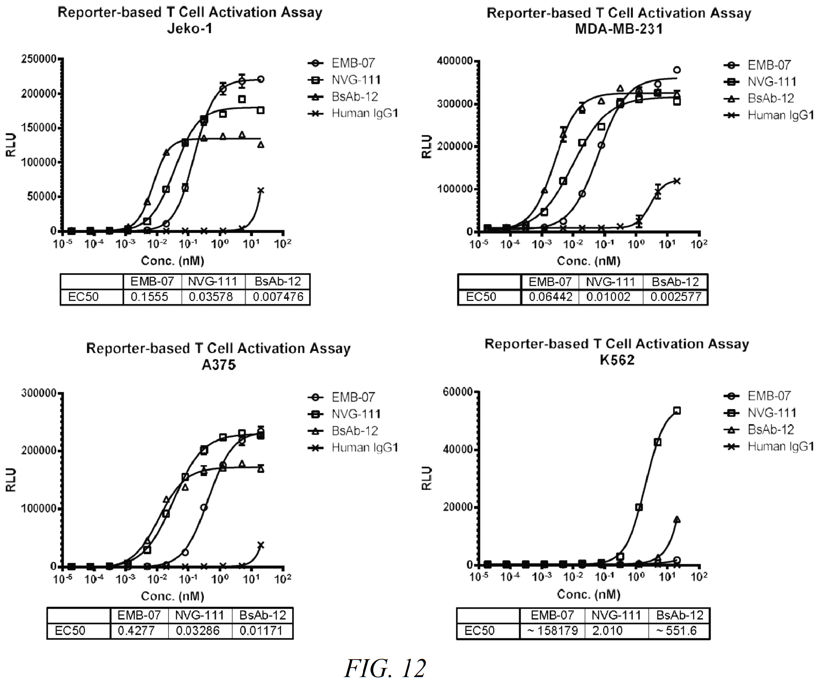

Target cells were collected, resuspended in RPMI 1640 culture medium containing 10% FBS (Gibco, Cat#22400-089), and adjusted to a density of 5 × 10⁵ cells/mL. 40 µL of the target cell suspension was transferred to the 96-well plate. NVG-111, EMB-07, and CD3×ROR1 bispecific antibody samples and negative control Human IgG1 were serially diluted 4-fold starting from the concentration of 20 nM to a total of 11 concentrations of 20 nM, 5 nM, 1.25 nM, 0.3125 nM, 0.07813 nM, 0.01953 nM, 0.004883 nM, 0.001221 nM, 0.0003052 nM, 0.00007629 nM, and 0.00001907 nM. 20 µL of each sample and control were transferred to the 96-well plate, followed by incubation at room temperature for 30 minutes. T cells containing reporter gene were collected, resuspended in RPMI 1640 culture medium containing 10% FBS (Gibco, Cat#22400-089), and adjusted to a density of 5 × 10⁵ cells/mL. 40 µL of the target cell suspension was transferred to the 96-well plate. The culture plate was then transferred to an incubator and incubated at 37°C with 5% CO₂ for 6 hours. The luciferase detection reagent working solution was formulated. The incubated assay plate was removed from the cell incubator, the luciferase detection reagent working solution was transferred into the corresponding wells of the assay plate, followed by incubation at room temperature for 3-5 minutes. The chemiluminescence values were read using a PHERA star microplate reader and the data were recorded. MDA-MB-231, Jeko-1, and A375 were used to simulate three different expression levels of ROR1, and K562 cells (ROR1-negative) served as the control. As shown in FIG. 12 and Table 8, on MDA-MB-231 and Jeko-1 cells, the EC₅₀ of T cell activation mediated by the bispecific antibody BsAb-12 based on reporter gene was significantly lower than that of EMB-07 and NVG-111. On A375 cells with low expression of ROR1, the activation window of T cell activation mediated by BsAb-12 based on reporter gene was lower than that of EMB-07 and NVG-111, and EC₅₀ was not significantly different from that of NVG-111. The results suggested that BsAb-12 showed different activation intensities in cell lines with high and low expression of ROR1.

**Table 8. In vitro experiment of T cell activation mediated by CD3 × ROR1 bispecific antibody based on reporter gene.**

| **Sample Name** | **EC₅₀(nM)** | | | |
|---|---|---|---|---|
| | **Jeko-1** | **MDA-MB-231** | **A375** | **K562** |
| EMB-07 | 0.1555 | 0.06442 | 0.4277 | N/A |
| NVG-111 | 0.03578 | 0.01002 | 0.03286 | 2.010 |
| BsAb-12 | 0.007476 | 0.002577 | 0.01171 | N/A |
| Human IgG1 | N/A | N/A | N/A | N/A |

### Example 8: CD3×ROR1 bispecific antibody-mediated in vitro killing assay of PBMC cells (Donor: NF0058)

Target cells (Jeko-1 and PSN-1) were collected, resuspended in MEM-α medium (Gibco, Cat#41061-029) containing 1% FBS, and adjusted to a density of 1 × 10⁵ cells/mL. 50 µL of target cell suspension was transferred into a 96-well plate. NVG-111, EMB-07, and CD3×ROR1 bispecific antibody samples and negative control Human IgG1 were serially diluted 3-fold starting from the concentration of 0.2 nM to a total of 7 concentrations of 0.2 nM, 0.066667 nM, 0.022222 nM, 0.0074074 nM, 0.0024691 nM, 0.00082305 nM, 0.00027435 nM. 50 µL of each sample and control were transferred to the 96-well plate, followed by incubation at room temperature for 30 minutes. After thawing the cryopreserved PBMC (Allcells, FPB004F-C, Donor: NF0058), they were resuspended in MEM-α medium containing 1% FBS, and adjusted to a density of 2.5 × 10⁶ cells/mL. 100 µL of effector cell suspension, 100 µL of 2% Triton X-100 (J&K Scientific, Cat#993361) (as the maximum killing signal value) or 100 µL of MEM-α medium containing 1% FBS (as the minimum killing signal value) were transferred into the 96-well plate, respectively. The culture plate was then transferred to an incubator, and incubated at 37°C with 5% CO₂ for 24 hours. After 24 hours of incubation, the culture plate was taken out and centrifuged at 2000 rpm for 5 minutes. Then, 50 µL of supernatant was aspirated to another 96-well plate, and LDH detection reagent (Roche, Cat#11644793001) was added. The absorbance values were measured using a microplate reader (PHERAstar FSX, BMG) at a detection wavelength of OD492 nm and a reference wavelength of OD650 nm.

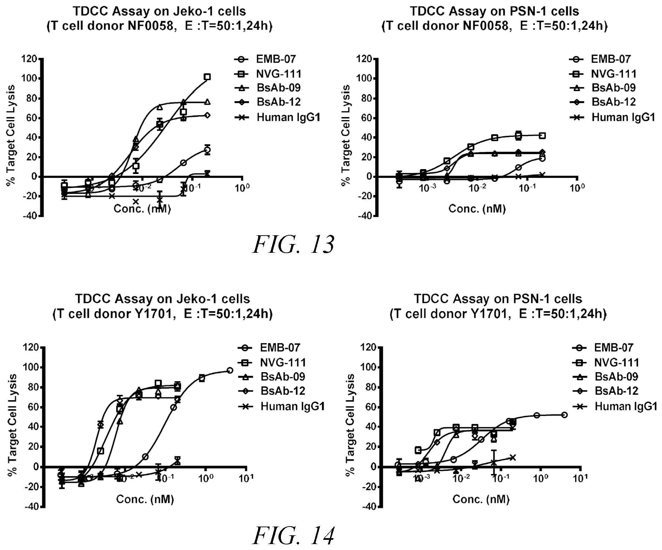

Killing calculation: Subtracting the background absorbance at OD650 nm from the absorbance at OD492 nm. The calculation formula was: % target cell lysis rate = 100 * (ODₛₐₘₚₗₑ - OD_{target cells+effector cells})/(OD_{maximum killing signal} - OD_{minimum killing signal}). The experimental results are shown in Table 9 and FIG. 13: Bispecific antibody samples BsAb-09 and BsAb-12 effectively mediated the killing against the Jeko-1 target cells by PBMCs. When the antibody concentration reached 0.2 nM, the killing rate may reach over 60%. Furthermore, the killing rate of by PBMC cells against PSN-1 target cells mediated by bispecific antibody samples BsAb-09 and BsAb-12 was less than 30%, while the negative control Human IgG1 did not have the effect of mediating the killing against target cells by PBMC cells.

**Table 9. CD3×ROR1 bispecific antibody-mediated in vitro killing assay of PBMC cells (Donor: NF0058)**

| **Sample Name** | **EC₅₀(nM)** | |
|---|---|---|
| | **Jeko-1** | **PSN-1** |
| EMB-07 | 0.05428 | 0.06205 |
| NVG-111 | 0.02967 | 0.003483 |
| BsAb-09 | 0.006424 | 0.00344 |
| BsAb-12 | 0.005585 | 0.003402 |
| Human IgG1 | N/A | N/A |

### Example 9: CD3×ROR1 bispecific antibody-mediated in vitro killing assay of PBMC cells (Donor: Y1701)

Target cells (Jeko-1 and PSN-1) were collected, resuspended in MEM-α medium (Gibco, Cat#41061-029) containing 1% FBS, and adjusted to a density of 1 × 10⁵ cells/mL. 50 µL of target cell suspension was transferred into a 96-well plate. The concentration of EMB-07 was increased and EMB-07 was serially diluted 5-fold starting from the concentration of 4 nM to a total of 7 concentrations of 4 nM, 0.8 nM, 0.16 nM, 0.032 nM, 0.0064 nM, 0.00128 nM, and 0.000256 nM, respectively. NVG-111 and CD3×ROR1 bispecific antibody samples and negative control Human IgG1 were serially diluted 3-fold starting from the concentration of 0.2 nM to a total of 7 concentrations of 0.2 nM, 0.066667 nM, 0.022222 nM, 0.0074074 nM, 0.0024691 nM, 0.00082305 nM, 0.00027435 nM. 50 µL of each sample and control were transferred to the 96-well plate, followed by incubation at room temperature for 30 minutes. After thawing the cryopreserved PBMC (Allcells, FPB004F-C, Donor: Y1701), they were resuspended in MEM-α medium containing 1% FBS, and adjusted to a density of 2.5 × 10 ⁶ cells/mL. 100 µL of effector cell suspension, 100 µL of 2% Triton X-100 (J&K Scientific, Cat#993361) (serving as the maximum killing signal value) or 100 µL of MEM-α medium containing 1% FBS (serving as the minimum killing signal value) were transferred into the 96-well plate, respectively. The culture plate was then transferred to an incubator, and incubated at 37°C with 5% CO₂ for 24 hours. After 24 hours of incubation, the culture plate was taken out and centrifuged at 2000 rpm for 5 minutes. Then, 50 µL of supernatant was aspirated to another 96-well plate, and LDH detection reagent (Roche, Cat#11644793001) was added. The absorbance values were measured using a microplate reader (PHERAstar FSX, BMG) at a detection wavelength of OD492 nm and a reference wavelength of OD650 nm.

Killing calculation: Subtracting the background absorbance at OD650 nm from the absorbance at OD492 nm. The calculation formula was: % target cell lysis rate = 100 * (ODₛₐₘₚₗₑ - OD_{target cells+effector cells})/(ODₘₐₓᵢₘᵤₘ killing signal - ODminimum _{killing signal}). The experimental results are shown in Table 10 and FIG. 14: Bispecific antibody samples BsAb-09 and BsAb-12 effectively mediated the killing against the Jeko-1 target cells by PBMCs. When the antibody concentration reached 0.2 nM, the killing rate may reach over 60%. Furthermore, the killing rate of by PBMC cells against PSN-1 target cells mediated by bispecific antibody samples BsAb-09 and BsAb-12 was less than 50%, while the negative control Human IgG1 did not have the effect of mediating the killing against target cells by PBMC cells.

**Table 10. CD3×ROR1 bispecific antibody-mediated in vitro killing assay of PBMC cells (Donor: Y1701)**

| **Sample Name** | **EC₅₀(nM)** | |
|---|---|---|
| | **Jeko-1** | **PSN-1** |
| EMB-07 | 0.09910 | 0.03358 |
| NVG-111 | 0.003523 | ~0.002064 |
| BsAb-09 | 0.005921 | 0.004098 |
| BsAb-12 | 0.001981 | 0.001556 |
| Human IgG1 | N/A | N/A |

### Example 10: CD3×ROR1 bispecific antibody-mediated in vitro cytokine release assay of PBMC cells (Donor: NF0058)

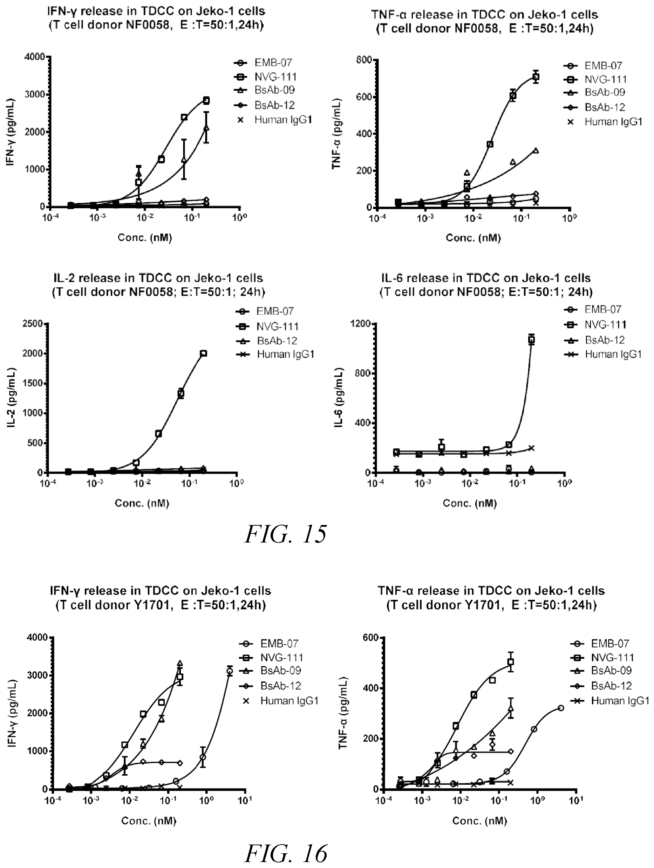

Target cells (Jeko-1) were collected, resuspended in MEM-α medium (Gibco, Cat#41061-029) containing 1% FBS, and adjusted to a density of 1 × 10⁵ cells/mL. 50 µL of target cell suspension was transferred into a 96-well plate. NVG-111, EMB-07, and CD3×ROR1 bispecific antibody samples and negative control Human IgG1 were serially diluted 3-fold starting from the concentration of 0.2 nM to a total of 7 concentrations of 0.2 nM, 0.066667 nM, 0.022222 nM, 0.0074074 nM, 0.0024691 nM, 0.00082305 nM, 0.00027435 nM. 50 µL of each sample and control were transferred to the 96-well plate, followed by incubation at room temperature for 30 minutes. After thawing the cryopreserved PBMC (Allcells, FPB004F-C, Donor: NF0058), they were resuspended in MEM-α medium containing 1% FBS, and adjusted to a density of 2.5 × 10⁶ cells/mL. 100 µL of effector cell suspension was transferred into the 96-well plate, respectively. The culture plate was then transferred to an incubator, and incubated at 37°C with 5% CO₂ for 24 hours. After 24 hours of incubation, the culture plate was taken out and centrifuged at 2000 rpm for 5 minutes. Then, 50 µL of supernatant was aspirated. The concentrations of IFN-γ and TNF-α in the supernatant were detected in accordance with the instructions of the IFN-γ detection kit (Cisbio, Cat#62HIFNGPEH) and TNF-α detection kit (Cisbio, Cat#62HTNFAPEH). The results are shown in Tables 11-14 and FIG. 15: EMB-07 and the negative control Human IgG1 at a concentration of 0.2 nM did not have the effect of significantly promoting cytokine release from PBMCs. The bispecific antibody sample BsAb-12 at the concentration 0.2 nM induced significantly lower secretion of the cytokines IFN-γ, TNF-α, IL-2, and IL-6 compared to NVG-111. The results suggested that BsAb-12 significantly reduced cytokine secretion while maintaining the killing activity against target cells.

**Table 11. CD3×ROR1 bispecific antibody-mediated in vitro IFN-γ release assay of PBMC cells (Donor: NF0058)**

| **Sample Name** | **Span** | **EC₅₀(nM)** |
|---|---|---|
| EMB-07 | 32 | N/A |
| NVG-111 | 2808 | 0.02755 |
| BsAb-09 | 2078 | N/A |
| BsAb-12 | 150 | 0.05746 |
| Human IgG1 | 0.46 | N/A |

**Table 12. CD3×ROR1 bispecific antibody-mediated in vitro TNF-α release assay of PBMC cells (Donor: NF0058)**

| **Sample Name** | **Span** | **EC₅₀(nM)** |
|---|---|---|
| EMB-07 | 23 | N/A |
| NVG-111 | 677 | 0.02478 |
| BsAb-09 | 289 | N/A |
| BsAb-12 | 55 | 0.01908 |
| Human IgG1 | 5 | N/A |

**Table 13. CD3×ROR1 bispecific antibody-mediated in vitro IL-2 release assay of PBMC cells (Donor: NF0058)**

| **Sample Name** | **Span** | **EC₅₀(nM)** |
|---|---|---|
| EMB-07 | 10 | N/A |
| NVG-111 | 1983 | 0.05540 |
| BsAb-12 | 67 | N/A |
| Human IgG1 | 32 | N/A |

**Table 14. CD3×ROR1 bispecific antibody-mediated in vitro IL-6 release assay of PBMC cells (Donor: NF0058)**

| **Sample Name** | **Span** | **EC₅₀(nM)** |
|---|---|---|
| EMB-07 | 12 | N/A |
| NVG-111 | 905 | 0.07997 |
| BsAb-12 | 8 | N/A |
| Human IgG1 | 50 | N/A |

### Example 11: CD3×ROR1 bispecific antibody-mediated in vitro cytokine release assay of PBMC cells (Donor: Y1701)

Target cells (Jeko-1) were collected, resuspended in MEM-α medium (Gibco, Cat#41061-029) containing 1% FBS, and adjusted to a density of 1 × 10⁵ cells/mL. 50 µL of target cell suspension was transferred into a 96-well plate. The concentration of EMB-07 was increased and EMB-07 was serially diluted 5-fold starting from the concentration of 4 nM to a total of 7 concentrations of 4 nM, 0.8 nM, 0.16 nM, 0.032 nM, 0.0064 nM, 0.00128 nM, and 0.000256 nM, respectively. NVG-111 and CD3×ROR1 bispecific antibody samples and negative control Human IgG1 were serially diluted 3-fold starting from the concentration of 0.2 nM to a total of 7 concentrations of 0.2 nM, 0.066667 nM, 0.022222 nM, 0.0074074 nM, 0.0024691 nM, 0.00082305 nM, 0.00027435 nM. 50 µL of each sample and control were transferred to the 96-well plate, followed by incubation at room temperature for 30 minutes. After thawing the cryopreserved PBMC (Allcells, FPB004F-C, Donor: Y1701), they were resuspended in MEM-α medium containing 1% FBS, and adjusted to a density of 2.5 × 10⁶ cells/mL. 100 µL of effector cell suspension was transferred into the 96-well plate, respectively. The culture plate was then transferred to an incubator, and incubated at 37°C with 5% CO₂ for 24 hours. After 24 hours of incubation, the culture plate was taken out and centrifuged at 2000 rpm for 5 minutes. Then, 50 µL of supernatant was aspirated. The concentrations of IFN-γ and TNF-α in the supernatant were detected in accordance with the instructions of the IFN-γ detection kit (Cisbio, Cat#62HIFNGPEH) and TNF-α detection kit (Cisbio, Cat#62HTNFAPEH). The results are shown in Table 15, Table 16 and FIG. 16: The negative control Human IgG1 did not have the effect of promoting cytokine release from PBMCs. The bispecific antibody samples BsAb-09 (0.2 nM), NVG-111 (0.2 nM) and EMB-07 (4 nM) induced comparable concentrations of the cytokines IFN-γ and TNF-α released by PBMCs, and the bispecific antibody sample BsAb-12 significantly reduced the concentrations of cytokines IFN-γ and TNF-α released by PBMCs.

**Table 15. CD3×ROR1 bispecific antibody-mediated in vitro IFN-γ release assay of PBMC cells (Donor: Y1701)**

| **Sample Name** | **Span** | **EC₅₀(nM)** |
|---|---|---|
| EMB-07 | 3093 | 9.360 |
| NVG-111 | 2955 | 0.01265 |
| BsAb-09 | 3296 | N/A |
| BsAb-12 | 590 | 0.003543 |
| Human IgG1 | 20 | N/A |

**Table 16. CD3×ROR1 bispecific antibody-mediated in vitro TNF-α release assay of PBMC cells (Donor: Y1701)**

| **Sample Name** | **Span** | **EC₅₀(nM)** |
|---|---|---|
| EMB-07 | 308 | 0.4744 |
| NVG-111 | 485 | 0.008297 |
| BsAb-09 | 300 | N/A |
| BsAb-12 | 115 | 0.001925 |
| Human IgG1 | 6 | N/A |

### Example 12: Experiment on the anti-tumour in vivo efficacy of CD3×ROR1 bispecific antibody in C-NKG mice transplanted with MDA-MB-231 breast cancer cells and human PBMC.

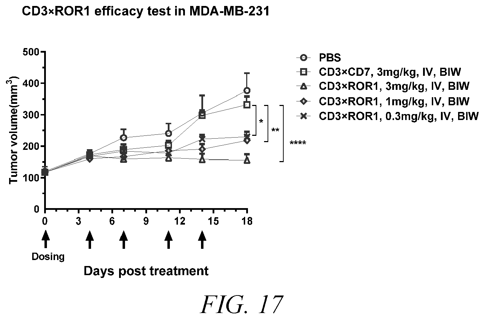

Anti-tumour efficacy was evaluated in C-NKG mice (Cyagen Biosciences Inc. (Suzhou)). C-NKG is a severely immunodeficient mouse lacking T cells, B cells, and natural killer cells. On Day 0, 5 x 10⁶ human PBMCs were transplanted into mice by tail vein injection. On day 4, each mouse was transplanted with 10 x 10⁶ MDA-MB-231 cells via subcutaneous injection in a volume of 0.2 mL. On day 9, 100 µL of peripheral blood was collected from each mouse to detect the reconstitution level of human CD45-positive cells. On day 10, the animals were randomly grouped based on tumour size (about 100-120 mm³), and drug administration and treatment were initiated. Tumour growth was detected by caliper measurement. The experiment was terminated 18 days after the first administration, and the mice were euthanized upon showing signs of severe GVHD. BsAb-12 CD3×ROR1 (3 mg/kg, 1 mg/kg, and 0.3 mg/kg), CD3×CD7 (3 mg/kg), or vehicle (PBS) was administered twice weekly for a total of five doses via tail vein injection. The CD7 VHH sequence of the control group CD3×CD7 control bispecific antibody was derived from clone AHP19509 in Patent No. CN 117362437 A. Both the bispecific antibody and BsAb-12 of the present invention had a configuration of 1+1+1, and the bispecific antibody had a sequence and overall configuration except for the CD7 sequence completely identical to those of BsAb-12. As shown in FIG. 17, the mice in the CD3×ROR1 (3 mg/kg, 1 mg/kg, and 0.3 mg/kg) treatment group exhibit a significant tumour growth inhibition compared to the CD3×CD7 (3 mg/kg) group, indicating that the bispecific antibody of the present invention has an effect of inhibiting tumour growth, which effect is not produced by CD3 alone. (****P < 0.0001, ***P < 0.001, **P < 0.01, *P < 0.05; compared with the CD3×CD7 (3 mg/kg) group, using one-way ANOVA test)

### Sequence Information:

**Amino acid sequence of BsAb-1 Chain 1: SEQ ID NO: 1** 
EVQLVESGGGLVQPGGSLRLSCAASGFISRFYAMGWYRQAPGKQRELVAR
ISSMGWTYYADSVKGRFTISIDNSKNTLYLQMNSLRAEDTAVYYCNARNL
WGQGTLVTVSSDKTHTCPPCPAPESTRGPSVFLFPPKPKDTLMISRTPEVTC
 
VVVDVSHEDPEVKFNWYVDGVEVHNAKTKPREEQYNSTYRVVSVLTVLH
QDWLNGKEYKCKVSNKALPAPIEKTISKAKGQPREPQVCTLPPSRDELTKN
QVSLSCAVKGFYPSDIAVEWESNGQPENNYKTTPPVLDSDGSFFLVSKLTV
DKSRWQQGNVFSCSVMHEALHNHYTQKSLSLSPGK
**Amino acid sequence of BsAb-1 Chain 2: SEQ ID NO: 2** 
EVQLVESGGGLVQPGRSLRLSCAASGFTFNTYAMNWVRQAPGKGLEWVA
RIRSKYNNYATYYADSVKDRFTISRDDSKSIAYLQMNSLKTEDTAVYYCV
RHGNFGNSYVSWFAYWGQGTLVTVSSASTKGPSVFPLAPSSKSTSGGTAA
LGCLVKDYFPEPVTVSWNSGALTSGVHTFPAVLQSSGLYSLSSWTVPSSSL
GTQTYICNVNHKPSNTKVDKKVEPKSCDKTHTCPPCPAPESTRGPSVFLFPP
KPKDTLMISRTPEVTCVVVDVSHEDPEVKFNWYVDGVEVHNAKTKPREE
QYNSTYRWSVLTVLHQDWLNGKEYKCKVSNKALPAPIEKTISKAKGQPRE
PQVYTLPPCRDELTKNQVSLWCLVKGFYPSDIAVEWESNGQPENNYKTTP
PVLDSDGSFFLYSKLTVDKSRWQQGNVFSCSVMHEALHNHYTQKSLSLSP
GK
**Amino acid sequence of BsAb-1 Chain 3: SEQ ID NO: 3** 
QTVVTQEPSLTVSPGGTVTLTCRSSTGAVTTSNYANWVQQKPGQAPRGLI
GGTNKRAPGVPARFSGSLLGGKAALTLSGVQPEDEAEYFCALWYSNLWV
FGGGTKLTVLGQPKAAPSVTLFPPSSEELQANKATLVCLISDFYPGAVTVA
WKADSSPVKAGVETTTPSKQSNNKYAASSYLSLTPEQWKSHRSYSCQVTH
EGSTVEKTVAPTECS
**Amino acid sequence of BsAb-2 Chain 1: SEQ ID NO: 4** 
EVQLVESGGGLVQPGGSLRLSCAASYFISRFYAVGWYRQAPGKQRELVAR
ISSGGWTYYADSVKGRFTISIDNSKNTLYLQMNSLRAEDTAVYYCNARNL
WGQGTLVTVSSDKTHTCPPCPAPESTRGPSVFLFPPKPKDTLMISRTPEVTC
VVVDVSHEDPEVKFNWYVDGVEVHNAKTKPREEQYNSTYRVVSVLTVLH
QDWLNGKEYKCKVSNKALPAPIEKTISKAKGQPREPQVCTLPPSRDELTKN
QVSLSCAVKGFYPSDIAVEWESNGQPENNYKTTPPVLDSDGSFFLVSKLTV
DKSRWQQGNVFSCSVMHEALHNHYTQKSLSLSPGK
**Amino acid sequence of BsAb-2 Chain 2: SEQ ID NO: 2** 
EVQLVESGGGLVQPGRSLRLSCAASGFTFNTYAMNWVRQAPGKGLEWVA
RIRSKYNNYATYYADSVKDRFTISRDDSKSIAYLQMNSLKTEDTAVYYCV
RHGNFGNSYVSWFAYWGQGTLVTVSSASTKGPSVFPLAPSSKSTSGGTAA
LGCLVKDYFPEPVTVSWNSGALTSGVHTFPAVLQSSGLYSLSSWTVPSSSL
 
GTQTYICNVNHKPSNTKVDKKVEPKSCDKTHTCPPCPAPESTRGPSVFLFPP
KPKDTLMISRTPEVTCVVVDVSHEDPEVKFNWYVDGVEVHNAKTKPREE
QYNSTYRWSVLTVLHQDWLNGKEYKCKVSNKALPAPIEKTISKAKGQPRE
PQVYTLPPCRDELTKNQVSLWCLVKGFYPSDIAVEWESNGQPENNYKTTP
PVLDSDGSFFLYSKLTVDKSRWQQGNVFSCSVMHEALHNHYTQKSLSLSP
GK
**Amino acid sequence of BsAb-2 Chain 3: SEQ ID NO: 3** 
QTVVTQEPSLTVSPGGTVTLTCRSSTGAVTTSNYANWVQQKPGQAPRGLI
GGTNKRAPGVPARFSGSLLGGKAALTLSGVQPEDEAEYFCALWYSNLWV
FGGGTKLTVLGQPKAAPSVTLFPPSSEELQANKATLVCLISDFYPGAVTVA
WKADSSPVKAGVETTTPSKQSNNKYAASSYLSLTPEQWKSHRSYSCQ
VTHEGSTVEKTVAPTECS
**Amino acid sequence of BsAb-3 Chain 1: SEQ ID NO: 5** 
QVQLVESGGGVVQPGGSLRLSCAASGFVLRVNTVSWYRQAPGKQRELVA
LITSESNEYYDDSVKGRFTISRDNAKNTIYLHMNSLKPEDTGVYFCNFITTW
GQGTQVTVSSDKTHTCPPCPAPESTRGPSVFLFPPKPKDTLMISRTPEVTCV
VVDVSHEDPEVKFNWYVDGVEVHNAKTKPREEQYNSTYRVVSVLTVLHQ
DWLNGKEYKCKVSNKALPAPIEKTISKAKGQPREPQVCTLPPSRDELTKNQ
VSLSCAVKGFYPSDIAVEWESNGQPENNYKTTPPVLDSDGSFFLVSKLTVD
KSRWQQGNVFSCSVMHEALHNHYTQKSLSLSPGK
**Amino acid sequence of BsAb-3 Chain 2: SEQ ID NO: 2** 
EVQLVESGGGLVQPGRSLRLSCAASGFTFNTYAMNWVRQAPGKGLEWVA
RIRSKYNNYATYYADSVKDRFTISRDDSKSIAYLQMNSLKTEDTAVYYCV
RHGNFGNSYVSWFAYWGQGTLVTVSSASTKGPSVFPLAPSSKSTSGGTAA
LGCLVKDYFPEPVTVSWNSGALTSGVHTFPAVLQSSGLYSLSSWTVPSSSL
GTQTYICNVNHKPSNTKVDKKVEPKSCDKTHTCPPCPAPESTRGPSVFLFPP
KPKDTLMISRTPEVTCVVVDVSHEDPEVKFNWYVDGVEVHNAKTKPREE
QYNSTYRWSVLTVLHQDWLNGKEYKCKVSNKALPAPIEKTISKAKGQPRE
PQVYTLPPCRDELTKNQVSLWCLVKGFYPSDIAVEWESNGQPENNYKTTP
PVLDSDGSFFLYSKLTVDKSRWQQGNVFSCSVMHEALHNHYTQKSLSLSP
GK
**Amino acid sequence of BsAb-3 Chain 3: SEQ ID NO: 3** 
QTVVTQEPSLTVSPGGTVTLTCRSSTGAVTTSNYANWVQQKPGQAPRGLI
GGTNKRAPGVPARFSGSLLGGKAALTLSGVQPEDEAEYFCALWYSNLWV
 
FGGGTKLTVLGQPKAAPSVTLFPPSSEELQANKATLVCLISDFYPGAVTVA
WKADSSPVKAGVETTTPSKQSNNKYAASSYLSLTPEQWKSHRSYSCQ
VTHEGSTVEKTVAPTECS
**Amino acid sequence of BsAb-4 Chain 1: SEQ ID NO: 6** 
EVQLVESGGGLVQPGGSLRLSCAASGRSFSSFQMGWFRQAPGKEREFVAA
SGWSGGPTYYWDSVIGRFTISTDNSKNTLYLQMNSLRAEDTAVYYCYEDR
VLSGRPVRYWGQGTLVTVSSDKTHTCPPCPAPESTRGPSVFLFPPKPKDTL
MISRTPEVTCVVVDVSHEDPEVKFNWYVDGVEVHNAKTKPREEQYNSTY
RVVSVLTVLHQDWLNGKEYKCKVSNKALPAPIEKTISKAKGQPREPQVCT
LPPSRDELTKNQVSLSCAVKGFYPSDIAVEWESNGQPENNYKTTPPVLDSD
GSFFLVSKLTVDKSRWQQGNVFSCSVMHEALHNHYTQKSLSLSPGK
**Amino acid sequence of BsAb-4 Chain 2: SEQ ID NO: 2** 
EVQLVESGGGLVQPGRSLRLSCAASGFTFNTYAMNWVRQAPGKGLEWVA
RIRSKYNNYATYYADSVKDRFTISRDDSKSIAYLQMNSLKTEDTAVYYCV
RHGNFGNSYVSWFAYWGQGTLVTVSSASTKGPSVFPLAPSSKSTSGGTAA
LGCLVKDYFPEPVTVSWNSGALTSGVHTFPAVLQSSGLYSLSSWTVPSSSL
GTQTYICNVNHKPSNTKVDKKVEPKSCDKTHTCPPCPAPESTRGPSVFLFPP
KPKDTLMISRTPEVTCVVVDVSHEDPEVKFNWYVDGVEVHNAKTKPREE
QYNSTYRWSVLTVLHQDWLNGKEYKCKVSNKALPAPIEKTISKAKGQPRE
PQVYTLPPCRDELTKNQVSLWCLVKGFYPSDIAVEWESNGQPENNYKTTP
PVLDSDGSFFLYSKLTVDKSRWQQGNVFSCSVMHEALHNHYTQKSLSLSP
GK
**Amino acid sequence of BsAb-4 Chain 3: SEQ ID NO: 3** 
QTVVTQEPSLTVSPGGTVTLTCRSSTGAVTTSNYANWVQQKPGQAPRGLI
GGTNKRAPGVPARFSGSLLGGKAALTLSGVQPEDEAEYFCALWYSNLWV
FGGGTKLTVLGQPKAAPSVTLFPPSSEELQANKATLVCLISDFYPGAVTVA
WKADSSPVKAGVETTTPSKQSNNKYAASSYLSLTPEQWKSHRSYSCQVTH
EGSTVEKTVAPTECS
**Amino acid sequence of BsAb-15 Chain 1: SEQ ID NO: 7** 
EVQLVESGGGLVQPGGSLRLSCAASGRSFSSFQMGWFRQAPGKEREFVAA
SGWSGGPTYYWDSVIGRFTISTDNSKNTLYLQMNSLRAEDTAVYYCYEDR
VLSGRHVRYWGQGTLVTVSSDKTHTCPPCPAPESTRGPSVFLFPPKPKDTL
MISRTPEVTCVVVDVSHEDPEVKFNWYVDGVEVHNAKTKPREEQYNSTY
RVVSVLTVLHQDWLNGKEYKCKVSNKALPAPIEKTISKAKGQPREPQVCT
 
LPPSRDELTKNQVSLSCAVKGFYPSDIAVEWESNGQPENNYKTTPPVLDSD
GSFFLVSKLTVDKSRWQQGNVFSCSVMHEALHNHYTQKSLSLSPGK
**Amino acid sequence of BsAb-15 Chain 2: SEQ ID NO: 2** 
EVQLVESGGGLVQPGRSLRLSCAASGFTFNTYAMNWVRQAPGKGLEWVA
RIRSKYNNYATYYADSVKDRFTISRDDSKSIAYLQMNSLKTEDTAVYYCV
RHGNFGNSYVSWFAYWGQGTLVTVSSASTKGPSVFPLAPSSKSTSGGTAA
LGCLVKDYFPEPVTVSWNSGALTSGVHTFPAVLQSSGLYSLSSWTVPSSSL
GTQTYICNVNHKPSNTKVDKKVEPKSCDKTHTCPPCPAPESTRGPSVFLFPP
KPKDTLMISRTPEVTCVVVDVSHEDPEVKFNWYVDGVEVHNAKTKPREE
QYNSTYRWSVLTVLHQDWLNGKEYKCKVSNKALPAPIEKTISKAKGQPRE
PQVYTLPPCRDELTKNQVSLWCLVKGFYPSDIAVEWESNGQPENNYKTTP
PVLDSDGSFFLYSKLTVDKSRWQQGNVFSCSVMHEALHNHYTQKSLSLSP
GK
**Amino acid sequence of BsAb-15 Chain 3: SEQ ID NO: 3** 
QTVVTQEPSLTVSPGGTVTLTCRSSTGAVTTSNYANWVQQKPGQAPRGLI
GGTNKRAPGVPARFSGSLLGGKAALTLSGVQPEDEAEYFCALWYSNLWV
FGGGTKLTVLGQPKAAPSVTLFPPSSEELQANKATLVCLISDFYPGAVTVA
WKADSSPVKAGVETTTPSKQSNNKYAASSYLSLTPEQWKSHRSYSCQ
VTHEGSTVEKTVAPTECS
**Amino acid sequence of BsAb-5 Chain 1: SEQ ID NO: 8** 
EVQLVESGGGLVQPGGSLRLSCAASGFISRFYAMGWYRQAPGKQRELVAR
ISSMGWTYYADSVKGRFTISIDNSKNTLYLQMNSLRAEDTAVYYCNARNL
WGQGTLVTVSSGGGGSGGGGSGGGGSEVQLVESGGGLVQPGGSLRLSCA
ASGFISRFYAMGWYRQAPGKQRELVARISSMGWTYYADSVKGRFTISIDN
SKNTLYLQMNSLRAEDTAVYYCNARNLWGQGTLVTVSSDKTHTCPPCPA
PESTRGPSVFLFPPKPKDTLMISRTPEVTCVVVDVSHEDPEVKFNWYVDGV
EVHNAKTKPREEQYNSTYRVVSVLTVLHQDWLNGKEYKCKVSNKALPAP
IEKTISKAKGQPREPQVCTLPPSRDELTKNQVSLSCAVKGFYPSDIAVEWES
NGQPENNYKTTPPVLDSDGSFFLVSKLTVDKSRWQQGNVFSCSVMHEALH
NHYTQKSLSLSPGK
**Amino acid sequence of BsAb-5 Chain 2: SEQ ID NO: 2** 
EVQLVESGGGLVQPGRSLRLSCAASGFTFNTYAMNWVRQAPGKGLEWVA
RIRSKYNNYATYYADSVKDRFTISRDDSKSIAYLQMNSLKTEDTAVYYCV
RHGNFGNSYVSWFAYWGQGTLVTVSSASTKGPSVFPLAPSSKSTSGGTAA
 
LGCLVKDYFPEPVTVSWNSGALTSGVHTFPAVLQSSGLYSLSSWTVPSSSL
GTQTYICNVNHKPSNTKVDKKVEPKSCDKTHTCPPCPAPESTRGPSVFLFPP
KPKDTLMISRTPEVTCVVVDVSHEDPEVKFNWYVDGVEVHNAKTKPREE
QYNSTYRWSVLTVLHQDWLNGKEYKCKVSNKALPAPIEKTISKAKGQPRE
PQVYTLPPCRDELTKNQVSLWCLVKGFYPSDIAVEWESNGQPENNYKTTP
PVLDSDGSFFLYSKLTVDKSRWQQGNVFSCSVMHEALHNHYTQKSLSLSP
GK
**Amino acid sequence of BsAb-5 Chain 3: SEQ ID NO: 3** 
QTVVTQEPSLTVSPGGTVTLTCRSSTGAVTTSNYANWVQQKPGQAPRGLI
GGTNKRAPGVPARFSGSLLGGKAALTLSGVQPEDEAEYFCALWYSNLWV
FGGGTKLTVLGQPKAAPSVTLFPPSSEELQANKATLVCLISDFYPGAVTVA
WKADSSPVKAGVETTTPSKQSNNKYAASSYLSLTPEQWKSHRSYSCQ
VTHEGSTVEKTVAPTECS
**Amino acid sequence of BsAb-6 Chain 1: SEQ ID NO: 9** 
EVQLVESGGGLVQPGGSLRLSCAASYFISRFYAVGWYRQAPGKQRELVAR
ISSGGWTYYADSVKGRFTISIDNSKNTLYLQMNSLRAEDTAVYYCNARNL
WGQGTLVTVSSGGGGSGGGGSGGGGSEVQLVESGGGLVQPGGSLRLSCA
ASYFISRFYAVGWYRQAPGKQRELVARISSGGWTYYADSVKGRFTISIDNS
KNTLYLQMNSLRAEDTAVYYCNARNLWGQGTLVTVSSDKTHTCPPCPAP
ESTRGPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPAPI
EKTISKAKGQPREPQVCTLPPSRDELTKNQVSLSCAVKGFYPSDIAVEWES
NGQPENNYKTTPPVLDSDGSFFLVSKLTVDKSRWQQGNVFSCSVMHEALH
NHYTQKSLSLSPGK
**Amino acid sequence of BsAb-6 Chain 2: SEQ ID NO: 2** 
EVQLVESGGGLVQPGRSLRLSCAASGFTFNTYAMNWVRQAPGKGLEWVA
RIRSKYNNYATYYADSVKDRFTISRDDSKSIAYLQMNSLKTEDTAVYYCV
RHGNFGNSYVSWFAYWGQGTLVTVSSASTKGPSVFPLAPSSKSTSGGTAA
LGCLVKDYFPEPVTVSWNSGALTSGVHTFPAVLQSSGLYSLSSWTVPSSSL
GTQTYICNVNHKPSNTKVDKKVEPKSCDKTHTCPPCPAPESTRGPSVFLFPP
KPKDTLMISRTPEVTCVVVDVSHEDPEVKFNWYVDGVEVHNAKTKPREE
QYNSTYRWSVLTVLHQDWLNGKEYKCKVSNKALPAPIEKTISKAKGQPRE
PQVYTLPPCRDELTKNQVSLWCLVKGFYPSDIAVEWESNGQPENNYKTTP
 
PVLDSDGSFFLYSKLTVDKSRWQQGNVFSCSVMHEALHNHYTQKSLSLSP
GK
**Amino acid sequence of BsAb-6 Chain 3: SEQ ID NO: 3** 
QTVVTQEPSLTVSPGGTVTLTCRSSTGAVTTSNYANWVQQKPGQAPRGLI
GGTNKRAPGVPARFSGSLLGGKAALTLSGVQPEDEAEYFCALWYSNLWV
FGGGTKLTVLGQPKAAPSVTLFPPSSEELQANKATLVCLISDFYPGAVTVA
WKADSSPVKAGVETTTPSKQSNNKYAASSYLSLTPEQWKSHRSYSCQ
VTHEGSTVEKTVAPTECS
**Amino acid sequence of BsAb-7 Chain 1: SEQ ID NO: 10** 
QVQLVESGGGVVQPGGSLRLSCAASGFVLRVNTVSWYRQAPGKQRELVA
LITSESNEYYDDSVKGRFTISRDNAKNTIYLHMNSLKPEDTGVYFCNFITTW
GQGTQVTVSSGGGGSGGGGSGGGGSQVQLVESGGGVVQPGGSLRLSCAA
SGFVLRVNTVSWYRQAPGKQRELVALITSESNEYYDDSVKGRFTISRDNA
KNTIYLHMNSLKPEDTGVYFCNFITTWGQGTQVTVSSDKTHTCPPCPAPES
TRGPSVFLFPPKPKDTLMISRTPEVTCVVVDVSHEDPEVKFNWYVDGVEV
HNAKTKPREEQYNSTYRVVSVLTVLHQDWLNGKEYKCKVSNKALPAPIE
KTISKAKGQPREPQVCTLPPSRDELTKNQVSLSCAVKGFYPSDIAVEWESN
GQPENNYKTTPPVLDSDGSFFLVSKLTVDKSRWQQGNVFSCSVMHEALHN
HYTQKSLSLSPGK
**Amino acid sequence of BsAb-7 Chain 2: SEQ ID NO: 2** 
EVQLVESGGGLVQPGRSLRLSCAASGFTFNTYAMNWVRQAPGKGLEWVA
RIRSKYNNYATYYADSVKDRFTISRDDSKSIAYLQMNSLKTEDTAVYYCV
RHGNFGNSYVSWFAYWGQGTLVTVSSASTKGPSVFPLAPSSKSTSGGTAA
LGCLVKDYFPEPVTVSWNSGALTSGVHTFPAVLQSSGLYSLSSWTVPSSSL
GTQTYICNVNHKPSNTKVDKKVEPKSCDKTHTCPPCPAPESTRGPSVFLFPP
KPKDTLMISRTPEVTCVVVDVSHEDPEVKFNWYVDGVEVHNAKTKPREE
QYNSTYRWSVLTVLHQDWLNGKEYKCKVSNKALPAPIEKTISKAKGQPRE
PQVYTLPPCRDELTKNQVSLWCLVKGFYPSDIAVEWESNGQPENNYKTTP
PVLDSDGSFFLYSKLTVDKSRWQQGNVFSCSVMHEALHNHYTQKSLSLSP
GK
**Amino acid sequence of BsAb-7 Chain 3: SEQ ID NO: 3** 
QTVVTQEPSLTVSPGGTVTLTCRSSTGAVTTSNYANWVQQKPGQAPRGLI
GGTNKRAPGVPARFSGSLLGGKAALTLSGVQPEDEAEYFCALWYSNLWV
FGGGTKLTVLGQPKAAPSVTLFPPSSEELQANKATLVCLISDFYPGAVTVA
 
WKADSSPVKAGVETTTPSKQSNNKYAASSYLSLTPEQWKSHRSYSCQVTH
EGSTVEKTVAPTECS
**Amino acid sequence of BsAb-8 Chain 1: SEQ ID NO: 11** 
EVQLVESGGGLVQPGGSLRLSCAASGRSFSSFQMGWFRQAPGKEREFVAA
SGWSGGPTYYWDSVIGRFTISTDNSKNTLYLQMNSLRAEDTAVYYCYEDR
VLSGRPVRYWGQGTLVTVSSGGGGSGGGGSGGGGSEVQLVESGGGLVQP
GGSLRLSCAASGRSFSSFQMGWFRQAPGKEREFVAASGWSGGPTYYWDS
VIGRFTISTDNSKNTLYLQMNSLRAEDTAVYYCYEDRVLSGRPVRYWGQG
TLVTVSSDKTHTCPPCPAPESTRGPSVFLFPPKPKDTLMISRTPEVTCVWDV
SHEDPEVKFNWYVDGVEVHNAKTKPREEQYNSTYRVVSVLTVLHQDWL
NGKEYKCKVSNKALPAPIEKTISKAKGQPREPQVCTLPPSRDELTKNQVSL
SCAVKGFYPSDIAVEWESNGQPENNYKTTPPVLDSDGSFFLVSKLTVDKSR
WQQGNVFSCSVMHEALHNHYTQKSLSLSPGK
**Amino acid sequence of BsAb-8 Chain 2: SEQ ID NO: 2** 
EVQLVESGGGLVQPGRSLRLSCAASGFTFNTYAMNWVRQAPGKGLEWVA
RIRSKYNNYATYYADSVKDRFTISRDDSKSIAYLQMNSLKTEDTAVYYCV
RHGNFGNSYVSWFAYWGQGTLVTVSSASTKGPSVFPLAPSSKSTSGGTAA
LGCLVKDYFPEPVTVSWNSGALTSGVHTFPAVLQSSGLYSLSSWTVPSSSL
GTQTYICNVNHKPSNTKVDKKVEPKSCDKTHTCPPCPAPESTRGPSVFLFPP
KPKDTLMISRTPEVTCVVVDVSHEDPEVKFNWYVDGVEVHNAKTKPREE
QYNSTYRWSVLTVLHQDWLNGKEYKCKVSNKALPAPIEKTISKAKGQPRE
PQVYTLPPCRDELTKNQVSLWCLVKGFYPSDIAVEWESNGQPENNYKTTP
PVLDSDGSFFLYSKLTVDKSRWQQGNVFSCSVMHEALHNHYTQKSLSLSP
GK
**Amino acid sequence of BsAb-8 Chain 3: SEQ ID NO: 3** 
QTVVTQEPSLTVSPGGTVTLTCRSSTGAVTTSNYANWVQQKPGQAPRGLI
GGTNKRAPGVPARFSGSLLGGKAALTLSGVQPEDEAEYFCALWYSNLWV
FGGGTKLTVLGQPKAAPSVTLFPPSSEELQANKATLVCLISDFYPGAVTVA
WKADSSPVKAGVETTTPSKQSNNKYAASSYLSLTPEQWKSHRSYSCQVTH
EGSTVEKTVAPTECS
**Amino acid sequence of BsAb-16 Chain 1: SEQ ID NO: 12** 
EVQLVESGGGLVQPGGSLRLSCAASGRSFSSFQMGWFRQAPGKEREFVAA
SGWSGGPTYYWDSVIGRFTISTDNSKNTLYLQMNSLRAEDTAVYYCYEDR
VLSGRHVRYWGQGTLVTVSSGGGGSGGGGSGGGGSEVQLVESGGGLVQP
 
GGSLRLSCAASGRSFSSFQMGWFRQAPGKEREFVAASGWSGGPTYYWDS
VIGRFTISTDNSKNTLYLQMNSLRAEDTAVYYCYEDRVLSGRHVRYWGQ
GTLVTVSSDKTHTCPPCPAPESTRGPSVFLFPPKPKDTLMISRTPEVTCVWD
VSHEDPEVKFNWYVDGVEVHNAKTKPREEQYNSTYRVVSVLTVLHQDW
LNGKEYKCKVSNKALPAPIEKTISKAKGQPREPQVCTLPPSRDELTKNQVS
LSCAVKGFYPSDIAVEWESNGQPENNYKTTPPVLDSDGSFFLVSKLTVDKS
RWQQGNVFSCSVMHEALHNHYTQKSLSLSPGK
**Amino acid sequence of BsAb-16 Chain 2: SEQ ID NO: 2** 
EVQLVESGGGLVQPGRSLRLSCAASGFTFNTYAMNWVRQAPGKGLEWVA
RIRSKYNNYATYYADSVKDRFTISRDDSKSIAYLQMNSLKTEDTAVYYCV
RHGNFGNSYVSWFAYWGQGTLVTVSSASTKGPSVFPLAPSSKSTSGGTAA
LGCLVKDYFPEPVTVSWNSGALTSGVHTFPAVLQSSGLYSLSSWTVPSSSL
GTQTYICNVNHKPSNTKVDKKVEPKSCDKTHTCPPCPAPESTRGPSVFLFPP
KPKDTLMISRTPEVTCVVVDVSHEDPEVKFNWYVDGVEVHNAKTKPREE
QYNSTYRWSVLTVLHQDWLNGKEYKCKVSNKALPAPIEKTISKAKGQPRE
PQVYTLPPCRDELTKNQVSLWCLVKGFYPSDIAVEWESNGQPENNYKTTP
PVLDSDGSFFLYSKLTVDKSRWQQGNVFSCSVMHEALHNHYTQKSLSLSP
GK
**Amino acid sequence of BsAb-16 Chain 3: SEQ ID NO: 3** 
QTVVTQEPSLTVSPGGTVTLTCRSSTGAVTTSNYANWVQQKPGQAPRGLI
GGTNKRAPGVPARFSGSLLGGKAALTLSGVQPEDEAEYFCALWYSNLWV
FGGGTKLTVLGQPKAAPSVTLFPPSSEELQANKATLVCLISDFYPGAVTVA
WKADSSPVKAGVETTTPSKQSNNKYAASSYLSLTPEQWKSHRSYSCQVTH
EGSTVEKTVAPTECS
**Amino acid sequence of BsAb-9 Chain 1: SEQ ID NO: 1** 
EVQLVESGGGLVQPGGSLRLSCAASGFISRFYAMGWYRQAPGKQRELVAR
ISSMGWTYYADSVKGRFTISIDNSKNTLYLQMNSLRAEDTAVYYCNARNL
WGQGTLVTVSSDKTHTCPPCPAPESTRGPSVFLFPPKPKDTLMISRTPEVTC
VVVDVSHEDPEVKFNWYVDGVEVHNAKTKPREEQYNSTYRVVSVLTVLH
QDWLNGKEYKCKVSNKALPAPIEKTISKAKGQPREPQVCTLPPSRDELTKN
QVSLSCAVKGFYPSDIAVEWESNGQPENNYKTTPPVLDSDGSFFLVSKLTV
DKSRWQQGNVFSCSVMHEALHNHYTQKSLSLSPGK
**Amino acid sequence of BsAb-9 Chain 2: SEQ ID NO: 13** 
EVQLVESGGGLVQPGGSLRLSCAASGFISRFYAMGWYRQAPGKQRELVAR
ISSMGWTYYADSVKGRFTISIDNSKNTLYLQMNSLRAEDTAVYYCNARNL
WGQGTLVTVSSGGGGSGGGGSGGGGSEVQLVESGGGLVQPGRSLRLSCA
ASGFTFNTYAMNWVRQAPGKGLEWVARIRSKYNNYATYYADSVKDRFTI
SRDDSKSIAYLQMNSLKTEDTAVYYCVRHGNFGNSYVSWFAYWGQGTLV
TVSSASTKGPSVFPLAPSSKSTSGGTAALGCLVKDYFPEPVTVSWNSGALT
SGVHTFPAVLQSSGLYSLSSVVTVPSSSLGTQTYICNVNHKPSNTKVDKKV
EPKSCDKTHTCPPCPAPESTRGPSVFLFPPKPKDTLMISRTPEVTCVVVDVS
HEDPEVKFNWYVDGVEVHNAKTKPREEQYNSTYRVVSVLTVLHQDWLN
GKEYKCKVSNKALPAPIEKTISKAKGQPREPQVYTLPPCRDELTKNQVSLW
CLVKGFYPSDIAVEWESNGQPENNYKTTPPVLDSDGSFFLYSKLTVDKSR
WQQGNVFSCSVMHEALHNHYTQKSLSLSPGK
**Amino acid sequence of BsAb-9 Chain 3: SEQ ID NO: 3** 
QTVVTQEPSLTVSPGGTVTLTCRSSTGAVTTSNYANWVQQKPGQAPRGLI
GGTNKRAPGVPARFSGSLLGGKAALTLSGVQPEDEAEYFCALWYSNLWV
FGGGTKLTVLGQPKAAPSVTLFPPSSEELQANKATLVCLISDFYPGAVTVA
WKADSSPVKAGVETTTPSKQSNNKYAASSYLSLTPEQWKSHRSYSCQVTH
EGSTVEKTVAPTECS
**Amino acid sequence of BsAb-10 Chain 1: SEQ ID NO: 4** 
EVQLVESGGGLVQPGGSLRLSCAASYFISRFYAVGWYRQAPGKQRELVAR
ISSGGWTYYADSVKGRFTISIDNSKNTLYLQMNSLRAEDTAVYYCNARNL
WGQGTLVTVSSDKTHTCPPCPAPESTRGPSVFLFPPKPKDTLMISRTPEVTC
VVVDVSHEDPEVKFNWYVDGVEVHNAKTKPREEQYNSTYRVVSVLTVLH
QDWLNGKEYKCKVSNKALPAPIEKTISKAKGQPREPQVCTLPPSRDELTKN
QVSLSCAVKGFYPSDIAVEWESNGQPENNYKTTPPVLDSDGSFFLVSKLTV
DKSRWQQGNVFSCSVMHEALHNHYTQKSLSLSPGK
**Amino acid sequence of BsAb-10 Chain 2: SEQ ID NO: 14** 
EVQLVESGGGLVQPGGSLRLSCAASYFISRFYAVGWYRQAPGKQRELVAR
ISSGGWTYYADSVKGRFTISIDNSKNTLYLQMNSLRAEDTAVYYCNARNL
WGQGTLVTVSSGGGGSGGGGSGGGGSEVQLVESGGGLVQPGRSLRLSCA
ASGFTFNTYAMNWVRQAPGKGLEWVARIRSKYNNYATYYADSVKDRFTI
SRDDSKSIAYLQMNSLKTEDTAVYYCVRHGNFGNSYVSWFAYWGQGTLV
TVSSASTKGPSVFPLAPSSKSTSGGTAALGCLVKDYFPEPVTVSWNSGALT
SGVHTFPAVLQSSGLYSLSSVVTVPSSSLGTQTYICNVNHKPSNTKVDKKV
 
EPKSCDKTHTCPPCPAPESTRGPSVFLFPPKPKDTLMISRTPEVTCVVVDVS
HEDPEVKFNWYVDGVEVHNAKTKPREEQYNSTYRVVSVLTVLHQDWLN
GKEYKCKVSNKALPAPIEKTISKAKGQPREPQVYTLPPCRDELTKNQVSLW
CLVKGFYPSDIAVEWESNGQPENNYKTTPPVLDSDGSFFLYSKLTVDKSR
WQQGNVFSCSVMHEALHNHYTQKSLSLSPGK
**Amino acid sequence of BsAb-10 Chain 3: SEQ ID NO: 3** 
QTVVTQEPSLTVSPGGTVTLTCRSSTGAVTTSNYANWVQQKPGQAPRGLI
GGTNKRAPGVPARFSGSLLGGKAALTLSGVQPEDEAEYFCALWYSNLWV
FGGGTKLTVLGQPKAAPSVTLFPPSSEELQANKATLVCLISDFYPGAVTVA
WKADSSPVKAGVETTTPSKQSNNKYAASSYLSLTPEQWKSHRSYSCQVTH
EGSTVEKTVAPTECS
**Amino acid sequence of BsAb-11 Chain 1: SEQ ID NO: 5** 
QVQLVESGGGVVQPGGSLRLSCAASGFVLRVNTVSWYRQAPGKQRELVA
LITSESNEYYDDSVKGRFTISRDNAKNTIYLHMNSLKPEDTGVYFCNFITTW
GQGTQVTVSSDKTHTCPPCPAPESTRGPSVFLFPPKPKDTLMISRTPEVTCV
VVDVSHEDPEVKFNWYVDGVEVHNAKTKPREEQYNSTYRVVSVLTVLHQ
DWLNGKEYKCKVSNKALPAPIEKTISKAKGQPREPQVCTLPPSRDELTKNQ
VSLSCAVKGFYPSDIAVEWESNGQPENNYKTTPPVLDSDGSFFLVSKLTVD
KSRWQQGNVFSCSVMHEALHNHYTQKSLSLSPGK
**Amino acid sequence of BsAb-11 Chain 2: SEQ ID NO: 15** 
QVQLVESGGGVVQPGGSLRLSCAASGFVLRVNTVSWYRQAPGKQRELVA
LITSESNEYYDDSVKGRFTISRDNAKNTIYLHMNSLKPEDTGVYFCNFITTW
GQGTQVTVSSGGGGSGGGGSGGGGSEVQLVESGGGLVQPGRSLRLSCAAS
GFTFNTYAMNWVRQAPGKGLEWVARIRSKYNNYATYYADSVKDRFTISR
DDSKSIAYLQMNSLKTEDTAVYYCVRHGNFGNSYVSWFAYWGQGTLVT
VSSASTKGPSVFPLAPSSKSTSGGTAALGCLVKDYFPEPVTVSWNSGALTS
GVHTFPAVLQSSGLYSLSSVVTVPSSSLGTQTYICNVNHKPSNTKVDKKVE
PKSCDKTHTCPPCPAPESTRGPSVFLFPPKPKDTLMISRTPEVTCVVVDVSH
EDPEVKFNWYVDGVEVHNAKTKPREEQYNSTYRVVSVLTVLHQDWLNG
KEYKCKVSNKALPAPIEKTISKAKGQPREPQVYTLPPCRDELTKNQVSLWC
LVKGFYPSDIAVEWESNGQPENNYKTTPPVLDSDGSFFLYSKLTVDKSRW
QQGNVFSCSVMHEALHNHYTQKSLSLSPGK
**Amino acid sequence of BsAb-11 Chain 3: SEQ ID NO: 3** 
QTVVTQEPSLTVSPGGTVTLTCRSSTGAVTTSNYANWVQQKPGQAPRGLI
GGTNKRAPGVPARFSGSLLGGKAALTLSGVQPEDEAEYFCALWYSNLWV
FGGGTKLTVLGQPKAAPSVTLFPPSSEELQANKATLVCLISDFYPGAVTVA
WKADSSPVKAGVETTTPSKQSNNKYAASSYLSLTPEQWKSHRSYSCQVTH
EGSTVEKTVAPTECS
**Amino acid sequence of BsAb-12 Chain 1: SEQ ID NO: 6** 
EVQLVESGGGLVQPGGSLRLSCAASGRSFSSFQMGWFRQAPGKEREFVAA
SGWSGGPTYYWDSVIGRFTISTDNSKNTLYLQMNSLRAEDTAVYYCYEDR
VLSGRPVRYWGQGTLVTVSSDKTHTCPPCPAPESTRGPSVFLFPPKPKDTL
MISRTPEVTCVVVDVSHEDPEVKFNWYVDGVEVHNAKTKPREEQYNSTY
RVVSVLTVLHQDWLNGKEYKCKVSNKALPAPIEKTISKAKGQPREPQVCT
LPPSRDELTKNQVSLSCAVKGFYPSDIAVEWESNGQPENNYKTTPPVLDSD
GSFFLVSKLTVDKSRWQQGNVFSCSVMHEALHNHYTQKSLSLSPGK
**Amino acid sequence of BsAb-12 Chain 2: SEQ ID NO: 16** 
EVQLVESGGGLVQPGGSLRLSCAASGRSFSSFQMGWFRQAPGKEREFVAA
SGWSGGPTYYWDSVIGRFTISTDNSKNTLYLQMNSLRAEDTAVYYCYEDR
VLSGRPVRYWGQGTLVTVSSGGGGSGGGGSGGGGSEVQLVESGGGLVQP
GRSLRLSCAASGFTFNTYAMNWVRQAPGKGLEWVARIRSKYNNYATYYA
DSVKDRFTISRDDSKSIAYLQMNSLKTEDTAVYYCVRHGNFGNSYVSWFA
YWGQGTLVTVSSASTKGPSVFPLAPSSKSTSGGTAALGCLVKDYFPEPVTV
SWNSGALTSGVHTFPAVLQSSGLYSLSSVVTVPSSSLGTQTYICNVNHKPS
NTKVDKKVEPKSCDKTHTCPPCPAPESTRGPSVFLFPPKPKDTLMISRTPEV
TCVVVDVSHEDPEVKFNWYVDGVEVHNAKTKPREEQYNSTYRVVSVLTV
LHQDWLNGKEYKCKVSNKALPAPIEKTISKAKGQPREPQVYTLPPCRDELT
KNQVSLWCLVKGFYPSDIAVEWESNGQPENNYKTTPPVLDSDGSFFLYSK
LTVDKSRWQQGNVFSCSVMHEALHNHYTQKSLSLSPGK
**Amino acid sequence of BsAb-12 Chain 3: SEQ ID NO: 3** 
QTVVTQEPSLTVSPGGTVTLTCRSSTGAVTTSNYANWVQQKPGQAPRGLI
GGTNKRAPGVPARFSGSLLGGKAALTLSGVQPEDEAEYFCALWYSNLWV
FGGGTKLTVLGQPKAAPSVTLFPPSSEELQANKATLVCLISDFYPGAVTVA
WKADSSPVKAGVETTTPSKQSNNKYAASSYLSLTPEQWKSHRSYSCQVTH
EGSTVEKTVAPTECS
**Amino acid sequence of BsAb-17 Chain 1: SEQ ID NO: 7** 
EVQLVESGGGLVQPGGSLRLSCAASGRSFSSFQMGWFRQAPGKEREFVAA
SGWSGGPTYYWDSVIGRFTISTDNSKNTLYLQMNSLRAEDTAVYYCYEDR
VLSGRHVRYWGQGTLVTVSSDKTHTCPPCPAPESTRGPSVFLFPPKPKDTL
MISRTPEVTCVVVDVSHEDPEVKFNWYVDGVEVHNAKTKPREEQYNSTY
RVVSVLTVLHQDWLNGKEYKCKVSNKALPAPIEKTISKAKGQPREPQVCT
LPPSRDELTKNQVSLSCAVKGFYPSDIAVEWESNGQPENNYKTTPPVLDSD
GSFFLVSKLTVDKSRWQQGNVFSCSVMHEALHNHYTQKSLSLSPGK
**Amino acid sequence of BsAb-17 Chain 2: SEQ ID NO: 17** 
EVQLVESGGGLVQPGGSLRLSCAASGRSFSSFQMGWFRQAPGKEREFVAA
SGWSGGPTYYWDSVIGRFTISTDNSKNTLYLQMNSLRAEDTAVYYCYEDR
VLSGRHVRYWGQGTLVTVSSGGGGSGGGGSGGGGSEVQLVESGGGLVQP
GRSLRLSCAASGFTFNTYAMNWVRQAPGKGLEWVARIRSKYNNYATYYA
DSVKDRFTISRDDSKSIAYLQMNSLKTEDTAVYYCVRHGNFGNSYVSWFA
YWGQGTLVTVSSASTKGPSVFPLAPSSKSTSGGTAALGCLVKDYFPEPVTV
SWNSGALTSGVHTFPAVLQSSGLYSLSSVVTVPSSSLGTQTYICNVNHKPS
NTKVDKKVEPKSCDKTHTCPPCPAPESTRGPSVFLFPPKPKDTLMISRTPEV
TCVVVDVSHEDPEVKFNWYVDGVEVHNAKTKPREEQYNSTYRVVSVLTV
LHQDWLNGKEYKCKVSNKALPAPIEKTISKAKGQPREPQVYTLPPCRDELT
KNQVSLWCLVKGFYPSDIAVEWESNGQPENNYKTTPPVLDSDGSFFLYSK
LTVDKSRWQQGNVFSCSVMHEALHNHYTQKSLSLSPGK
**Amino acid sequence of BsAb-17 Chain 3: SEQ ID NO: 3** 
QTVVTQEPSLTVSPGGTVTLTCRSSTGAVTTSNYANWVQQKPGQAPRGLI
GGTNKRAPGVPARFSGSLLGGKAALTLSGVQPEDEAEYFCALWYSNLWV
FGGGTKLTVLGQPKAAPSVTLFPPSSEELQANKATLVCLISDFYPGAVTVA
WKADSSPVKAGVETTTPSKQSNNKYAASSYLSLTPEQWKSHRSYSCQVTH
EGSTVEKTVAPTECS
**Amino acid sequence of heavy chain variable region of AHP15662-H4-M21721 single domain antibody: SEQ ID NO: 18** 
EVQLVESGGGLVQPGGSLRLSCAASGFISRFYAMGWYRQAPGKQRELVAR
ISSMGWTYYADSVKGRFTISIDNSKNTLYLQMNSLRAEDTAVYYCNARNL
WGQGTLVTVSS
**Amino acid sequence of HCDR1 region of AHP15662-H4-M21721 single domain antibody: SEQ ID NO: 19**
   **GFISRFYA**
**Amino acid sequence of HCDR2 region of AHP15662-H4-M21721 single domain antibody: SEQ ID NO: 20**
   ISSMGWT
**Amino acid sequence of HCDR3 region of AHP15662-H4-M21721 single domain antibody: SEQ ID NO: 21**
   NARNL
**Amino acid sequence of heavy chain variable region of AHP15662-H4-MV2 single domain antibody: SEQ ID NO: 22** 
EVQLVESGGGLVQPGGSLRLSCAASYFISRFYAVGWYRQAPGKQRELVAR
ISSGGWTYYADSVKGRFTISIDNSKNTLYLQMNSLRAEDTAVYYCNARNL
WGQGTLVTVSS
**Amino acid sequence of HCDR1 region of AHP15662-H4-MV2 single domain antibody: SEQ ID NO: 23**
   YFISRFYA
**Amino acid sequence of HCDR2 region of AHP15662-H4-MV2 single domain antibody: SEQ ID NO: 24**
   ISSGGWT
**Amino acid sequence of HCDR3 region of AHP15662-H4-MV2 single domain antibody: SEQ ID NO: 25**
   NARNL
**Amino acid sequence of heavy chain variable region of AHP15580 single domain antibody: SEQ ID NO: 26** 
QVQLVESGGGVVQPGGSLRLSCAASGFVLRVNTVSWYRQAPGKQRELVA
LITSESNEYYDDSVKGRFTISRDNAKNTIYLHMNSLKPEDTGVYFCNFITTW
GQGTQVTVSS
**Amino acid sequence of HCDR1 region of AHP15580 single domain antibody: SEQ ID NO: 27**
   GFVLRVNT
**Amino acid sequence of HCDR2 region of AHP15580 single domain antibody: SEQ ID NO: 28**
   ITSESNE
**Amino acid sequence of HCDR3 region of AHP15580 single domain antibody: SEQ ID NO: 29**
   NFITT
**Amino acid sequence of heavy chain variable region of AHP15485-H4-MV2 single domain antibody: SEQ ID NO: 30** 
EVQLVESGGGLVQPGGSLRLSCAASGRSFSSFQMGWFRQAPGKEREFVAA
SGWSGGPTYYWDSVIGRFTISTDNSKNTLYLQMNSLRAEDTAVYYCYEDR
VLSGRPVRYWGQGTLVTVSS
**Amino acid sequence of HCDR1 region of AHP15485-H4-MV2 single domain antibody: SEQ ID NO: 31**
   GRSFSSFQ
**Amino acid sequence of HCDR2 region of AHP15485-H4-MV2 single domain antibody: SEQ ID NO: 32**
   SGWSGGPT
**Amino acid sequence of HCDR3 region of AHP15485-H4-MV2 single domain antibody: SEQ ID NO: 33**
   YEDRVLSGRPVRY
**Amino acid sequence of heavy chain variable region of AHP15485-H4-M22013 single domain antibody: SEQ ID NO: 34** 
EVQLVESGGGLVQPGGSLRLSCAASGRSFSSFQMGWFRQAPGKEREFVAA
SGWSGGPTYYWDSVIGRFTISTDNSKNTLYLQMNSLRAEDTAVYYCYEDR
VLSGRHVRYWGQGTLVTVSS
**Amino acid sequence of HCDR1 region of AHP15485-H4-M22013 single domain antibody: SEQ ID NO: 35**
   GRSFSSFQ
**Amino acid sequence of HCDR2 region of AHP15485-H4-M22013 single domain antibody: SEQ ID NO: 36**
   SGWSGGPT
**Amino acid sequence of HCDR3 region of AHP15485-H4-M22013 single domain antibody: SEQ ID NO: 37**
   YEDRVLSGRHVRY
**Amino acid sequence of heavy chain variable region of AED-2018 humanized antibody: SEQ ID NO: 38** 
EVQLVESGGGLVQPGRSLRLSCAASGFTFNTYAMNWVRQAPGKGLEWVA
RIRSKYNNYATYYADSVKDRFTISRDDSKSIAYLQMNSLKTEDTAVYYCV
RHGNFGNSYVSWFAYWGQGTLVTVSS
**Amino acid sequence of heavy chain CDR1 region of AED-2018 humanized antibody: SEQ ID NO: 39**
   GFTFNTYAMN
**Amino acid sequence of heavy chain CDR2 region of AED-2018 humanized antibody: SEQ ID NO: 40**
   RIRSKYNNYATYYADSVKD
**Amino acid sequence of heavy chain CDR3 region of AED-2018 humanized antibody: SEQ ID NO: 41**
   HGNFGNSYVSWFAY
**Amino acid sequence of light chain variable region of AED-2018 humanized antibody: SEQ ID NO: 42** 
QTVVTQEPSLTVSPGGTVTLTCRSSTGAVTTSNYANWVQQKPGQAPRGLI
GGTNKRAPGVPARFSGSLLGGKAALTLSGVQPEDEAEYFCALWYSNLWV
FGGGTKLTVL
**Amino acid sequence of light chain CDR1 region of AED-2018 humanized antibody: SEQ ID NO: 43**
   RSSTGAVTTSNYAN
**Amino acid sequence of light chain CDR2 region of AED-2018 humanized antibody: SEQ ID NO: 44**
   GTNKRAP
**Amino acid sequence of light chain CDR3 region of AED-2018 humanized antibody: SEQ ID NO: 45**
   ALWYSNLWV
**Amino acid sequence of HuIgG1Fc (STR hole): SEQ ID NO: 46** 
DKTHTCPPCPAPESTRGPSVFLFPPKPKDTLMISRTPEVTCVVVDVSHEDPE
VKFNWYVDGVEVHNAKTKPREEQYNSTYRVVSVLTVLHQDWLNGKEYK
CKVSNKALPAPIEKTISKAKGQPREPQVCTLPPSRDELTKNQVSLSCAVKGF
YPSDIAVEWESNGQPENNYKTTPPVLDSDGSFFLVSKLTVDKSRWQQGNV
FSCSVMHEALHNHYTQKSLSLSPGK
**Amino acid sequence of HuIgGCL: SEQ ID NO: 47** 
GQPKAAPSVTLFPPSSEELQANKATLVCLISDFYPGAVTVAWKADSSPVKA
GVETTTPSKQSNNKYAASSYLSLTPEQWKSHRSYSCQVTHEGSTVEKTVA
PTECS
**Amino acid sequence of HuIgG1CH (STR knob): SEQ ID NO: 48** 
ASTKGPSVFPLAPSSKSTSGGTAALGCLVKDYFPEPVTVSWNSGALTSGVH
TFPAVLQSSGLYSLSSVVTVPSSSLGTQTYICNVNHKPSNTKVDKKVEPKS
CDKTHTCPPCPAPESTRGPSVFLFPPKPKDTLMISRTPEVTCVVVDVSHEDP
EVKFNWYVDGVEVHNAKTKPREEQYNSTYRVVSVLTVLHQDWLNGKEY
KCKVSNKALPAPIEKTISKAKGQPREPQVYTLPPCRDELTKNQVSLWCLVK
GFYPSDIAVEWESNGQPENNYKTTPPVLDSDGSFFLYSKLTVDKSRWQQG
NVFSCSVMHEALHNHYTQKSLSLSPGK
**Amino acid sequence of (GGGGS)₃ linker: SEQ ID NO: 49**
   GGGGSGGGGSGGGGS
**Amino acid sequence of (GGGGS)n linker: SEQ ID NO: 50**
   (GGGGS)n

## Claims

1. A bispecific antibody targeting ROR1 and CD3, **characterized in that** the bispecific antibody targeting ROR1 and CD3 comprises a first antigen-binding portion and a second antigen-binding portion, wherein the first antigen-binding portion is an antibody that specifically binds to ROR1 or an antigen-binding fragment thereof, and the second antigen-binding portion is an antibody that specifically binds to CD3 or an antigen-binding fragment thereof, wherein the second antigen-binding portion comprises a heavy chain variable region VH and a light chain variable region VL, the VH comprises HCDR1 as set forth in SEQ ID NO: 39, HCDR2 as set forth in SEQ ID NO: 40, and HCDR3 as set forth in SEQ ID NO: 41, and the VL comprises LCDR1 as set forth in SEQ ID NO: 43, LCDR2 as set forth in SEQ ID NO: 44, and LCDR3 as set forth in SEQ ID NO: 45.

2. The bispecific antibody according to claim 1, **characterized in that** the first antigen-binding portion comprises a heavy chain variable region VH, the VH comprises HCDR1, HCDR2 and HCDR3, and the HCDR1, HCDR2 and HCDR3 are selected from the following groups:
(1) HCDR1 as set forth in SEQ ID NO: 19, HCDR2 as set forth in SEQ ID NO: 20, and HCDR3 as set forth in SEQ ID NO: 21;
(2) HCDR1 as set forth in SEQ ID NO: 23, HCDR2 as set forth in SEQ ID NO: 24, and HCDR3 as set forth in SEQ ID NO: 25;
(3) HCDR1 as set forth in SEQ ID NO: 27, HCDR2 as set forth in SEQ ID NO: 28, and HCDR3 as set forth in SEQ ID NO: 29;
(4) HCDR1 as set forth in SEQ ID NO: 31, HCDR2 as set forth in SEQ ID NO: 32, and HCDR3 as set forth in SEQ ID NO: 33; and
(5) HCDR1 as set forth in SEQ ID NO: 35, HCDR2 as set forth in SEQ ID NO: 36 and HCDR3 as set forth in SEQ ID NO: 37.

3. The bispecific antibody according to claim 2, **characterized in that** the VH of the first antigen-binding portion comprises an amino acid sequence as set forth in SEQ ID NO: 18, 22, 26, 30, or 34, or a sequence having at least 80% sequence identity to the amino acid sequence as set forth in SEQ ID NO: 18, 22, 26, 30, or 34.

4. The bispecific antibody according to any one of claims 1-3, **characterized in that** the first antigen-binding portion is a single domain antibody.

5. The bispecific antibody according to any one of claims 1-4, **characterized in that** the VH of the second antigen-binding portion comprises an amino acid sequence as set forth in SEQ ID NO: 38 or a sequence having at least 80% sequence identity to the amino acid sequence as set forth in SEQ ID NO: 38, and the VL of the second antigen-binding portion comprises an amino acid sequence as set forth in SEQ ID NO: 42 or a sequence having at least 80% sequence identity to the amino acid sequence as set forth in SEQ ID NO: 42.

6. The bispecific antibody according to any one of claims 1-5, **characterized in that** the second antigen-binding portion is an Fab fragment.

7. The bispecific antibody according to any one of claims 1-6, **characterized in that** the bispecific antibody further comprises an Fc fragment.

8. The bispecific antibody according to claim 7, **characterized in that** the Fc fragment has a Knob-Hole structure.

9. The bispecific antibody according to claim 7 or 8, **characterized in that** the Fc fragment has a mutation that reduces or eliminates the binding of the antibody to Fcy receptor, preferably a L234S/L235T/G236R triple mutation.

10. The bispecific antibody according to any one of claims 1-9, **characterized in that** the first antigen-binding portion is fused to the N-terminus of a first Fc fragment, and the second antigen-binding portion is fused to the N-terminus of a second Fc fragment.

11. The bispecific antibody according to claim 10, **characterized in that** the second antigen-binding portion is an Fab fragment, and the heavy chain of the Fab fragment is fused to the N-terminus of the second Fc fragment.

12. The bispecific antibody according to any one of claims 1-11, **characterized in that** the bispecific antibody further comprises a third antigen-binding portion, wherein the third antigen-binding portion is an antibody that specifically binds to ROR1 or an antigen-binding fragment thereof.

13. The bispecific antibody according to claim 12, **characterized in that** the third antigen-binding portion comprises a heavy chain variable region VH, the VH comprises HCDR1, HCDR2 and HCDR3, and the HCDR1, HCDR2 and HCDR3 are selected from the following groups:
(1) HCDR1 as set forth in SEQ ID NO: 19, HCDR2 as set forth in SEQ ID NO: 20, and HCDR3 as set forth in SEQ ID NO: 21;
(2) HCDR1 as set forth in SEQ ID NO: 23, HCDR2 as set forth in SEQ ID NO: 24, and HCDR3 as set forth in SEQ ID NO: 25;
(3) HCDR1 as set forth in SEQ ID NO: 27, HCDR2 as set forth in SEQ ID NO: 28, and HCDR3 as set forth in SEQ ID NO: 29;
(4) HCDR1 as set forth in SEQ ID NO: 31, HCDR2 as set forth in SEQ ID NO: 32, and HCDR3 as set forth in SEQ ID NO: 33; and
(5) HCDR1 as set forth in SEQ ID NO: 35, HCDR2 as set forth in SEQ ID NO: 36 and HCDR3 as set forth in SEQ ID NO: 37.

14. The bispecific antibody according to claim 13, **characterized in that** the VH of the third antigen-binding portion comprises an amino acid sequence as set forth in SEQ ID NO: 18, 22, 26, 30, or 34, or a sequence having at least 80% sequence identity to the amino acid sequence as set forth in SEQ ID NO: 18, 22, 26, 30, or 34.

15. The bispecific antibody according to any one of claims 12-14, **characterized in that** the third antigen-binding portion is a single domain antibody.

16. The bispecific antibody according to any one of claims 12-15, **characterized in that** the third antigen-binding portion has the same HCDR1, HCDR2, and HCDR3 as the first antigen-binding portion.

17. The bispecific antibody according to any one of claims 12-16, **characterized in that** the third antigen-binding portion has the same VH as the first antigen-binding portion.

18. The bispecific antibody according to any one of claims 12-17, **characterized in that** the third antigen-binding portion is fused to the N-terminus of the first antigen-binding portion or to the N-terminus of the Fab heavy chain of the second antigen-binding portion.

19. The bispecific antibody according to claim 18, **characterized in that** the third antigen-binding portion is fused to the N-terminus of the first antigen-binding portion or to the N-terminus of the Fab heavy chain of the second antigen-binding portion via a peptide linker, preferably, the sequence of the peptide linker is (GGGGS)n, wherein n is an integer not less than 1.

20. The bispecific antibody according to any one of claims 1-19, **characterized in that** the bispecific antibody consists of a first chain, a second chain, and a third chain, the first chain comprises a fusion protein of the first antigen-binding portion and the first Fc fragment, the second chain comprises a fusion protein of the Fab heavy chain of the second antigen-binding portion and the second Fc fragment, and the third chain comprises the Fab light chain of the second antigen-binding portion.

21. The bispecific antibody according to claim 20, **characterized in that** the first chain comprises an amino acid sequence as set forth in SEQ ID NO: 1, 4, 5, 6 or 7, or a sequence having at least 80% sequence identity to the amino acid sequence as set forth in SEQ ID NO: 1, 4, 5, 6, or 7, the second chain comprises an amino acid sequence as set forth in SEQ ID NO: 2, or a sequence having at least 80% sequence identity to the amino acid sequence as set forth in SEQ ID NO: 2, and the third chain comprises an amino acid sequence as set forth in SEQ ID NO: 3, or a sequence having at least 80% sequence identity to the amino acid sequence as set forth in SEQ ID NO: 3.

22. The bispecific antibody according to any one of claims 1-19, **characterized in that** the bispecific antibody consists of a first chain, a second chain, and a third chain, the first chain comprises a fusion protein of the third antigen-binding portion, the first antigen-binding portion, and the first Fc fragment, the second chain comprises a fusion protein of the Fab heavy chain of the second antigen-binding portion and the second Fc fragment, and the third chain comprises the Fab light chain of the second antigen-binding portion.

23. The bispecific antibody according to claim 22, **characterized in that** the first chain comprises an amino acid sequence as set forth in SEQ ID NO: 8, 9, 10, 11, or 12, or a sequence having at least 80% sequence identity to the amino acid sequence as set forth in SEQ ID NO: 8, 9, 10, 11, or 12, the second chain comprises an amino acid sequence as set forth in SEQ ID NO: 2, or a sequence having at least 80% sequence identity to the amino acid sequence as set forth in SEQ ID NO: 2, and the third chain comprises an amino acid sequence as set forth in SEQ ID NO: 3, or a sequence having at least 80% sequence identity to the amino acid sequence as set forth in SEQ ID NO: 3.

24. The bispecific antibody according to any one of claims 1-19, **characterized in that** the bispecific antibody consists of a first chain, a second chain, and a third chain, the first chain comprises a fusion protein of the first antigen-binding portion and the first Fc fragment, the second chain comprises a fusion protein of the third antigen-binding portion, the Fab heavy chain of the second antigen-binding portion, and the second Fc fragment, and the third chain comprises the Fab light chain of the second antigen-binding portion.

25. The bispecific antibody according to claim 24, **characterized in that** the first chain comprises an amino acid sequence as set forth in SEQ ID NO: 1, 4, 5, 6, or 7, or a sequence having at least 80% sequence identity to the amino acid sequence as set forth in SEQ ID NO: 1, 4, 5, 6, or 7, the second chain comprises an amino acid sequence as set forth in SEQ ID NO: 13, 14, 15, 16, or 17, or a sequence having at least 80% sequence identity to the amino acid sequence as set forth in SEQ ID NO: 13, 14, 15, 16, or 17, and the third chain comprises an amino acid sequence as set forth in SEQ ID NO: 3, or a sequence having at least 80% sequence identity to the amino acid sequence as set forth in SEQ ID NO: 3.

26. The bispecific antibody according to claim 25, **characterized in that** the first chain, the second chain, and the third chain are selected from the following groups:
(1) the first chain comprises an amino acid sequence as set forth in SEQ ID NO: 1, or a sequence having at least 80%, at least 85%, at least 90%, at least 95%, at least 96%, at least 97%, at least 98%, or at least 99% sequence identity to the amino acid sequence as set forth in SEQ ID NO: 1, the second chain comprises an amino acid sequence as set forth in SEQ ID NO: 13, or a sequence having at least 80%, at least 85%, at least 90%, at least 95%, at least 96%, at least 97%, at least 98%, or at least 99% sequence identity to the amino acid sequence as set forth in SEQ ID NO: 13, and the third chain comprises an amino acid sequence as set forth in SEQ ID NO: 3, or a sequence having at least 80%, at least 85%, at least 90%, at least 95%, at least 96%, at least 97%, at least 98%, or at least 99% sequence identity to the amino acid sequence as set forth in SEQ ID NO: 3;
(2) the first chain comprises an amino acid sequence as set forth in SEQ ID NO: 4, or a sequence having at least 80% sequence identity to the amino acid sequence as set forth in SEQ ID NO: 4, the second chain comprises an amino acid sequence as set forth in SEQ ID NO: 14, or a sequence having at least 80% sequence identity to the amino acid sequence as set forth in SEQ ID NO: 14, and the third chain comprises an amino acid sequence as set forth in SEQ ID NO: 3, or a sequence having at least 80% sequence identity to the amino acid sequence as set forth in SEQ ID NO: 3;
(3) the first chain comprises an amino acid sequence as set forth in SEQ ID NO: 5, or a sequence having at least 80% sequence identity to the amino acid sequence as set forth in SEQ ID NO: 5, the second chain comprises an amino acid sequence as set forth in SEQ ID NO: 15, or a sequence having at least 80% sequence identity to the amino acid sequence as set forth in SEQ ID NO: 15, and the third chain comprises an amino acid sequence as set forth in SEQ ID NO: 3, or a sequence having at least 80% sequence identity to the amino acid sequence as set forth in SEQ ID NO: 3;
(4) the first chain comprises an amino acid sequence as set forth in SEQ ID NO: 6, or a sequence having at least 80% sequence identity to the amino acid sequence as set forth in SEQ ID NO: 6, the second chain comprises an amino acid sequence as set forth in SEQ ID NO: 16, or a sequence having at least 80% sequence identity to the amino acid sequence as set forth in SEQ ID NO: 16, and the third chain comprises an amino acid sequence as set forth in SEQ ID NO: 3, or a sequence having at least 80% sequence identity to the amino acid sequence as set forth in SEQ ID NO: 3; and
(5) the first chain comprises an amino acid sequence as set forth in SEQ ID NO: 7, or a sequence having at least 80% sequence identity to the amino acid sequence as set forth in SEQ ID NO: 7, the second chain comprises an amino acid sequence as set forth in SEQ ID NO: 17, or a sequence having at least 80% sequence identity to the amino acid sequence as set forth in SEQ ID NO: 17, and the third chain comprises an amino acid sequence as set forth in SEQ ID NO: 3, or a sequence having at least 80% sequence identity to the amino acid sequence as set forth in SEQ ID NO: 3.

27. A polynucleotide, **characterized in that** the polynucleotide encodes the bispecific antibody according to any one of claims 1-26.

28. A recombinant expression vector, **characterized in that** the recombinant expression vector comprises the polynucleotide according to claim 27.

29. A host cell, **characterized in that** the host cell comprises the polynucleotide according to claim 27 or the recombinant expression vector according to claim 28.

30. A pharmaceutical composition, **characterized in that** the pharmaceutical composition comprises the bispecific antibody according to any one of claims 1-26, the polynucleotide according to claim 27, the recombinant expression vector according to claim 28, or the host cell according to claim 29, and a pharmaceutically acceptable carrier.

31. The bispecific antibody according to any one of claims 1-26, the polynucleotide according to claim 27, the recombinant expression vector according to claim 28, or the host cell according to claim 29 for use in the preparation of a drug for treating a disease associated with ROR1.

32. The use according to claim 31, **characterized in that** the disease associated with ROR1 is a tumour or cancer.

33. A method for treating a disease associated with ROR1, **characterized in that** the method comprises administering to a subject in need thereof the bispecific antibody according to any one of claims 1-26, the polynucleotide according to claim 27, the recombinant expression vector according to claim 28, the host cell according to claim 29, or the pharmaceutical composition according to claim 30.

34. The method according to claim 33, **characterized in that** the disease associated with ROR1 is a tumour or cancer.
